# EUROPÄISCHE PATENTSCHRIFT

(11) **EP 2 542 206 B1**
(45) Veröffentlichungstag und Bekanntmachung des Hinweises auf die Patenterteilung: **13.05.2015**
(21) Anmeldenummer: 11708015.0
(22) Anmeldetag: 01.03.2011
(51) Int. Cl.: A61K 8/00, A61K 8/72, A61Q 5/02, C08F 220/06, C08F 220/10

(54) **BLOCKCOPOLYMERE UND DEREN VERWENDUNG**
BLOCK COPOLYMERS AND USE THEREOF
COPOLYMÈRES EN BLOC ET LEURS UTILISATIONS

(30) Priorität: 02.03.2010 EP 10155218
(43) Veröffentlichungstag der Anmeldung: 09.01.2013
(73) Patentinhaber: BASF SE, 67056 Ludwigshafen (DE)
(72) Erfinder: NGUYEN-KIM, Son, 69502 Hemsbach (DE); JAHNEL, Wolfgang, 76756 Bellheim (DE)
(86) Internationale Anmeldenummer: PCT/EP2011/053000
(87) Internationale Veröffentlichungsnummer: WO 2011/107460

(56) Entgegenhaltungen:
- DE-A1- 4 325 158
- US-A- 4 692 502

## Beschreibung

Die vorliegende Erfindung betrifft ein Verfahren zur Herstellung eines vernetzten Copolymers mit anionogenen/anionischen Gruppen durch radikalische Copolymerisation nach der Methode der Fällungspolymerisation, die nach diesem Verfahren erhaltenen Copolymere und deren Verwendung.

Rheologiemodifizierungsmittel, die häufig in fester, pulverförmiger Form verarbeitet werden, werden in vielen technischen Bereichen, z. B. der Anstrichmittel, Papierherstellung, Textilindustrie, der Hygienemittel, kosmetischen und pharmazeutischen Mittel eingesetzt. Zu den derzeit am meisten eingesetzten Rheologiemodifizierungsmitteln zählen vernetzte Polyacrylsäuren.

Die US 3,915,921 beschreibt Copolymere, die eine olefinisch ungesättigte Carbonsäure, ein C₁₀-C₃₀-Alkyl(meth)acrylat und gegebenenfalls ein vernetzendes Monomer mit wenigstens 2 ethylenisch ungesättigten Doppelbindungen einpolymerisiert enthalten. In neutralisierter Form dienen sie als Verdicker für diverse Anwendungen.

Die US 2,798,053 beschreibt Copolymere von Acrylsäure und Polyethern mit wenigstens zwei Allylgruppen pro Molekül.

Die WO 2007/010034 beschreibt ein ampholytisches Copolymer A), erhältlich durch radikalische Copolymerisation von
a) wenigstens einer Verbindung mit einer radikalisch polymerisierbaren, α,β-ethylenisch ungesättigten Doppelbindung und mindestens einer anionogenen und/oder anionischen Gruppe pro Molekül,
b) wenigstens einer Verbindung, die ausgewählt ist unter N-Vinylimidazol-Verbindungen, N-[3-(dimethylamino)propyl]acrylamid, N-[3-(dimethylamino)propyl]methacrylamid und Mischungen davon, und
c) wenigstens einer radikalisch polymerisierbaren vernetzenden Verbindung, die wenigstens zwei α,β-ethylenisch ungesättigte Doppelbindungen pro Molekül enthält.

Die WO 2007/012610 beschreibt ein Silicongruppen-haltiges Copolymer A) erhältlich durch radikalische Copolymerisation von
a) wenigstens einer Verbindung mit einer radikalisch polymerisierbaren, α,β-ethylenisch ungesättigten Doppelbindung und mindestens einer ionogenen und/oder ionischen Gruppe pro Molekül,
b) wenigstens einer radikalisch polymerisierbaren vernetzenden Verbindung, die wenigstens zwei α,β-ethylenisch ungesättigte Doppelbindungen pro Molekül enthält,
in Gegenwart wenigstens einer eine Polyethergruppe und/oder eine radikalisch polymerisierbare olefinisch ungesättigte Doppelbindung enthaltenden Siliconverbindung c), wobei die Copolymerisation nach der Methode der Fällungspolymerisation erfolgen kann.
Die WO 2007/010035 beschreibt die Verwendung eines ampholytischen Copolymers, das einen molaren Überschuss an anionogenen/anionischen Gruppen gegenüber kationogenen/kationischen Gruppen aufweist oder das einen molaren Überschuss an kationogenen/kationischen Gruppen gegenüber anionogenen/anionischen Gruppen aufweist und das erhältlich ist durch radikalische Copolymerisation von
a1) wenigstens einer Verbindung mit einer radikalisch polymerisierbaren, α,β-ethylenisch ungesättigten Doppelbindung und mindestens einer anionogenen und/oder anionischen Gruppe pro Molekül,
a2) wenigstens einer Verbindung mit einer radikalisch polymerisierbaren, α,β-ethylenisch ungesättigten Doppelbindung, und mindestens einer kationogenen und/oder kationischen Gruppe pro Molekül,
b) wenigstens einer radikalisch polymerisierbaren vernetzenden Verbindung, die wenigstens zwei α,β-ethylenisch ungesättigte Doppelbindungen pro Molekül enthält,
c) gegebenenfalls in Gegenwart wenigstens einer eine Polyethergruppe und/oder eine radikalisch polymerisierbare olefinisch ungesättigte Doppelbindung enthaltenden Siliconverbindung,
als Rheologiemodifizierungsmittel für haarkosmetische Zusammensetzungen, wobei das ampholytische Copolymer durch radikalische Copolymerisation nach der Methode der Fällungspolymerisation hergestellt werden kann.

Die US 4,758,641 (und die äquivalente EP 279892) beschreibt ein Verfahren zur Herstellung von Polymeren olefinisch ungesättigter C₃-C₅-Carbonsäuren in einem Lösungsmittel, das ausgewählt ist unter Aceton und niederen Alkylacetaten, und in Gegenwart eines Vernetzers.

Die US 4,692,502 beschreibt ein Verfahren zur Polymerisation olefinisch ungesättigter Carbonsäuren in einem organischen Lösungsmittel und in Gegenwart von ionischen Tensiden.

Die US 4,526,937 beschreibt ein Verfahren zur Fällungspolymerisation olefinisch ungesättigter Carbonsäuren in Gegenwart von Polyoxyethylen-polyoxypropylen-Blockcopolymeren mit terminalen OH-Gruppen und einem HLB-Wert von größer als 10.

Die US 4,267,103 beschreibt die Lösungspolymerisation Carboxylgruppen-haltiger Monomere, wobei diese durch Umsatz mit einem Alkalihydroxid, Ammoniak oder einem Amin zumindest teilneutralisiert werden.

Die US 4,419,502 beschreibt ein Verfahren zur Fällungspolymerisation von olefinisch ungesättigten Carbonsäuren in Methylenchlorid und in Gegenwart eines Polyoxyethylenalkylethers und/oder Polyoxyethylensorbitesters mit einem HLB-Wert von größer als 12.

Die EP 0 584 771 A1 beschreibt ein Polymer einer olefinisch ungesättigten Carbonsäure und eines sterischen Stabilisator. Geeignete sterische Stabilisatoren sind lineare Blockcopolymere und statistische Kammpolymere mit hydrophilen und hydrophoben Einheiten.

Die US 4,375,533 beschreibt ein Verfahren zur Polymerisation olefinisch ungesättigter Carbonsäuren in einem Polymerisationsmedium, in dem das Carbonsäurepolymer unlöslich ist, in Gegenwart von einem Tensid mit einem HLB-Wert von weniger als 10.

Die US 4,420,596 beschreibt ein Verfahren zur Polymerisation olefinisch ungesättigter Carbonsäuren in einem Polymerisationsmedium, das Lösungsbenzin enthält, in Gegenwart von 1) einem Sorbitanester, 2) einem nichtionischen Tensid mit einem HLB-Wert von weniger als 10, wobei es sich um einen Ester von Glycerin oder einem Alkylenglykol handelt und 3) einem langkettigen Alkohol.

Die EP 1 209 198 A1 beschreibt eine Polymerzusammensetzung, die A) ein vernetztes Carboxylgruppen-haltiges Polymer und B) wenigstens eine Verbindung, die ausgewählt ist unter Estern mehrwertiger Alkohole mit Fettsäuren und den Alkylenoxidaddukten davon, enthält. Die Polymerzusammensetzung dient als Verdicker für diverse wässrige Lösungen.

Die unveröffentlichte europäische Patentanmeldung 08163686.2 beschreibt ein Verfahren zur Herstellung einer Copolymerzusammensetzung A) durch radikalische Copolymerisation einer Monomerzusammensetzung, die Acrylsäure und einen Vernetzer umfasst, durch Fällungspolymerisation in Gegenwart einer Hilfsstoffzusammensetzung H), die H1) Glycerinmonostearat, und H2) wenigstens eine Verbindung mit einem HLB-Wert im Bereich von 4 bis 10, umfasst.

Es ist weiterhin bekannt, Polyisobutenpolymere mit einer polaren Endgruppe in kosmetischen oder dermatologischen Zusammensetzungen einzusetzen. So beschreibt die EP 1 481 677 A1 eine topische Zusammensetzung, die Ascorbinsäure, ein Polyisobutenpolymer mit einer terminalen polaren Gruppe und ein N-Vinylimidazolpolymer enthält.

Es besteht nach wie vor Bedarf an polymeren Verdickern, die sich gut für eine Einstellung der rheologischen Eigenschaften diverser Produkte eignen, so dass sie z. B. in Form von Gelen formuliert werden können. Bevorzugt sind dabei pulverförmige Produkte, die sich durch eine hohe Auflösungsgeschwindigkeit bzw. Redispergiergeschwindigkeit auszeichnen. Vorzugsweise sollen sich die Produkte durch eine hohe Auflösungsgeschwindigkeit bzw. Redispergiergeschwindigkeit in wässrigen Medien auszeichnen. Wässrige Medien im Sinne der Erfindung sind Wasser und Gemische aus Wasser und wenigstens einem wassermischbaren Lösungsmittel. Die resultierenden Polymerformulierungen sollen eine hohe Stabilität haben und nicht zur Bildung von abgesetztem Feststoff neigen.

Überraschenderweise wurde nun gefunden, dass diese Aufgabe durch ein Polymerisationsverfahren gelöst wird, wobei die Herstellung eines vernetzen Copolymers mit anionogenen und/oder anionischen Gruppen durch radikalische Copolymerisation nach der Methode der Fällungspolymerisation in Gegenwart einer Hilfsstoffzusammensetzung erfolgt, die i) wenigstens eine Verbindung mit Blockstruktur und ii) wenigstens eine von i) verschiedene Verbindung, die wenigstens eine stickstoffatomhaltige Gruppe aufweist, die ausgewählt ist unter Amingruppen und Ammoniumgruppen, enthält.

Ein erster Gegenstand der Erfindung ist daher ein Verfahren zur Herstellung einer Copolymerzusammensetzung CP) durch radikalische Copolymerisation einer Monomerzusammensetzung, enthaltend
a) 70 bis 100 Gew.-%, bezogen auf das Gesamtgewicht der zur Polymerisation eingesetzten Monomere, Acrylsäure,
b) 0 bis 30 Gew.-%, bezogen auf das Gesamtgewicht der zur Polymerisation eingesetzten Monomere, wenigstens eine von a) verschiedene wasserlösliche nichtionische Verbindung mit einer radikalisch polymerisierbaren, α,β-ethylenisch ungesättigten Doppelbindung,
c) 0 bis 1 Gew.-%, bezogen auf das Gesamtgewicht der zur Polymerisation eingesetzten Monomere, wenigstens eine radikalisch polymerisierbare vernetzende Verbindung, die wenigstens zwei α,β-ethylenisch ungesättigte Doppelbindungen pro Molekül enthält,
nach der Methode der Fällungspolymerisation in Gegenwart einer Hilfsstoffzusammensetzung H), enthaltend
H1) wenigstens eine Verbindung mit Blockstruktur, die wenigstens eine hydrophobe Gruppe und wenigstens eine hydrophile Gruppe umfasst, wobei die Verbindung mit Blockstruktur H1) ausgewählt ist unter
   - Polyisobutenylalkoholalkoxilaten,
   - Polyisobutenylaminalkoxilaten,
   - Umsetzungsprodukten aus wenigstens einem Polyisobuten mit mindestens einer Carbonsäureendgruppe oder einem Derivat davon und wenigstens einem Polyalkylenoxid mit einer terminalen, gegenüber Anhydridgruppen reaktiven Gruppe,
   - Siliconverbindungen, die wenigstens eine Polyethergruppe aufweisen,
   - Umsetzungsprodukten aus wenigstens einer Verbindung, die mindestens eine hydrophobe Gruppe und mindestens eine gegenüber Isocyanatgruppen reaktive Gruppe umfasst, wenigstens einer Verbindung, die mindestens eine hydrophile Gruppe und mindestens eine gegenüber Isocyanatgruppen reaktive Gruppe umfasst, und wenigstens einem Polyisocyanat,
   und
H2) wenigstens eine von H1) verschiedene basische Verbindung, wobei die Komponente H2) ausgewählt ist unter NH₃, (NH₄)₂CO₃, NH₄HCO₃, Monoalkylaminen, Dialkylaminen, Trialkylaminen, Aminoalkoholen, stickstoffhaltigen Heterocyclen und Mischungen davon.

In einer bevorzugten Ausführung ist die Komponente H2) ausgewählt unter von H1) verschiedenen basischen Verbindungen, die wenigstens eine stickstoffatomhaltige Gruppe aufweisen, die ausgewählt ist unter Amingruppen und Ammoniumgruppen.

In einer weiteren bevorzugten Ausführung enthält die zur Herstellung der Copolymerzusammensetzung CP) eingesetzte Monomerzusammensetzung
a) 70 bis 99,99 Gew.-%, bezogen auf das Gesamtgewicht der zur Polymerisation eingesetzten Monomere, Acrylsäure,
b) 0 bis 29,99 Gew.-%, bezogen auf das Gesamtgewicht der zur Polymerisation eingesetzten Monomere, wenigstens eine von a) verschiedene hydrophile nichtionische Verbindung mit einer radikalisch polymerisierbaren, α,β-ethylenisch ungesättigten Doppelbindung,
c) 0,01 bis 1 Gew.-%, bezogen auf das Gesamtgewicht der zur Polymerisation eingesetzten Monomere, wenigstens eine radikalisch polymerisierbare vernetzende Verbindung, die wenigstens zwei α,β-ethylenisch ungesättigte Doppelbindungen pro Molekül enthält.

Ein weiterer Gegenstand der Erfindung ist eine nach dem zuvor und im Folgenden beschriebenen Verfahren erhältliche Copolymerzusammensetzung CP).

Ein weiterer Gegenstand der Erfindung ist die Verwendung einer solchen Copolymerzusammensetzung CP) oder eines daraus erhältlichen Copolymers in einer wässrigen Zusammensetzung zur Modifizierung der rheologischen Eigenschaften dieser Zusammensetzung.

Ein weiterer Gegenstand der Erfindung ist ein kosmetisches Mittel, enthaltend
A) wenigstens eine Copolymerzusammensetzung CP), erhältlich durch ein Verfahren, wie zuvor und im Folgenden definiert,
B) wenigstens einen kosmetisch akzeptablen Wirkstoff und
C) gegebenenfalls wenigstens einen weiteren, von CP) und B) verschiedenen kosmetisch akzeptablen Hilfsstoff.
Ein weiterer Gegenstand der Erfindung ist ein pharmazeutisches Mittel, enthaltend
A) wenigstens eine Copolymerzusammensetzung CP), erhältlich durch ein Verfahren, wie zuvor und im Folgenden definiert,
B) wenigstens einen pharmazeutisch akzeptablen Wirkstoff und
C) gegebenenfalls wenigstens einen weiteren, von A) und B) verschiedenen pharmazeutisch akzeptablen Hilfsstoff.

Ein weiterer Gegenstand der Erfindung ist die Verwendung einer Copolymerzusammensetzung CP), erhältlich durch ein Verfahren, wie zuvor und im Folgenden definiert, im Lebensmittelbereich zur Modifizierung der rheologischen Eigenschaften.

Ein weiterer Gegenstand der Erfindung ist die Verwendung einer Copolymerzusammensetzung CP), erhältlich durch ein Verfahren, wie zuvor und im Folgenden definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

Bevorzugt ist die Verwendung eines Blockcopolymers, wie zuvor und im Folgenden definiert, als Hilfsstoff zur Herstellung einer Copolymerzusammensetzung CP) durch radikalische Copolymerisation einer Monomerzusammensetzung, enthaltend
a) 70 bis 99,99 Gew.-%, bezogen auf das Gesamtgewicht der zur Polymerisation eingesetzten Monomere, Acrylsäure,
b) 0 bis 29,99 Gew.-%, bezogen auf das Gesamtgewicht der zur Polymerisation eingesetzten Monomere, wenigstens eine von a) verschiedene hydrophile nichtionische Verbindung mit einer radikalisch polymerisierbaren, α,β-ethylenisch ungesättigten Doppelbindung,
c) 0,01 bis 1 Gew.-%, bezogen auf das Gesamtgewicht der zur Polymerisation eingesetzten Monomere, wenigstens eine radikalisch polymerisierbare vernetzende Verbindung, die wenigstens zwei α,β-ethylenisch ungesättigte Doppelbindungen pro Molekül enthält.

Der Einsatz des erfindungsgemäßen Hilfsstoffsystems zur Herstellung von CP) durch Fällungspolymerisation bringt wenigstens einen der folgenden Vorteile mit sich:
- Die Copolymerzusammensetzung auf Basis dieses Hilfsstoffsystems lässt sich gut trocknen; die erhaltenen trockenen Zusammensetzungen sind sehr gut redispergierbar und zeichnen sich durch eine hohe Auflösungsgeschwindigkeit aus;
- die Copolymerzusammensetzung auf Basis dieses Hilfsstoffsystems lässt sich gut als stabile flüssige Zusammensetzung hoher Konzentration formulieren (z. B. als so genannte Stammlösung) und zu Produkten weiterverarbeiten;
- auch in einem physiologisch verträglichen pH-Bereich von etwa 5 bis 9 werden sehr gute Verdickungseigenschaften erzielt;
- das Reaktionsgemisch bei der Herstellung der Copolymerzusammensetzung CP) weist eine geringere Viskosität auf, so dass die Reaktionswärme besser abgeführt werden kann;
- bei der Reaktion und/oder den Formulierungen werden höhere Feststoffgehalte ermöglicht;
- eine Belagbildung im Polymerisationsreaktor kann in der Regel erfolgreich vermieden werden;
- durch die geringere Viskosität und/oder die hohen Feststoffgehalte lässt sich das Verfahren wirtschaftlicher gestalten;
- die erhaltenen Gele zeichnen sich durch wenigstens eine der folgenden anwendungstechnischen Eigenschaften aus: eine sehr gute Klarheit, sehr gute Struktur, gute Auswaschbarkeit.

Im Rahmen der vorliegenden Erfindung umfasst der Ausdruck Alkyl geradkettige und verzweigte Alkylgruppen. Geeignete kurzkettige Alkylgruppen sind z. B. geradkettige oder verzweigte C₁-C₇-Alkyl-, bevorzugt C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, etc.

Geeignete längerkettige C₈-C₃₀-Alkyl- bzw. C₈-C₃₀-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, bzw. um überwiegend lineare Alkenylreste, wie sie auch in natürlichen oder synthetischen Fettsäuren und Fettalkoholen sowie Oxoalkoholen vorkommen, die einfach, zweifach oder mehrfach ungesättigt sein können. Geeignete längerkettige C₈-C₃₀-Alkylgruppen sind z. B. n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Arachinyl, Behenyl, Lignocerinyl, Melissinyl, etc. Geeignete längerkettige C₈-C₃₀-Alkenylgruppen umfassen z. B. n-Octenyl, n-Nonenyl, n-Decenyl, n-Undecenyl, n-Dodecenyl, n-Tridecenyl, n-Tetradecenyl, n-Pentadecenyl, n-Hexadecenyl, n-Heptadecenyl, n-Octadecenyl, n-Nonadecenyl, n-Eicosenyl, n-Docosenyl, n-Tetracosenyl, Hexacosenyl, Triacontenyl, etc.

Cycloalkyl steht vorzugsweise für C₅-C₈-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Die erfindungsgemäßen Copolymerzusammensetzungen CP) lassen sich unter Normalbedingungen (20 °C) vorteilhaft als Gele formulieren. "Gelförmige Konsistenz" zeigen Formulierungen, die eine höhere Viskosität als eine Flüssigkeit aufweisen und die selbsttragend sind, d. h. die eine ihnen verliehene Form ohne formstabilisierende Umhüllung behalten. Im Gegensatz zu festen Formulierungen lassen sich gelförmige Formulierungen jedoch leicht unter Anwendung von Scherkräften deformieren. Die Viskosität der gelförmigen Mittel liegt vorzugsweise in einem Bereich von größer als 600 bis etwa 60000 mPas, besonders bevorzugt von 6000 bis 30000 mPas. Vorzugsweise handelt es sich bei den Gelen um Haargele.

Unter wasserlöslichen Monomeren und Polymeren werden im Rahmen der vorliegenden Erfindung Monomere und Polymere verstanden, die sich zu mindestens 1 g/l bei 20 °C in Wasser lösen. Unter wasserdispergierbaren Monomeren und Polymeren werden Monomere und Polymere verstanden, die unter Anwendung von Scherkräften, beispielsweise durch Rühren, in dispergierbare Partikel zerfallen. Hydrophile Monomere sind vorzugsweise wasserlöslich oder zumindest wasserdispergierbar. Die in den erfindungsgemäßen Copolymerzusammensetzungen CP) enthaltenen Copolymere sind im Allgemeinen wasserlöslich.

Unter einer gegenüber Isocyanatgruppen reaktiven Gruppe wird im Rahmen der Erfindung vorzugsweise eine Hydroxylgruppe, eine primäre Aminogruppe oder eine sekundäre Aminogruppe verstanden.

### H1) Verbindung mit Blockstruktur

Bevorzugt ist die Verbindung H1 ausgewählt unter Verbindungen der allgemeinen Formeln (I), (II) oder (III)

(A)ₙ-X-(B)ₘ (I)

[A-X]ₚ-B (II)

(A)-[X-B]_{q} (III)

worin
n für eine ganze Zahl von wenigstens 1 steht,
m für eine ganze Zahl von wenigstens 1 steht,
p für eine ganze Zahl von wenigstens 2 steht,
q für eine ganze Zahl von wenigstens 2 steht,
A für eine hydrophobe Gruppe steht, wobei
in den Verbindungen der Formeln (I) und (II) die Gruppen A jeweils gleiche oder verschiedene Bedeutungen aufweisen können und jeweils eine Bindungsstelle zu der Gruppe X aufweisen,
in den Verbindungen der Formel (III) die Gruppe A q Bindungsstellen zu je einer der Gruppen X aufweist,
X in den Verbindungen der Formel (I) für eine chemische Bindung oder einen (n + m)valenten organischen Rest steht und in den Verbindungen der Formeln (II) und (III) für eine chemische Bindung oder einen bivalenten organischen Rest steht,

### B für eine hydrophile Gruppe steht.

### Hydrophobe Gruppe von H1)

Bevorzugt steht in den Verbindungen der allgemeinen Formeln (I), (II) oder (III) wenigstens eine der Gruppen A oder B für eine polymere Gruppe einem zahlenmittleren Molekulargewicht in einem Bereich von 150 bis 1 000 000, besonders bevorzugt 250 bis 500 000.

Bevorzugt ist in den Verbindungen H1) die hydrophobe Gruppe ausgewählt ist unter
- C₈-C₃₀-Alkylgruppen
- Polyisobutenylgruppen
- Polytetrahydrofurangruppen
- Polyestergruppen
- Polysilicongruppen
und Kombinationen davon.

Speziell ist die hydrophobe Gruppe A in den Verbindungen der allgemeinen Formeln (I), (II) oder (III) ausgewählt unter
- C₈-C₃₀-Alkylgruppen
- Polyisobutenylgruppen
- Polytetrahydrofurangruppen
- Polyestergruppen
- Polysilicongruppen
und Kombinationen davon.

Geeignete C₈-C₃₀-Alkylgruppen sind geradkettige und verzweigte C₈-C₃₀-Alkylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen bzw. um überwiegend lineare Alkenylreste, wie sie auch in natürlichen oder synthetischen Fettsäuren und Fettalkoholen sowie Oxoalkoholen vorkommen, die einfach, zweifach oder mehrfach ungesättigt sein können. Geeignete längerkettige C₈-C₃₀-Alkylgruppen sind z. B. n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Arachinyl, Behenyl, Lignocerinyl, Melissinyl, etc.

Als hydrophobe Gruppe geeignete Polyisobutenylgruppen können linear oder verzweigt sein. Die Polyisobutenylgruppe kann dabei nur aus Isobuteneinheiten aufgebaut sein oder auch Comonomere einpolymerisiert enthalten. Bevorzugt sind im Wesentlichen homopolymere Polyisobutenylgruppen. Darunter werden im Rahmen der vorliegenden Erfindung solche Polyisobutenylgruppen verstanden, die zu wenigstens 85 Gew.-%, vorzugsweise zu wenigstens 90 Gew.-% und besonders bevorzugt zu wenigstens 95 Gew.-% aus Isobuteneinheiten [-CH₂C(CH₃)₂-] aufgebaut sind. Der Anteil an Comonomeren beträgt vorzugsweise weniger als 15 Gew.-%, besonders bevorzugt weniger als 10 Gew.-%, insbesondere weniger als 5 Gew.-%, bezogen auf das Gesamtgewicht der Polyisobutenylgruppe. Bevorzugte Comonomere sind Vinylaromaten, von Isobuten verschiedene Olefine mit 4 bis 10 C-Atomen und Mischungen davon. Die Comonomere sind vorzugsweise ausgewählt unter Styrol, C₁-C₄-Alkylstyrolen, wie 2-, 3-, 4-Methylstyrol und 4-tert.-Butylstyrol, 2-Methyl-buten-1, 2- Methylpenten-1, 2-Methylhexen-1, 2-Ethylpenten-1, 2-Ethylhexen-1, 2-Propylhepten-1, 1-Buten, cis-2-Buten, trans-2-Buten und Mischungen davon.

Die Polyisobutenylgruppen weisen vorzugsweise ein zahlenmittleres Molekulargewicht Mₙ von 150 bis 10 000, besonders bevorzugt von 200 bis 6 000, insbesondere von 300 bis 4 000 auf.

Die Polyisobutenylgruppen weisen vorzugsweise eine enge Molekulargewichtsverteilung auf. Die Polydispersität beträgt vorzugsweise höchstens 1,4, besonders bevorzugt höchstens 1,3, insbesondere höchstens 1,2. Unter Polydispersität versteht man den Quotienten aus gewichtsmittlerem Molekulargewicht M_{w} und zahlenmittlerem Molekulargewicht Mₙ (PDI = M_{w}/Mₙ).

Die Herstellung von Verbindungen H1) mit Polyisobutenylgruppen geht vorzugsweise aus von "hochreaktiven" Polyisobutenen aus. "Hochreaktive" Polyisobutene unterscheiden sich von den "niedrigreaktiven" Polyisobutenen durch den Gehalt an terminal angeordneten Doppelbindungen. So enthalten hochreaktive Polyisobutene wenigstens 50 Mol-%, bezogen auf die Gesamtanzahl an Polyisobuten-Makromolekülen, terminal angeordnete Doppelbindungen. Besonders bevorzugt sind Polyisobutene mit wenigstens 60 Mol-% und insbesondere mit wenigstens 80 Mol-%, bezogen auf die Gesamtanzahl an Polyisobuten-Makromolekülen, terminal angeordneten Doppelbindungen. Bei den terminal angeordneten Doppelbindungen kann es sich sowohl um Vinyldoppelbindungen [-CH=C(CH₃)₂] (β-Olefin) als auch um Vinyliden-Doppelbindungen [-CH-C(=CH₂)-CH₃] (α-Olefin) handeln. Außerdem werden zur Herstellung von Verbindungen H1), wie erwähnt, im Wesentlichen homopolymere Polyisobutene eingesetzt. Darunter werden im Rahmen der vorliegenden Erfindung solche Polyisobutene verstanden, die zu wenigstens 85 Gew.-%, vorzugsweise zu wenigstens 90 Gew.-% und besonders bevorzugt zu wenigstens 95 Gew.-% aus Isobuteneinheiten [-CH₂C(CH₃)₂-] aufgebaut sind.

Geeignete Polyisobutene für die Herstellung von Verbindungen H1) sind alle durch gängige kationische oder lebende kationische Polymerisation erhältlichen Polyisobutene. Bevorzugt sind jedoch so genannte "hochreaktive" Polyisobutene, die vorstehend bereits beschrieben wurden.

Geeignete Polyisobutene für die Herstellung von Verbindungen H1) sind beispielsweise die Glissopal-Marken der BASF SE, so z. B. Glissopal® 1000, Glissopal® 1300, Glissopal® 2300, Glissopal® SA, Glissopal® V 190, Glissopal® V 230, Glissopal® V 500, Glissopal® V 640, Glissopal® V 700 und Glissopal® V 1500.

Verfahren zur Herstellung von für die Herstellung von Verbindungen H1) geeigneten Polyisobutenen sind bekannt, beispielsweise aus der DE-A 27 02 604, EP-A 145 235, EP-A 481 297, EP-A 671 419, EP-A 628 575, EP-A 807 641 und WO 99/31151. Polyisobutene, die durch lebende kationische Polymerisation von Isobuten- bzw. Isobutenhaltigen Monomerengemischen hergestellt werden, sind beispielsweise in US 4,946,899, US 4,327,201, US 5,169,914, EP-A 206 756, EP-A 265 053, WO 02/48216 und in J. P. Kennedy, B. Ivan, "Designed Polymers by Carbocationic Macromolecular Engineering", Oxford University Press, New York 1991 beschrieben. Auf diese und andere Publikationen, die Polyisobutene beschreiben, wird hiermit in vollem Umfang Bezug genommen.

Die Herstellung von Verbindungen H1) mit Polyisobutenylgruppen geht vorzugsweise aus von Polyisobutenen, die ausgewählt sind unter:
- hochreaktiven Polyisobutenen,
- Polyisobutenen mit mindestens einer stickstoffhaltigen Endgruppe,
- Polyisobutenylalkoholen,
- Polyisobutenylaldehyden,
- Polyisobutenen mit mindestens einer Carbonsäureendgruppe oder einem Derivat davon,
- Mischungen aus zwei oder mehr als zwei der zuvor genannten Verbindungen.

Die Herstellung von Verbindungen H1) mit Polyisobutenylgruppen geht vorzugsweise aus von hochreaktiven Polyisobutenen oder in anderer Weise funktionalisierten Polyisobutenen, die sich zur Anbindung wenigstens einer hydrophilen Gruppe eignen. Die in anderer Weise funktionalisierten Polyisobutene können z. B. aus hochreaktiven Polyisobutenen durch ein- oder mehrstufige Funktionalisierung der terminalen Doppelbindung erhalten werden.

In einer ersten Ausführungsform wird zur Herstellung von Verbindungen H1) mit wenigstens einer Polyisobutenylgruppe als hydrophober Gruppe ein hochreaktives Polyisobuten eingesetzt.

In einer zweiten Ausführungsform wird zur Herstellung von Verbindungen H1) mit wenigstens einer Polyisobutenylgruppe als hydrophober Gruppe ein Polyisobuten mit mindestens einer stickstoffhaltigen Endgruppe eingesetzt.

Die stickstoffhaltige Endgruppe kann ein Stickstoffatom oder mehrere Stickstoffatome umfassen. Bevorzugt weist die stickstoffhaltige Endgruppe 1, 2, 3, 4, 5, 6, 7, 8, 9 oder 10 Stickstoffatome auf. Die Stickstoffatome können beispielsweise in Form von Aminogruppen, Iminogruppen oder Amidgruppen in die stickstoffhaltige Endgruppe eingebaut sein. Geeignete Aminogruppen sind primäre, sekundäre oder tertiäre Aminogruppen.

In einer speziellen Ausführung wird zur Herstellung von Verbindungen H1) mit wenigstens einer Polyisobutenylgruppe als hydrophober Gruppe ein Polyisobuten mit mindestens einer stickstoffhaltigen Endgruppe eingesetzt, die wenigstens eine primäre oder sekundäre Aminogruppe aufweist. Diese eignen sich vorteilhaft für die weitere Umsetzung zur Herstellung der Verbindungen H1) mit wenigstens einer Verbindung mit mindestens einer hydrophilen Gruppe. Die Anbindung kann dabei auch durch Umsetzung wenigstens eines Polyisobutens mit mindestens einer stickstoffhaltigen Endgruppe mit wenigstens einer Verbindung mit mindestens einer hydrophilen Gruppe und mit einer weiteren Verbindung, die zur Reaktion mit beiden befähigt ist, erfolgen. Dazu zählt z. B. die Verknüpfung mittels Polyisocyanaten, wie im Folgenden eingehend beschrieben.

Bevorzugt handelt es sich um 1 bis 10 Aminogruppen pro terminaler Gruppe. Weiterhin bevorzugt sind die Aminogruppen ausgewählt unter primären, sekundären und/oder tertiären Aminogruppen. Bei den kann sich beispielsweise um von geradkettigen oder verzweigten Alkylenpolyaminen abgeleitete Gruppen handeln. Die stickstoffhaltigen Endgruppen können neben den Stickstofffunktionalitäten auch noch andere Funktionalitäten umfassen. Dazu zählen insbesondere sauerstoffhaltige funktionelle Gruppen wie OH-Gruppen oder Ethergruppen.

Polyisobutene mit mindestens einer stickstoffhaltigen Endgruppe (auch als Polyisobutenamine oder PIBA bezeichnet) und Verfahren zu ihrer Herstellung sind bekannt. Übersichten über geeignete Verfahren zur Aminofunktionalisierung findet man in der der WO 03/085011 sowie der darin zitierten Literatur, speziell EP-A 382 405, WO 98/20053 und die nachfolgend aufgeführten Schriften. Zudem sind in der Literatur zahlreiche Verfahren zur Herstellung OH- oder Aldehyd-funktionalisierter Polyisobutene beschrieben (siehe z. B. EP-A 468 966). Die so hergestellten Polyisobuten-Derivate können in an sich bekannter Weise durch reduktive Aminierung aminofunktionalisiert werden. Beispiele für geeignete Funktionalisierungsverfahren sind die nachfolgend aufgeführten, aus der Literatur bekannten Funktionalisierungsverfahren (1) bis (8):
(1) Umsetzung des Polyisobutens zu Polyisobutenylsuccinanhydriden (PIBSA) und weitere Umsetzung mit Ammoniak oder Aminen;
(2) Hydroformylierung des Polyisobutens mit nachfolgender reduktiver Aminierung des Hydroformylierungsprodukts in Gegenwart von Ammoniak, Aminen oder Aminoalkoholen oder Hydroformylierung des Polyisobutens in Gegenwart von Ammoniak oder Aminen unter reduzierenden Bedingungen wie in EP-A 244 616 oder WO 94/24231 beschrieben;
(3) Hydroborierung des Polyisobutens mit anschließender oxidativer Spaltung des Boran-Addukts (siehe J.P. Kennedy und B. Ivan "Designed Polymers by Carbocationic Macromolecular Engineering", S. 178f.) und nachfolgender reduktiver Aminierung in Gegenwart von Ammoniak oder Aminen in an sich bekannter Weise;
(4) Hydroborierung oder Hydroformylierung unter reduzierenden Bedingungen zu einem Polyisobutenyl-Alkohol, gefolgt von einer Alkoxylierung und einer reduktiven Aminierung in Gegenwart von Ammoniak oder Aminen (siehe EP-A 277 345, WO 98/20053 und WO 00/50543);
(5) Umsetzung des Polyisobutens mit einem Stickoxid-haltigen Oxidans und anschließende Reduktion der so eingeführten NOₓ-Gruppen zu NH₂-Gruppen, vgl. z. B. DE-A 4425834, WO 96/03367, WO 96/03479, WO 97/03946;
(6) Epoxidierung des Polyisobutens und anschließende Umsetzung des Epoxidierungsprodukts mit Ammoniak, einem Amin oder einem Aminoalkohol, gegebenenfalls mit anschließender oder gleichzeitiger Eliminierung von Wasser und katalytischer Reduktion, vgl. z. B. WO 92/12221, WO 92/14806, EP-A 476 485, EP 539 821, EP-A 696572 und DE-A 19620262;
(7) Hydrocyanierung des Polyisobutens unter saurer Katalyse und anschließende Hydrolyse im Sinne einer Ritter-Reaktion wie in DE-OS 2061057 oder EP-A 567 810 beschrieben (zur Ritter-Reaktion siehe auch Houben-Weyl, E5, S. 1032-1041 (1985) bzw. Houben-Weyl, XI/1, S. 994 f. (1957); oder
(8) Umsetzung des Polyisobutens mit Phenol unter Friedel-Crafts-Bedingungen und nachfolgende Umsetzung des Polyisobutenylphenols mit Formaldehyd und Amin im Sinne einer Mannich-Reaktion (siehe z. B. WO 01/25293 und WO 01/25294).

Vorzugsweise sind die Amine zur Herstellung von Polyisobutenen mit mindestens einer stickstoffhaltigen Endgruppe ausgewählt unter Ammoniak und davon verschiedenen Aminen der Formel HNR^{c}R^{d}, worin die Reste R^{c} und R^{d} ausgewählt sind unter Wasserstoff, und C₁- bis C₂₀-Alkyl-, C₃- bis C₈-Cycloalkyl- und C₁- bis C₂₀-Alkoxyresten. Dabei können die Alkyl- und Alkoxygruppen in Abhängigkeit von ihrer Kettenlänge durch 1 bis 10 nicht benachbarte Heteroatome, vorzugsweise ausgewählt unter N und O, unterbrochen sein, wobei die N-Heteroatome wiederum jeweils einen Substituenten, vorzugsweise ausgewählt unter C₁-C₆-Alkyl, C₃-C₈-Cycloalkyl, C₆-C₁₄-Aryl und Heteroaryl, tragen können. Weiterhin können R^{c} und R^{d} gemeinsam mit dem N-Atom, an das sie gebunden sind, einen 5-, 6-oder 7-gliedrigen Cyclus bilden, der ein oder zwei weitere Heteroatome, ausgewählt unter N und O aufweisen und/oder mit einem, zwei oder drei C₁- bis C₆-Alkylresten substituiert sein kann. Weiterhin können R^{c} und R^{d} auch für Aryl- und Heteroarylreste stehen. Aryl- und Heteroarylreste weisen gegebenenfalls einen bis drei Substituenten, ausgewählt z. B. unter Hydroxy und den vorgenannten Alkyl-, Cycloalkyl- oder Alkoxyresten und Polyisobutenresten, auf.

Geeignete Reste R^{c}, R^{d} sind beispielsweise Wasserstoff, Methyl, Ethyl, n-Propyl, Isopropyl, n-Butyl, iso-Butyl, sek.-Butyl, tert.-Butyl, n-Pentyl und n-Hexyl, Cyclopentyl, Cyclohexyl, Phenyl, Tolyl, Xylyl, Naphthyl, Tetrahydrofuranyl, Tetrahydropyranyl, Dioxanyl, Pyrrolidyl, Piperidyl, Pyridyl und Pyrimidyl.

Geeignete Verbindungen der Formel HNR^{c}R^{d}, die ausschließlich eine primäre Aminofunktion aufweisen, sind beispielsweise Methylamin, Ethylamin, n-Propylamin, Isopropylamin, n-Butylamin, iso-Butylamin, sek.-Butylamin, tert.-Butylamin, Pentylamin, Hexylamin, Cyclopentylamin, Cyclohexylamin, Anilin und Benzylamin.

Geeignete Verbindungen der Formel HNR^{c}R^{d}, die ausschließlich eine primäre Aminofunktion aufweisen und bei denen der Rest R^{c} oder R^{d} für durch das Heteroatom O unterbrochene und/oder substituierte Alkylreste steht, sind beispielweise CH₃-O-C₂H₄-NH₂, C₂H₅-O-C₂H₄-NH₂, CH₃-O-C₃H₆-NH₂, C₂H₅-O-C₃H₆-NH₂, n-C₄H₉-O-C₄H₈-NH₂,- HO-C₂H₄-NH₂, HO-C₃H₇-NH₂ und HO-C₄H₈-NH2.

Geeignete Verbindungen der Formel HNR^{c}R^{d}, die ausschließslich eine sekundäre Aminofunktion aufweisen, sind beispielweise Dimethylamin, Diethylamin, Methylethylamin, Di-n-propylamin, Diisopropylamin, Diisobutylamin, Di-sek.-butylamin, Di-tert.-butylamin, Dipentylamin, Dihexylamin, Dicyclopentylamin, Dicyclohexylamin und Diphenylamin.

Geeignete Verbindungen der Formel HNR^{c}R^{d}, die ausschließlich eine sekundäre Aminofunktion aufweisen und bei denen der Rest R^{c} und R^{d} für durch das Heteroatom O unterbrochene und/oder substituierte Alkylreste steht, sind beispielweise (CH₃-O-C₂H₄)₂NH, (C₂H₅-O-C₂H₄)₂NH, (CH₃-O-C₃H₆)₂NH, (C₂H₅-O-C₃H₆)₂NH, (n-C₄H₉-O-C₄H₈)₂NH, (HO-C₂H₄)₂NH, (HO-C₃H₆)₂NH und (HO-C₄H₈)₂NH.

Geeignete Verbindungen der Formel HNR^{c}R^{d}, bei denen R^{c} und R^{d} gemeinsam mit dem N-Atom, an das sie gebunden sind, einen 5-, 6- oder 7-gliedrigen Cyclus bilden, der ein oder zwei Heteroatome, ausgewählt unter N und O, aufweisen und mit einem, zwei oder drei C₁-bis C₆-Alkylresten substituiert sein kann, sind beispielsweise Pyrrolidin, Piperidin, Morpholin und Piperazin sowie deren substituierte Derivate, wie N-C₁-bis C₆-Alkylpiperazine und Dimethylmorpholin.

Geeignete Verbindungen der Formel HNR^{c}R^{d}, die durch N unterbrochene und/oder substituierte Alkylreste aufweisen, sind Alkylendiamine, Dialkylentriamine, Trialkylentetramine und davon verschiedene Polyalkylenpolyamine. Dazu zählen Oligo- oder Polyalkylenimine, insbesondere Oligo- oder Polyethylenimine. Bevorzugte Oligoethylenimine, bestehend aus 2 bis 20, besonders bevorzugt 2 bis 10 und insbesondere 2 bis 3 Ethylenimineinheiten. Geeignet sind insbesondere Ethylendiamin, n-Propylendiamin, 1,4-Butandiamin, 1,6-Hexandiamin, Diethylentriamin und Triethylentetramin, sowie deren Alkylierungsprodukte, die noch wenigstens eine primäre oder sekundäre Aminofunktion aufweisen, z. B. 3-(Dimethylamino)-n-propylamin, N, N-Dimethylethylendiamin, N,N-Diethylethylendiamin und N,N,N',N'-Tetramethyldiethylentriamin.

Weitere geeignete Verbindungen der Formel HNR^{c}R^{d} sind die Umsetzungsprodukte von Alkylenoxiden, insbesondere Ethylenoxid, mit primären Aminen, sowie Copolymerisate von Ethylenoxid mit Ethylenimin und/oder primären oder sekundären C₁- bis C₆-Alkylaminen.

Zur Herstellung von Verbindungen H1) mit wenigstens einer Polyisobutenylgruppe als hydrophober Gruppe besonders geeignete Polyisobutene mit mindestens einer stickstoffhaltigen Endgruppe eingesetzt sind die Umsetzungsprodukte von Polyisobutenylsuccinanhydriden (PIBSA) mit Ammoniak oder insbesondere Aminen. Polyisobutenylsuccinanhydride können durch Umsetzung von hochreaktiven Polyisobutenen mit Maleinsäureanhydrid hergestellt werden. Diese Umsetzung im Sinne einer En-Reaktion, und die weitere Umsetzung der so erhaltenen Produkte mit Aminen ist bereits in der DE 27 02 604 A1 beschrieben. Eine aktuelle Übersicht findet sich in der WO 2008/132083. Unter diesen PIBSA-Umsetzungsprodukten besonders bevorzugt sind Polyisobutenylsuccinimide (PIBSI) der Formel (A) 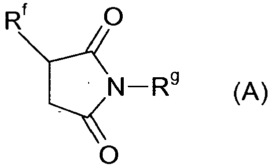 worin
R^{f} für einen Polyisobutenylrest steht, und
R^{g} für Kohlenwasserstoffrest steht, der eine endständige Aminogruppe aufweist.
PIBSI werden durch Umsetzung von PIBSA mit Ammoniak oder primären Aminen H₂NR^{g} (insbesondere Di- oder Polyaminen) erhalten.
R^{g} hat vorzugsweise eine der zuvor für die Reste R^{c} und R^{d} bei den Aminen der Formel HNR^{c}R^{d} angegebenen Bedeutungen. Darauf wird hier in vollem Umfang Bezug genommen.

Bevorzugt steht R^{g} für Wasserstoff oder einen Kohlenwasserstoffrest, der eine endständige Aminogruppe aufweist und aliphatisch oder aromatisch sein kann.

Besonders bevorzugt steht R^{g} für einen aliphatischen Kohlenstoffrest mit 1 bis 60 C-Atomen, insbesondere 2 bis 30 C-Atomen und vor allem 2 bis 16 C-Atomen.

Beispiele für besonders geeignete Reste R^{g} sind geradkettige oder verzweigte ω-Aminoalkylenreste, wie ω-Aminomethylen, ω-Aminoethylen, ω-Aminopropylen, ω-Aminobutylen, ω-Aminopentylen und ω-Aminohexylen.

Weiterhin bevorzugt steht R^{g} für einen Aminoalkylenrest, worin die Alkylenkette durch eine Aminogruppe oder mehrere Aminogruppen der Formel NR^{h} unterbrochen ist, wobei die Reste R^{h} unabhängig voneinander für Wasserstoff oder C₁-C₆-Alkyl stehen. Besonders bevorzugt steht R^{h} für Wasserstoff. Solche Reste R^{g} weisen z. B. folgende Struktur auf:

-(CH₂)ₓ-NH-[(CH₂)_{y}-NH]_{z}-(CH₂)ₓ-NR^{k}R^{l}

wobei x und y unabhängig voneinander für 1 bis 6, bevorzugt für 2 bis 4, besonders bevorzugt für 2 oder 3, und z für 0 bis 8 stehen und R^{k} und R^{l} unabhängig voneinander für Wasserstoff oder C₁-C₆-Alkyl stehen, Besonders stehen R^{k} und R^{l} für Wasserstoff oder Methyl und insbesondere für Wasserstoff.

Besonders bevorzugte Reste R^{g} leiten sich von Polyalkylenpolyaminen, vor allem Polyethylenpolyaminen und Polyethyleniminen, ab. Besonders geeignete Reste R^{g} leiten sich von den folgenden Aminen ab: Diethylentriamin, Triethylentetramin und Pentaethylentetramin.

Beispiel für einen ganz besonders bevorzugten Rest R^{g} ist

-CH₂-CH₂-NH-CH₂-CH₂-NH-CH₂-CH₂-NH-CH₂-CH₂-NH₂

Die Reste R^{g} können auch weitere funktionelle Gruppen, insbesondere Hydroxy- und/oder Ethergruppen, umfassen. Bevorzugt sind sie jedoch nur durch Aminogruppen funktionalisiert.

Selbstverständlich können auch Mischungen verschiedener Polyisobutylensuccinimide der allgemeinen Formel (A) oder Mischungen der Polyisobutylensuccinimide (A) mit weiteren, davon verschiedenen stickstoffhaltigen Polyisobutylenderivaten als Komponente zur Herstellung von (H1) eingesetzt werden.

Weitere geeignete Umsetzungsprodukte von PIBSA mit Aminen sind vorzugsweise ausgewählt unter Verbindungen der Formeln (B), (C) und (D) und Mischungen davon 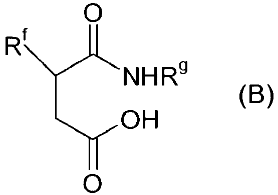 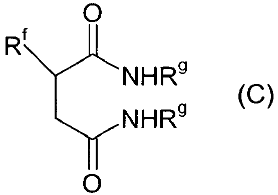 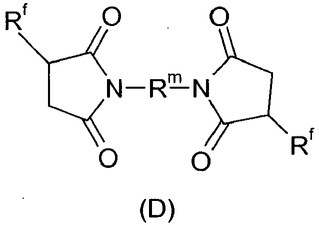 worin
R^{f} für einen Polyisobutenylrest steht, und
R^{m} für Kohlenwasserstoffrest steht.

Die Verbindungen der Formeln (B), (C) und (D) können durch Variation der Reaktionsbedingungen gezielt hergestellt werden oder als Nebenprodukte in Zusammensetzungen der Polyisobutylensuccinimide (A) enthalten sein.

Polyisobutylensuccinamidsäuren der Formel (B) resultieren intermediär bei der äquimolaren Umsetzung von PIBSA mit primären Aminen zu den Polyisobutylensuccinimiden (A).

Polyisobutylensuccinamide der Formel (C) entstehen als Hauptprodukte bei der Umsetzung von PIBSA mit einem molaren Überschuss Amin. Bevorzugt liegt das Molmengenverhältnis von PIBSA zu Amin bei der Herstellung von Verbindungen (C) etwa bei 1 : 2.

In den Di-Succinimiden der Formel (D) steht R^{m} für einen von R^{g} abgeleiteten Rest, der über eine Aminogruppe in den zweiten Succinimidring eingebaut ist.

In einer dritten Ausführungsform wird zur Herstellung von Verbindungen H1) mit wenigstens einer Polyisobutenylgruppe als hydrophober Gruppe wenigstens ein Polyisobuten eingesetzt, das ausgewählt ist unter Polyisobutenylalkoholen, Polyisobutenylaldehyden und Mischungen davon.

Die Herstellung von Verbindungen H1) mit Polyisobutenylgruppen geht nach dieser dritten Ausführungsform vorzugsweise aus von Polyisobutenen, die ausgewählt sind unter durch Hydroformylierung von Polyisobutenen erhaltenen Produkten. Bevorzugt werden zur Hydroformylierung hochreaktive Polyisobutene eingesetzt. Das bei der Hydroformylierung entstehende Oxoprodukt enthält Polyisobutenylaldehyde und/oder Polyisobutenylalkohole. Polyisobutenylaldehyde und Polyisobutenylaldehyd-haltige Gemische können durch Hydrierung nach bekannten Verfahren in Polyisobutenylalkohole überführt werden. Die Hydroformylierung und Hydrierung von hochreaktiven Polyisobutenen ist z. B. in der EP-A-244 616 beschrieben, worauf hier in vollem Umfang Bezug genommen wird. Produkte mit hohem Gehalt an Polyisobutenylalkoholen lassen sich auch durch reduzierende Hydroformylierung erzielen, wie sie z. B. in der EP-A-277 345 beschrieben ist. Durch Hydroformylierung erhaltene Polyisobutenylalkohole weisen reaktionsbedingt eine CH₂-Gruppe mehr auf als die Ausgangspolyisobutene. Sie können durch die Formel R-CH₂-OH wiedergegeben werden, worin R für einen Polyisobutenylrest steht. Zur Herstellung von Polyisobutenylalkoholen der Formel ROH, worin R für einen Polyisobutenylrest steht, geht man z. B. von Polyisobutenen mit Doppelbindungen aus, welche sich überwiegend weiter innen in der Polymerkette (beispielsweise in der β- oder γ-Position) befinden. Diese werden dann durch Ozonolyse und nachfolgende Reduktion oder durch Epoxidierung und nachfolgende Reduktion oder durch Hydroborierung und nachfolgende Hydrolyse oder durch Halogenierung mit Chlor oder Brom und nachfolgende alkalische Hydrolyse in die Polyisobutenalkohole umgewandelt. Die letztgenannten Verfahren sind in der WO 00/50543 beschrieben.

In einer vierten Ausführungsform wird zur Herstellung von Verbindungen H1) mit wenigstens einer Polyisobutenylgruppe als hydrophober Gruppe wenigstens ein Polyisobuten eingesetzt, das ausgewählt ist unter Polyisobutenen mit mindestens einer Carbonsäureendgruppe oder einem Derivat davon. Geeignete Derivate sind z. B. Carbonsäureanhydride, Carbonsäureester, Carbonsäureamide, Carbonsäureimide und Carbonsäuresalze.

Die Herstellung von Polyisobutenen mit Carbonsäuregruppen und/oder Carbonsäureanhydridgruppen kann, wie zuvor beschrieben, durch Umsetzung von hochreaktiven Polyisobutenen mit Maleinsäureanhydrid hergestellt werden. Diese Umsetzung im Sinne einer En-Reaktion zur Herstellung von Polyisobutenylbernsteinsäureanhydriden (PIBSA) ist in der DE 27 02 604 A1 und WO 2008/132083 beschrieben.

PIBSA sind kommerziell erhältlich, z. B. von BASF SE unter der Bezeichnung Glissopal® SA. Geeignet zur Herstellung von Verbindungen H1) mit wenigstens einer Polyisobutenylgruppe als hydrophober Gruppe sind auch Derivate. Zur Umsetzung mit den PIBSA geeignete Alkohole sind beispielsweise Di- oder Polyole mit vorzugsweise 2 bis 5 Hydroxylgruppen, z. B. Ethylenglykol, Glycerin, Diglycerin, Triglycerin, Trimethylolpropan, Pentaerythrit. Zur Umsetzung mit den PIBSA geeignete Aminoalkohole sind beispielsweise Alkanolamine wie Ethanolamin und 3-Aminopropanol.

Das Molverhältnis von PIBSA zu den genannten Aminen, Alkoholen oder Aminoalkoholen liegt bei der Umsetzung in der Regel im Bereich von 0,4 : 1 bis 4 : 1 , vorzugsweise 0,5 : 1 bis 3 : 1. Bei Verbindungen mit nur einer primären oder sekundären Aminogruppe wird man häufig wenigstens äquimolare Mengen an Amin einsetzen.

Zur Einführung einer hydrophilen Gruppe und Bereitstellung von Verbindungen H1) können die zuvor genannten PIBSA-Derivate einer Ethoxilierung und/oder Propoxilierung unterzogen werden. Bevorzugt sind ethoxylierte und/oder propoxylierte Derivate der Umsetzungsprodukte von PIBSA mit den genannten Diolen, Polyolen, Aminen und Aminoalkohole.

In einer speziellen Ausführung geht die Herstellung von Verbindungen H1) mit Polyisobutenylgruppen aus von Polyisobutenen, die wenigstens eine gegenüber Isocyanatgruppen reaktive Gruppe aufweisen.

Als hydrophobe Gruppe in den Verbindungen H1), speziell als hydrophobe Gruppe A in den Verbindungen der allgemeinen Formeln (I), (II) oder (III), eignen sich weiterhin Polytetrahydrofurangruppen. Bevorzugt sind Polytetrahydrofurangruppen mit einem zahlenmittleren Molekulargewicht im Bereich von etwa 300 bis 10 000, besonders bevorzugt etwa 400 bis 5000.

Die Herstellung von Verbindungen H1) mit Polytetrahydrofurangruppen geht vorzugsweise aus von Polytetrahydrofurandiolen. Geeignete Polytetrahydrofurandiole können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

In einer speziellen Ausführung geht die Herstellung von Verbindungen H1) mit Polytetrahydrofurangruppen aus von Polytetrahydrofuranen, die wenigstens eine gegenüber Isocyanatgruppen reaktive Gruppe aufweisen.

Als hydrophobe Gruppe in den Verbindungen H1), speziell als hydrophobe Gruppe A in den Verbindungen der allgemeinen Formeln (I), (II) oder (III), eignen sich weiterhin Polyestergruppen. Bevorzugt sind Polyestergruppen mit einem zahlenmittleren Molekulargewicht im Bereich von etwa 300 bis 10 000, bevorzugt 400 bis 5000.

Die Herstellung von Verbindungen H1) mit Polyestergruppen geht vorzugsweise aus von Polyesterdiolen. Bevorzugt sind Polyesterdiole auf Basis aromatischer Dicarbonsäuren, aliphatischer Dicarbonsäuren, cycloaliphatischer Dicarbonsäuren, und Mischungen davon. Die Dicarbonsäuren sind vorzugsweise ausgewählt unter Terephthalsäure, Isophthalsäure, Phthalsäure, Na- oder K-Sulfoisophthalsäure, Adipinsäure, Bernsteinsäure, 1,2-, 1,3- oder 1,4-Cyclohexandicarbonsäure und Mischungen davon. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, und Mischungen davon.

Bevorzugt sind Polyesterdiole auf Basis wenigstens einer aromatischen Dicarbonsäuren, wenigstens einer aliphatischen Dicarbonsäuren und wenigstens eines aliphatischen Diols. Dazu zählen insbesondere solche, bei denen die aromatische Dicarbonsäure 10 bis 95 Mol-%, insbesondere 40 bis 90 Mol-% des gesamten Dicarbonsäureanteils (Rest aliphatische Dicarbonsäuren) ausmacht.

Besonders bevorzugte Polyesterdiole sind die Umsetzungsprodukte aus Phthalsäure/Diethylenglykol, Isophthalsäure/1,4-Butandiol, Isophthalsäure/Adipinsäure/ 1,6-Hexandiol, 5-NaSO₃-Isophthalsäure/Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/Diethylenglykol/Dimethylolcyclohexan, Isophthalsäure/Adipinsäure, Neopentylglykol/Dimethylolcyclohexan.

Bevorzugt sind weiterhin Polyesterdiole auf Basis von linearen oder verzweigten, C₈-C₃₀-Di- oder Polycarbonsäuren und C₈-C₃₀-Hydroxycarbonsäuren. Bevorzugte Carbonsäuren und Hydroxycarbonsäuren sind z. B. Acelainsäure, Dodecandisäure, Korksäure, Pimelinsäure, Sebacinsäure, Tetradecandisäure, Citronensäure, Ricinolsäure, Hydroxystearinsäure und Gemische davon. Als Diolkomponente zur Herstellung dieser Polyesterdiole werden vorzugsweise 1,4-Butandiol, 1,5-Pentandiol, 1,6-Hexandiol, Neopentylglykol, 1,4-Dimethylolcyclohexan, Diethylenglykol und Gemische davon eingesetzt.

In einer speziellen Ausführung geht die Herstellung von Verbindungen H1) mit Polyestergruppen aus von Polyestern, die wenigstens eine gegenüber Isocyanatgruppen reaktive Gruppe aufweisen.

Als hydrophobe Gruppe in den Verbindungen H1), speziell als hydrophobe Gruppe A in den Verbindungen der allgemeinen Formeln (I), (II) oder (III), eignen sich weiterhin Silicongruppen.

Bevorzugt als Silicongruppen sind Polysiloxangruppen mit einem zahlenmittleren Molekulargewicht im Bereich von etwa 300 bis 50 000, besonders bevorzugt 400 bis 30 000.

In einer speziellen Ausführung geht die Herstellung von Verbindungen H1) mit Silicongruppen aus von Polysiloxanen, die wenigstens eine gegenüber Isocyanatgruppen reaktive Gruppe aufweisen.

Zur Einführung einer hydrophilen Gruppe und Bereitstellung von Verbindungen H1) können die zuvor genannten Silconverbindungen einer Umsetzung zur Einführung wenigstens einer Polyethergruppe unterzogen werden. Bevorzugt sind die Ethergruppen ausgewählt unter Polyethylenoxidgruppen, Polypropylenoxidgruppen und Poly(ethylenoxid/propylenoxid)gruppen.

Geeignete Siliconverbindungen H1) und zur Herstellung von Siliconverbindungen H1) sind die unter den INCI-Namen Dimethicone-Copolyole oder Silicontenside bekannten Verbindungen, wie zum Beispiel die unter den Markennamen Abil® (der Fa. Th. Goldschmidt), Alkasil® (der Fa. Rhône-Poulenc), Silicone Polyol Copolymer® (der Fa Genesee), Belsil® (der Fa. Wacker), Silwet® (der Fa. OSI) oder Dow Corning (der Fa. Dow Corning) erhältlich Verbindungen. Diese beinhalten Verbindungen mit den CAS-Nummern 64365-23-7; 68937-54-2; 68938-54-5; 68937-55-3. Eine geeignete, kommerziell erhältliche Verbindung ist Belsil ® DMC 6031.

Die im Folgenden aufgeführten Verbindungen (E), (G), (J) und (K), eignen sich aufgrund ihrer Silicongruppen bevorzugt zur Einführung hydrophober Gruppen (speziell Gruppen A)) bei der Herstellung der Verbindungen H1). Sofern die im Folgenden aufgeführten Verbindungen (E), (G), (J) und (K) auch bereits wenigstens eine hydrophile Gruppe (speziell Gruppen B))aufweisen, können sie auch als solche (d. h. ohne weitere Umsetzung) als Komponente H1) eingesetzt werden. Das gilt speziell für die Verbindungen (E), (G), (J) und (K), die wenigstens eine Gruppe (F) mit Alkylenoxid-Wiederholungseinheiten aufweisen.

Bevorzugte Siliconverbindungen H1) und Verbindungen zur Herstellung von Siliconverbindungen H1) sind Polysiloxane der allgemeinen Formel (E) 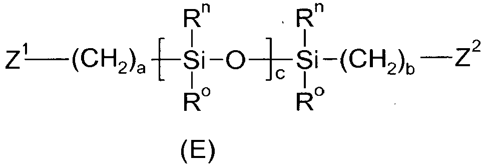 worin
a und b unabhängig voneinander für 1 bis 8 stehen,
c für 2 bis 1000 steht,
Rⁿ und R^{o} unabhängig voneinander für Alkyl, Cycloalkyl, Aryl oder Arylalkyl stehen,
Z¹ und Z² unabhängig voneinander für Reste der Formel (F)

-(OCH₂CH₂)ᵤ(OCH(CH₃)CH₂)ᵥ-X¹-H (F)

stehen, wobei

in der Formel (F) die Reihenfolge der Alkylenoxideinheiten beliebig ist,
u und v unabhängig voneinander für eine ganze Zahl von 0 bis 500 stehen, wobei die Summe aus u und v > 0 ist,

X¹ für O oder NR^{p} steht, worin R^{p} für Wasserstoff, Alkyl, Cycloalkyl oder Aryl steht.

Vorzugsweise ist in der Formel (E) die Summe aus u und v so gewählt, dass das Molekulargewicht der Polysiloxane H1) in einem Bereich von etwa 300 bis 30 000 liegt.

Bevorzugt liegt die Gesamtzahl der Alkylenoxideinheiten der Polysiloxane H1), d. h. die Summe aus u und v, in einem Bereich von etwa 3 bis 200, bevorzugt 5 bis 180.

Bevorzugt sind in den Verbindungen der Formel (E) die Reste Rⁿ und R^{o} unabhängig voneinander ausgewählt unter Methyl, Ethyl, Cyclohexyl, Phenyl und Benzyl. Besonders bevorzugt stehen Rⁿ und R^{o} beide für Methyl.

Ein Beispiel für geeignete Verbindungen der Formel (E) sind die Bis(polyethylenglycol)dimethicone der allgemeinen Formel (E.1) 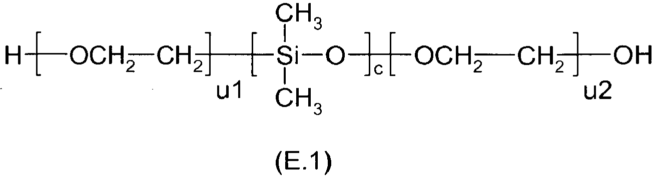 worin
c für eine ganze Zahl von 3 bis 500, bevorzugt 5 bis 250, steht und
u1 und u2 unabhängig voneinander für 2 bis 500, insbesondere 3 bis 250, speziell 5 bis 100, stehen.

Bevorzugte Siliconverbindungen H1) und Verbindungen zur Herstellung von Siliconverbindungen H1) sind weiterhin ausgewählt unter Polysiloxanen der allgemeinen Formel (G) 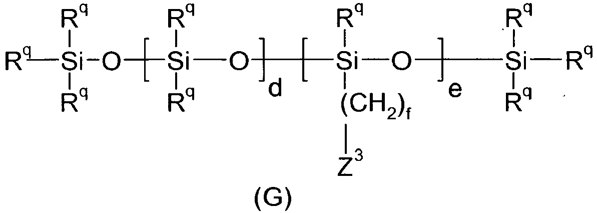 worin
die Reihenfolge der Siloxaneinheiten beliebig ist,
die Reste R^{q} jeweils unabhängig voneinander für Alkyl, Cycloalkyl oder Aryl stehen,
d für eine ganze Zahl von 2 bis 1000 steht,
e für eine ganze Zahl von 2 bis 100 steht,
f für eine ganze Zahl von 2 bis 8 steht, und
Z³ für OH, NHR^{r} oder einen Rest der Formel (F), wie zuvor definiert, steht,
wobei R^{r} für Wasserstoff, C₁-C₈-Alkyl, C₅-C₈-Cycloalkyl oder einen Rest der Formel -(CH₂)_{w}-NH₂ steht, wobei w für eine ganze Zahl von 1 bis 10, bevorzugt 2 bis 6, steht,
und Mischungen davon.

Ein Beispiel für geeignete Verbindungen der Formel (G) sind die ethoxilierten und/oder propoxylierten Polydimethylsiloxane der allgemeinen Formel (G.1) 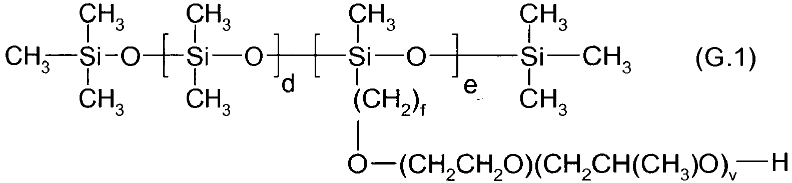 worin
die Reihenfolge der Siloxaneinheiten beliebig ist,
d für eine ganze Zahl von 2 bis 1000, bevorzugt 3 bis 500, insbesondere 5 bis 100, steht,
e für eine ganze Zahl von 2 bis 100, bevorzugt 3 bis 50, insbesondere 4 bis 20, steht,
f für eine ganze Zahl von 2 bis 8 steht, und
u und v unabhängig voneinander für eine ganze Zahl von 0 bis 500, bevorzugt 0 bis 250, steht, wobei die Summe aus u und v ≥ 1, bevorzugt ≥ 5, insbesondere ≥ 10, ist.

Geeignete Verbindungen der Formel G.1 sind unter der Bezeichnung Wacker-Belsil® DMC 6031 und Pluriol® ST 4005 (Fa. BASF SE) erhältlich.

Bevorzugte Siliconverbindungen H1) und Verbindungen zur Herstellung von Siliconverbindungen H1) sind weiterhin Polysiloxane mit sich wiederholenden Einheiten der allgemeinen Formel (J) 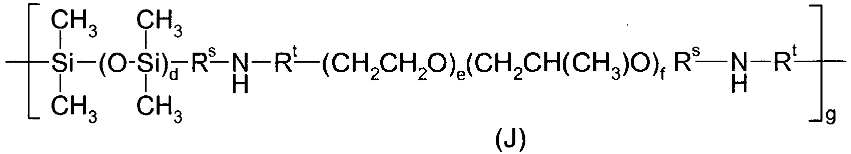 worin
d für eine ganze Zahl von 0 bis 100 steht,
g für eine ganze Zahl von 1 bis 8 steht,
R^{s} und R^{t} unabhängig voneinander für C₁-bis C₈-Alkylen stehen,
die Reihenfolge der Alkylenoxideinheiten beliebig ist und e und f unabhängig voneinander für eine ganze Zahl von 0 bis 200 stehen, wobei die Summe aus r und s > 0 ist.

Bevorzugt stehen in der Formel (J) R^{s} und R^{t} unabhängig voneinander für einen C₂- bis C₄-Alkylenrest.

Insbesondere stehen R^{s} und R^{t} unabhängig voneinander für einen C₂- bis C₃-Alkylenrest.

Vorzugsweise liegt das Molekulargewicht der Verbindung der Formel (J) in einem Bereich von etwa 300 bis 100 000.

Vorzugsweise steht in der Formel 1.3 d für eine ganze Zahl von 1 bis 20, wie z. B. 2 bis 10.

Bevorzugt liegt die Gesamtzahl der Alkylenoxideinheiten der Verbindung der Formel (J), d. h. die Summe aus e und f, in einem Bereich von etwa 3 bis 200, bevorzugt 5 bis 180.

Bevorzugt sind die Endgruppen der Polysiloxane mit Wiederholungseinheiten der allgemeinen Formel (J) ausgewählt unter (CH₃)₃SiO, H, C₁-C₈-Alkyl und Mischungen davon. Eine bevorzugte Alkylendgruppe ist Methyl.

Aminogruppenhaltige Verbindungen mit Wiederholungseinheiten der allgemeinen Formel (J) weisen bevorzugt eine Aminzahl in einem Bereich von etwa 2 bis 50, insbesondere 3 bis 20 auf.

Geeignete alkoxylierte Siloxanamine der Formel 1.3 sind z. B. in der WO-A-97/32917 beschrieben, auf die hier in vollem Umfang Bezug genommen wird. Kommerziell erhältliche Verbindungen sind z. B. die Silsoft-Marken der Momentive Performance Materials (vormals Fa. Witco), z. B. Silsoft A-843.

Bevorzugte Siliconverbindungen H1) und Verbindungen zur Herstellung von Siliconverbindungen H1) sind weiterhin Polysiloxane mit sich wiederholenden Einheiten der allgemeinen Formel (K) 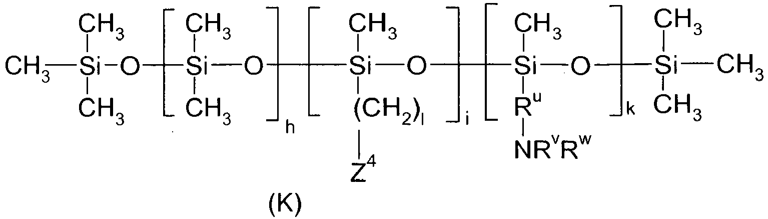 worin
R^{u} für einen C₁- bis C₈-Alkylenrest steht,
R^{v} und R^{w} unabhängig voneinander für Wasserstoff, C₁-C₈-Alkyl oder C₅-C₈-Cycloalkyl stehen,
die Reihenfolge der Siloxaneinheiten beliebig ist,
h, i und k unabhängig voneinander für 0 bis 100 stehen, wobei die Summe aus h, i und k mindestens 3 ist,
I für eine ganze Zahl von 2 bis 8 steht,
Z⁴ für einen Rest der Formel (F.1)

-(OCH₂CH₂)ᵤ(OCH(CH₃)CH₂)ᵥ-OR^{x} (F.1)

steht, wobei
in der Formel (F.1) die Reihenfolge der Alkylenoxideinheiten beliebig ist,
u und v unabhängig voneinander für eine ganze Zahl von 0 bis 500 stehen, wobei die Summe aus u und v > 0 ist,
R^{x} für Wasserstoff oder einen C₁-C₈-Alkylrest steht,
und Mischungen davon.

Bevorzugt steht in der Formel (K) der Rest R^{u} für einen C₂- bis C₄-Alkylenrest.

Bevorzugt stehen in der Formel (K) R^{v} und R^{w} unabhängig voneinander für Wasserstoff oder C₁- bis C₄-Alkyl.

Vorzugsweise wird die Summe aus h, I und k so gewählt, dass das Molekulargewicht der Verbindung der Formel (K) in einem Bereich von etwa 300 bis 100 000, bevorzugt 500 bis 50 000, liegt.

Bevorzugt liegt die Gesamtzahl der Alkylenoxideinheiten des Restes der Formel (F.1), d. h. die Summe aus u und v, in einem Bereich von etwa 3 bis 200, bevorzugt 5 bis 80.

Bevorzugt steht in der Formel (F.1) der Rest R^{x} für Wasserstoff oder C₁- bis C₄-Alkyl.

Eine geeignete Verbindung der Formel (K) ist z. B. Silsoft A-858 der Fa. der Momentive Performance Materials (vormals Fa. Witco).

Eine weitere bevorzugte Siliconverbindungen H1) und zur Herstellung von Siliconverbindungen H1) geeignete Verbindung ist die Verbindung mit der INCI-Bezeichnung Methoxy PEG/PPG-7/3 Aminopropyl Dimethicone. Diese ist kommerziell unter der Bezeichnung ABIL® Soft AF 100 von Evonik Industries erhältlich.

Geeignete Polysiloxandiamine zur Herstellung von Siliconverbindungen H1) sind Tegomer® A-Si 2122 (Mn = 900 g/mol) und Tegomer® A-Si 2322 (Mn = 2800 g/mol) der Evonik Industries (vormals Th. Goldschmidt).

### Hydrophile Gruppe von H1)

Bevorzugt ist in den Verbindungen H1) die hydrophile Gruppe ausgewählt unter
- Polyethylenoxid-haltigen Gruppen,
- Polypropylenoxid-haltigen Gruppen,
- Poly(ethylenoxid/propylenoxid)-haltigen Gruppen,
- Polyethylenimin-haltigen Gruppen,
- Polysorbat-haltigen Gruppen,
- Polyglycerin-haltigen Gruppen,
- Polyvinylpyrrolidon-haltigen Gruppen,
und Kombinationen davon.

Speziell ist die hydrophile Gruppe B in den Verbindungen der allgemeinen Formeln (I), (II) oder (III) ausgewählt unter
- Polyethylenoxid-haltigen Gruppen,
- Polypropylenoxid-haltigen Gruppen,
- Poly(ethylenoxid/propylenoxid)-haltigen Gruppen,
- Polyethylenimin-haltigen Gruppen,
- Polysorbat-haltigen Gruppen,
- Polyglycerin-haltigen Gruppen,
- Polyvinylpyrrolidon-haltigen Gruppen,
und Kombinationen davon.

Als hydrophile Gruppe in den Verbindungen H1), speziell als hydrophile Gruppe A in den Verbindungen der allgemeinen Formeln (I), (II) oder (III), eignen sich bevorzugt Polyether-haltige Gruppen, spezieller Polyethylenoxid-haltige Gruppen (= Polyethylenglykol-haltige Gruppen), Polypropylenoxid-haltige Gruppen (= Polypropylenglykolhaltige Gruppen) und Copolymere, deren Wiederholungseinheiten sich von Polyethylenoxid und Polypropylenoxid ableiten. Bevorzugt sind Polyether-haltige Gruppen mit einem zahlenmittleren Molekulargewicht im Bereich von etwa 300 bis 10 000, bevorzugt 400 bis 5000. Poly(ethylenoxid/propylenoxid)-haltigen Gruppen können die Ethylenoxideinheiten und die Propylenoxideinheiten statistisch verteilt oder in Form von Blöcken einpolymerisiert enthalten.

Die Herstellung von Verbindungen H1) mit Polyethergruppen geht vorzugsweise aus von Polyetherdiolen oder Polyethermonoalkoholen. Bevorzugte Polyethermonoalkohole sind die Polyalkylenglykolmonoalkylether. Bevorzugte Polyalkylenglykolmonoalkylether sind Polyalkylenglykolmonomethylether und Polyalkylenglykolmonoethylether. Besonders bevorzugt sind Polyethylenglykolmonomethylether.

Bevorzugt für die Herstellung von Verbindungen H1) sind Polyethylenglykolmonomethylether. Geeignete Polyethylenglykolmonomethylether sind die Pluriol ® A ... E Marken der BASF SE (Molekulargewicht in Klammern), speziell Pluriol A 350 E (350 g/mol), Pluriol A 500 E (500 g/mol), Pluriol A 750 E (750 g/mol), Pluriol A 760 E (750 g/mol), Pluriol A 1000 E (1000 g/mol), Pluriol A 1020 E* (1000 g/mol), Pluriol A 2000 E (2000 g/mol), Pluriol A 3010 E* (3000 g/mol) und Pluriol A 5010 E* (5000 g/mol).

Bevorzugt für die Herstellung von Verbindungen H1) sind Polyetherdiole in Form von Polyethylenoxiddiolen (Polyethylenglykolen). Kommerziell sind sie als die Pluriol® E-Marken der BASF SE erhältlich. Dazu zählen:
Pluriol E 200, 300, 400, 600, 1000, 1500, 3400, 4000, 6000, 8000, 9000 und 12000.

Geeignet für die Herstellung von Verbindungen H1) sind Polyetherdiole in Form von EO/PO/EO-Triblockcopolymeren. Kommerziell sind sie als die Pluronic® PE-Marken der BASF SE erhältlich. Dazu zählen:
(Bezeichnung/ EO-Gehalt in %/ Molekulargewicht)
Pluronic PE 3100 / ca. 10 / ca. 1000
Pluronic PE 3500 / ca. 50 / ca. 1900
Pluronic PE 4300 / ca. 30 / ca. 1750
Pluronic PE 6100 / ca. 10 / ca. 2000
Pluronic PE 6120 / ca. 12 / ca. 2100
Pluronic PE 6200 / ca. 20 / ca. 2450
Pluronic PE 6400 / ca. 40 / ca. 2900
Pluronic PE 6800 / ca. 80 / ca. 8000
Pluronic PE 7400 / ca. 40 / ca. 3500
Pluronic PE 8100 / ca. 10 / ca. 2600
Pluronic PE 9200 / ca. 20 / ca. 3650
Pluronic PE 9400 / ca. 40 / ca. 4600
Pluronic PE 10100 / ca. 10 / ca. 3500
Pluronic PE 10300 / ca. 30 / ca. 4950
Pluronic PE 10400 / ca. 40 / ca. 5900
Pluronic PE 10500 / ca. 50 / ca. 6500

Geeignet für die Herstellung von Verbindungen H1) sind Polyetherdiole in Form von PO/EO/PO-Triblockcopolymeren. Kommerziell sind sie als die Pluronic® RPE-Marken der BASF SE erhältlich. Dazu zählen:
(Bezeichnung/ EO-Gehalt in %/ Molekulargewicht)
Pluronic RPE 1720 / ca. 20 / ca. 2150
Pluronic RPE 1740 / ca. 40 / ca. 2650
Pluronic RPE 2035 / ca. 35 / ca. 4100
Pluronic RPE 2520 / ca. 20 / ca. 3100
Pluronic RPE 2525 / ca. 25 / ca. 2000
Pluronic RPE 3110 / ca. 10 / ca. 3500

Geeignet als hydrophile Gruppe für die Herstellung von Verbindungen H1) sind auch Polyether, die eine oder zwei endständige Aminogruppen aufweisen. Diese sind durch Aminierung der zuvor genannten Polyethylenoxid-haltigen Gruppen, Polypropylenoxid-haltigen Gruppen und Poly(ethylenoxid/propylenoxid)-haltigen Gruppen mit Ammoniak herstellbar.

Geeignet als hydrophile Gruppe für die Herstellung von Verbindungen H1) sind auch Polyethylenimin-haltigen Gruppen. Diese weisen vorzugsweise ein zahlenmittleres Molekulargewicht im Bereich von etwa 300 bis 10 000, bevorzugt 400 bis 5000, auf.

Geeignet als hydrophile Gruppe für die Herstellung von Verbindungen H1) sind auch Polysorbat-haltige Gruppen. Polysorbate werden durch Veresterung von Sorbitol mit einer Fettsäure und anschließende Ethoxilierung erhalten. Die Produkte sind kommerziell erhältlich, z. B. unter der Bezeichnung Tween.

Geeignet als hydrophile Gruppe für die Herstellung von Verbindungen H1) sind auch Polyglycerin-haltige Gruppen. Polyglycerinmischungen können durch alkalisch katalysierte Kondensation von Glycerin bei erhöhten Temperaturen (Fette, Seifen, Anstrichmittel, 88. Jahrgang, Nr. 3, 1986, S. 101-106 (4)) oder durch Umsetzung von Glycerin mit Epichlorhydrin in Gegenwart eines sauren Katalysators bei erhöhten Temperaturen (DE-A 38 42 692) hergestellt werden.

Eine typische geeignete Polyglycerinmischung hat die folgende Zusammensetzung: 0 bis 5 Gew.-% Glycerin,
20 bis 40 Gew.-% Diglycerin,
35 bis 55 Gew.-% Triglycerin,
10 bis 20 Gew.-% Tetraglycerin,
5 bis 10 Gew.-% Pentaglycerin,
1 bis 5 Gew.-% Hexaglycerin und
0 bis 5 Gew.-% höhere Polyglycerine.

Die Herstellung von Verbindungen H1) erfolgt vorzugsweise ausgehend von Polyglycerinen durch Veresterung mit wenigstens einer Verbindung, die wenigstens eine Carbonsäuregruppe oder ein zur Veresterung befähigtes Derivat davon und wenigstens eine hydrophobe Gruppe aufweist. Dazu zählen z. B. Fettsäuren und Fettsäuremischungen. Dazu zählen bevorzugt Polyisobutene mit mindestens einer Carbonsäureendgruppe oder ein Derivat davon.

Die Polyglycerinester stellt man durch Veresterungsreaktion zwischen den entsprechenden Polyglycerinmischungen und der gewünschten Carbonsäure oder Carbonsäuremischung oder einem Derivat davon nach den üblichen Methoden her. Geeignete Derivate sind die Anhydride, Halogenide und Ester mit C₁-C₄-Alkanolen. Üblicherweise erfolgt die Veresterung in Gegenwart eines sauren oder basischen Veresterungskatalysators wie hypophosphoriger Säure, phosphoriger Säure, Schwefelsäure, p-Toluolsulfonsäure, Citronensäure, Natriummethylat, Zinnoxid oder einer Seife.

Polyglycerine und Polyglycerinester und deren Herstellung sind in der DE 40 23 593 A1 beschrieben, auf die hier Bezug genommen wird.

Die Herstellung von Verbindungen H1) mit Polyvinylpyrrolidongruppen geht vorzugsweise von Polyvinylpyrrolidon-Homopolymeren und Copolymeren, die N-Vinylpyrrolidon und ein weiteres davon verschiedenes ethylenisch ungesättigtes Monomer einpolymerisiert enthalten. Geeignete N-Vinylpyrrolidon-Copolymere sind ganz allgemein neutrale, anionische, kationische und amphotere Polymere.
Bevorzugte N-Vinylpyrrolidon-Copolymere sind ausgewählt unter
Copolymeren aus N-Vinylpyrrolidon und Vinylacetat,
Copolymeren aus N-Vinylpyrrolidon und Vinylpropionat,
Copolymeren aus N-Vinylpyrrolidon, Vinylacetat und Vinylpropionat,
Copolymeren aus N-Vinylpyrrolidon und Vinylacrylat,
Copolymeren aus N-Vinylpyrrolidon, Ethylmethacrylat und Methacrylsäure, Copolymeren aus N-Vinylpyrrolidon und N-Vinylimidazol und deren durch Protonierung und/oder Quaternisierung erhaltenen Derivaten,
Copolymeren aus N-Vinylpyrrolidon und Dimethylaminoethyl-methacrylat und deren durch Protonierung und/oder Quaternisierung erhaltenen Derivaten,
Copolymeren aus N-Vinylpyrrolidon, N-Vinylcaprolactam und N-Vinylimidazol und deren durch Protonierung und/oder Quaternisierung erhaltenen Derivaten.

In einer speziellen Ausführung geht die Herstellung von Verbindungen H1) mit Polyvinylpyrrolidongruppen aus von Polyvinylpyrrolidon-Homopolymeren und Polyvinylpyrrolidon-Copolymeren, die jeweils wenigstens eine gegenüber Isocyanatgruppen reaktive Gruppe aufweisen.

### Linker X

Bevorzugt weisen die Verbindungen H1) wenigstens eine verbrückende Gruppe X) auf, die wenigstens eine hydrophobe Gruppe und wenigstens eine hydrophile Gruppe miteinander verbindet.

Ganz allgemein ist die verbrückende Gruppe X) ausgewählt unter einer chemischen Bindung oder einem divalenten oder polyvalenten Rest, an den wenigstens eine hydrophile Gruppe und wenigstens eine hydrophobe Gruppe gebunden sind.

Bevorzugt ist die Verbindung H1, ausgewählt ist unter Verbindungen der allgemeinen Formeln (I), (II) oder (III), wie zuvor definiert. Dann ist die Gruppe X vorzugsweise ausgewählt ist unter Gruppen der Formeln: 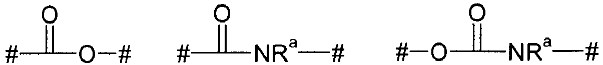 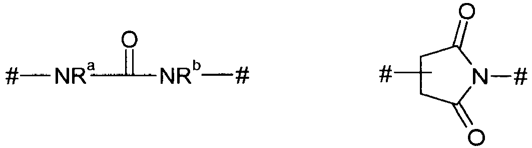 wobei # in jeder Gruppe X einmal für eine Bindungsstelle zu einer Gruppe A und einmal für eine Bindungsstelle zu einer Gruppe B steht,

R^{a} und R^{b} unabhängig voneinander für Wasserstoff, Alkyl, Cycloalkyl, Heterocycloalkyl, Aryl oder Hetaryl stehen.

Erfindungsgemäß ist die Verbindung mit Blockstruktur H1) ausgewählt unter
- Polyisobutenylalkoholalkoxilaten,
- Polyisobutenylaminalkoxilaten,
- Umsetzungsprodukten aus wenigstens einem Polyisobutenen mit mindestens einer Carbonsäureendgruppe oder einem Derivat davon und wenigstens einem Polyalkylenoxid mit einer terminalen gegenüber Anhydridgruppen reaktiven Gruppe,
- Siliconverbindungen, die wenigstens eine Polyethergruppe aufweisen,
- Umsetzungsprodukten aus wenigstens einer Verbindung, die mindestens eine hydrophobe Gruppe und mindestens eine gegenüber Isocyanatgruppen reaktive Gruppe umfasst, wenigstens einer Verbindung, die mindestens eine hydrophile Gruppe und mindestens eine gegenüber Isocyanatgruppen reaktive Gruppe umfasst, und wenigstens einem Polyisocyanat.

### Urethanverbindung H1)

Eine bevorzugte Ausführungsform sind Urethanverbindungen H1).

Bevorzugt ist eine Urethanverbindung H1), die
p1) wenigstens eine Verbindung, die mindestens eine hydrophobe Gruppe und mindestens eine gegenüber Isocyanatgruppen reaktive Gruppe umfasst,
p2) wenigstens eine Verbindung, die mindestens eine hydrophile Gruppe und mindestens eine gegenüber Isocyanatgruppen reaktive Gruppe, und
p3) wenigstens ein Polyisocyanat,
   eingebaut enthält.

Bezüglich zur Herstellung von Urethanverbindungen H1) geeigneter und bevorzugter Verbindungen mit mindestens eine hydrophoben Gruppe p1) und Verbindungen mit mindestens einer hydrophilen Gruppe p2) wird auf die vorherigen Angaben zu hydrophoben und hydrophilen Gruppen (speziell Gruppen A) und B)) und zu Verbindungen, die diese Gruppen aufweisen, in vollem Umfang Bezug genommen.

Geeignete Polyisocyanate p3) sind ausgewählt unter Verbindungen mit 2 bis 5 Isocyanatgruppen, Isocyanatpräpolymeren mit mittleren Anzahl von 2 bis 5 Isocyanatgruppen, und Mischungen davon. Dazu zählen z. B. aliphatische, cycloaliphatische und aromatische Di-, Tri- und Polyisocyanate. Geeignete Diisocyanate p3) sind z. B. Tetramethylendiisocyanat, Hexamethylendiisocyanat, 2,3,3-Trimethylhexamethylendiisocyanat, 1,4-Cyclohexylendüsocyanat, Isophorondiisocyanat, 1,4-Phenylendiisocyanat, 2,4- und 2,6-Toluylendiisocyanat und deren Isomerengemische (z. B. 80 % 2,4- und 20 % 2,6-Isomer), 1,5-Naphthylendiisocyanat, 2,4- und 4,4'-Diphenylmethandiisocyanat. Ein geeignetes Triisocyanat p3) ist z. B. Triphenylmethan-4,4',4"-triisocyanat. Weiterhin geeignet sind Isocyanatpräpolymere und Polyisocyanate, die durch Addition der zuvor genannten Isocyanate an polyfunktionelle Hydroxyl- oder Amingruppen-haltige Verbindungen erhältlich sind. Weiterhin geeignet sind Polyisocyanate, die durch Biuret-, Allophanat- oder Isocyanuratbildung entstehen.

Vorzugsweise umfasst die Polyisocyanat-Komponente p3) wenigstens ein Diisocyanat mit zwei unterschiedlich reaktiven Isocyanatgruppen. Besonders bevorzugt umfasst die Polyisocyanat-Komponente p3) dann Isophorondiisocyanat und dessen Biurete, Allophanate und/oder Isocyanurate. Des Weiteren bevorzugt umfasst die Polyisocyanat-Komponente p3) Hexamethylendiisocyanat. Insbesondere besteht die Polyisocyanat-Komponente p3) nur aus Isophorondiisocyanat oder nur aus Hexamethylendiisocyanat oder aus einem Gemisch aus Isophorondiisocyanat und Hexamethylendiisocyanat.

In einer weiteren Ausführungsform enthalten die Urethanverbindungen H1) zusätzlich wenigstens eine Verbindung p4) eingebaut, die vorzugsweise ausgewählt ist unter Verbindungen mit einem Molekulargewicht im Bereich von 56 bis 280 g/mol, die zwei gegenüber Isocyanatgruppen reaktive Gruppen pro Molekül enthalten. Geeignete Verbindungen p4) sind z. B. Diole, Diamine, Aminoalkohole und Mischungen davon.

Bevorzugt werden als Komponente p4) Diole eingesetzt, deren Molekulargewicht in einem Bereich von etwa 62 bis 286 g/mol liegt. Dazu zählen z. B. Diole mit 2 bis 18 Kohlenstoffatomen, vorzugsweise 2 bis 10 Kohlenstoffatomen, wie 1,2-Ethandiol, 1,3-Propandiol, 1,4-Butandiol, 1,6-Hexandiol, 1,5-Pentandiol, 1,10-Decandiol, 2-Methyl-1,3-propandiol, 2,2-Dimethyl-1,3-propandiol, Di-, Tri-, Tetra-, Penta- und Hexaethylenglykol, Neopentylglykol, Cyclohexandimethylol und Mischungen davon. Besonders bevorzugt ist Neopentylglykol.

Bevorzugte Aminoalkohole p4) sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol, N-Methyldiethanolamin, etc.

Bevorzugte Diamine p4) sind z. B. Ethylendiamin, Propylendiamin, 1,4-Diaminobutan, 1,5-Diaminopentan, 1,6-Diaminohexan (Hexamethylendiamin), Isophorondiamin und Mischungen davon.

Die als Komponente p4) genannten Verbindungen können einzeln oder in Mischungen eingesetzt werden. Besonders bevorzugt werden 1,2-Ethandiol, 1,4-Butandiol, 1,6-Hexandiol, Neopentylglykol, Diethylenglykol, Cyclohexandimethylol, N-Methyldiethänolamin und Mischungen davon eingesetzt.

In einer weiteren Ausführungsform enthalten die Urethanverbindungen H1) zusätzlich wenigstens eine Verbindung p5) eingebaut, die vorzugsweise ausgewählt ist unter Verbindungen mit einem Molekulargewicht im Bereich von 56 bis 280 g/mol, die eine gegenüber Isocyanatgruppen reaktive Gruppe pro Molekül enthalten. Derartige Verbindungen mit einer gegenüber Isocyanatgruppen reaktive Gruppe pro Molekül werden auch als Abstopper bezeichnet. Geeignete Verbindungen p5) sind z. B. Monoalkohole sowie Amine und Aminoalkohole, die nur eine gegenüber NCO-Gruppen reaktive Gruppe aufweisen. Dazu zählen Methanol, Ethanol, n-Propanol, etc.

Eine spezielle Ausführungsform ist eine Urethanverbindungen H1), die
p1) wenigstens eine Verbindung, die mindestens eine hydrophobe Gruppe und mindestens eine gegenüber Isocyanatgruppen reaktive Gruppe umfasst, ausgewählt unter Polyisobutenen mit mindestens einer stickstoffhaltigen Endgruppe, Polytetrahydrofuranen und Mischungen davon,
p2) wenigstens eine Verbindung, die mindestens eine hydrophile Gruppe und mindestens eine gegenüber Isocyanatgruppen reaktive Gruppe umfasst, ausgewählt unter Polyethylenoxiden , Polyvinylpyrrolidon und Mischungen davon,
p3) Hexamethylendiisocyanat und/oder Isophorondiisocyanat,
p4) gegebenenfalls wenigstens eine Verbindung, die ausgewählt ist unter Neopentylglykol, N-Methyldiethanolamin, 1,5-Diaminopentan, 1,6-Diaminohexan, Isophorondiamin und Mischungen davon,
   eingebaut enthält.

Die Herstellung der Urethanverbindungen H1) kann nach üblichen Verfahren erfolgen. Bevorzugt erfolgt die Herstellung der Urethanverbindungen H1 durch ein zweistufiges Verfahren. Die Umsetzung der Komponenten erfolgt dabei vorzugsweise so, dass die erhaltenen Urethanverbindungen wenigstens eine hydrophobe Verbindung p1) und wenigstens eine hydrophile Verbindung p2) eingebaut enthalten.

Gegenstand der Erfindung ist auch ein Verfahren zur Herstellung einer Urethanverbindung H1), die
p1) wenigstens eine Verbindung, die mindestens eine hydrophobe Gruppe und mindestens eine gegenüber Isocyanatgruppen reaktive Gruppe umfasst,
p2) wenigstens eine Verbindung, die mindestens eine hydrophile Gruppe und mindestens eine gegenüber Isocyanatgruppen reaktive Gruppe umfasst, und
p3) wenigstens ein Polyisocyanat,
p4) wenigstens eine Verbindung, die ausgewählt ist unter Verbindungen mit einem Molekulargewicht im Bereich von 56 bis 280 g/mol, die zwei gegenüber Isocyanatgruppen reaktive Gruppen pro Molekül enthalten,
p5) wenigstens eine Verbindung, die ausgewählt ist unter Verbindungen mit einem Molekulargewicht im Bereich von 56 bis 280 g/mol, die eine gegenüber Isocyanatgruppen reaktive Gruppe pro Molekül enthalten,
eingebaut enthält, bei dem man
i) in einer ersten Stufe die hydrophoben Verbindungen p1), die Polyisocyanate p3) sowie, falls vorhanden, gegebenenfalls wenigstens einen Teil der Verbindungen p4) und/oder p5) zu einem Isocyanatgruppen-haltigen Präpolymer umsetzt, und
ii) in einer zweiten Stufe das in i) erhaltene Präpolymer mit den hydrophilen Verbindungen p2) und, falls vorhanden, den nicht bereits in Schritt i) eingesetzten Verbindungen p4) und/oder p5) umsetzt.

In der ersten Stufe i) wird zunächst aus den hydrophoben Verbindungen p1) und den Polyisocyanaten p3) ein NCO-Gruppen-haltiges Präpolymer hergestellt. Gewünschtenfalls können in dieser Stufe, falls vorhanden, auch die Verbindungen der Komponente p4) und/oder Abstopper p5) teilweise oder vollständig zur Herstellung des Präpolymers eingesetzt werden. Abstopper p5) werden jedoch vorzugsweise in der stufe ii) eingesetzt. In jedem Falle wird durch geeignete Wahl der Menge der Komponente p3) sichergestellt, dass in der Stufe i) ein Isocyanatgruppen-haltiges Präpolymer erhalten wird. Das Verhältnis von NCO-Äquivalenten der Komponente p3) zu Äquivalenten aktiven Wasserstoffatomen der Komponenten p1) und, falls vorhanden, p4) und p5) liegt vorzugsweise in einem Bereich von etwa 1 : 1 bis 3 : 1, besonders bevorzugt 1,01 : 1 bis 2,5 : 1, insbesondere 1,05 : 1 bis 2 : 1.

Vorzugsweise erfolgt die Reaktion in beiden Stufen i) und ii) unter einer Inertgasatmosphäre, wie z. B. unter Stickstoff. Des Weiteren erfolgt die Reaktion vorzugsweise in beiden Stufen i) und ii) bei Umgebungsdruck oder unter erhöhtem Druck.

Die Reaktion wird in der Stufe i) vorzugsweise in einem geeigneten inerten Lösungsmittel oder Lösungsmittelgemisch durchzuführen. Geeignete Lösungsmittel sind Kohlenwasserstoffe und Kohlenwasserstoffgemische, wie Pentan, Hexan, Cyclohexan, Dekalin, Ligroin, Petrolether, etc. Geeignet sind auch aromatische Kohlenwasserstoffe, wie Benzol, Toluol oder Xylol. Geeignete Lösungsmittel sind auch aprotisch polare Lösungsmittel, z. B. Tetrahydrofuran, Essigsäureethylester, N-Methylpyrrolidon, Dimethylformamid und bevorzugt Ketone, wie Aceton und, Methylethylketon.

Die Reaktionstemperatur in Schritt i) liegt vorzugsweise in einem Bereich von etwa 0 bis 120 °C, besonders bevorzugt 5 bis 90 °C. Enthalten die Komponenten p1) und, falls vorhanden, p4) und/oder p5) Amingruppen-haltige Verbindungen, so liegt die Reaktionstemperatur bevorzugt in einem Bereich von etwa 0 bis 60 °C, besonders bevorzugt von 10 bis 40 °C. Im Falle des Einsatzes von Amingruppen-haltigen Komponenten kann die Reaktion gewünschtenfalls auch in einem Lösungsmittel oder Lösungsmittelgemisch erfolgen, welches aktive Wasserstoffatome aufweisen kann. Neben den zuvor genannten werden dann bevorzugt kosmetisch akzeptable Lösungsmittel, vorzugsweise Alkohole, wie Ethanol und Isopropanol sowie Gemische aus Alkoholen und Wasser eingesetzt.

Die in Schritt i) erhaltenen NCO-Gruppen-haltigen Präpolymere können gewünschtenfalls vor der weiteren Umsetzung in Schritt ii) einer Isolierung und/oder Reinigung nach üblichen, dem Fachmann bekannten Verfahren unterzogen werden. Vorzugsweise erfolgt die Herstellung der Präpolymere und die Herstellung der Urethanverbindungen H1) daraus ohne Isolierung eines Zwischenprodukts.

Die Herstellung der erfindungsgemäßen Urethanverbindung in Schritt ii) erfolgt durch Umsetzung der in Schritt i) erhaltenen Präpolymere mit den hydrophilen Verbindungen p2) sowie gegebenenfalls Verbindungen der Komponenten p4) und/oder p5). Dabei liegt das Verhältnis von NCO-Äquivalenten der Präpolymere zu Äquivalenten aktiven Wasserstoffatomen der Komponenten p2) und, falls vorhanden, p4) und p5) in einem Bereich von etwa 0,6 : 1 bis 1,4 : 1, bevorzugt 0,8 : 1 bis 1,2 : 1, insbesondere 0,9 : 1 bis 1,1 : 1.

Die Reaktion erfolgt vorzugsweise auch in der zweiten Stufe ii) in einem der zuvor genannten Lösungsmittel, vorzugsweise in einem kosmetisch akzeptablen Lösungsmittel. Sofern die Anbindung der Komponente p2) über Amingruppen erfolgt, und auch die optional eingesetzten Verbindungen p4) und p5) Amingruppen als NCO-reaktive Gruppen aufweisen, kann die Reaktion in Alkoholen und Alkohol/Wasser-Gemischen durchgeführt werden. Bevorzugt sind Ethanol, Isopropanol, deren Gemische und Gemische dieser Alkohole mit Wasser. Die Reaktionstemperatur in Schritt ii) liegt dann bevorzugt in einem Bereich von etwa 0 bis 60 °C, besonders bevorzugt 10 bis 40 °C. Weisen die resultierenden Urethanverbindungen noch freie Isocyanatgruppen auf, so werden diese abschließend durch Zusatz von Verbindungen p4), p5) oder von Wasser inaktiviert. Bevorzugt wird zur Inaktivierung freier Isocyanatgruppen z. B. 2-Amino-2-methyl-1-propanol verwendet.

### Polyisobutenylalkoholalkoxilate und Polyisobutenylaminalkoxilate H1)

Eine spezielle Ausführung von Verbindungen H1) sind die Alkoxilate von Polyisobutenylalkoholen und Polyisobutenylaminen mit Ethylenoxid und/oder Propylenoxid.

Der Alkoxilierungsgrad, d. h. die mittlere Kettenlänge der Polyetherketten der Alkoxilate, kann durch das Molmengenverhältnis von Alkohol oder Amin zu Alkylenoxid bestimmt werden. Bevorzugt sind Alkoholalkoxilate mit etwa 1 bis 1000, bevorzugt etwa 2 bis 500, insbesondere 3 bis 100 Alkylenoxideinheiten. In Abhängigkeit von den für die Umsetzung gewählten Einsatzmengen an Alkylenoxid(en) sowie den Reaktionsbedingungen ergibt sich der jeweilige Alkoxilierungsgrad. Hierbei handelt es sich in der Regel um einen statistischen Mittelwert, da die Anzahl von Alkylenoxid-Einheiten der aus der Umsetzung resultierenden Alkoholalkoxilate variiert.

Eine bevorzugte Ausführung sind Ethylenoxid-Homoalkoxilate oder 1,2-Propylenoxid-Homoalkoxilate. Besonders bevorzugt sind Ethylenoxid-Homoalkoxilate.

Ein weiterer Typ zu verwendender Polyisobutenylalkoholalkoxilate oder Polyisobutenylaminalkoxilate basiert auf Ethylenoxid und 1,2-Propylenoxid. Dabei können die Alkylenoxide gewünschtenfalls statistisch eingebaut werden. Dazu können die Alkylenoxide in Form von Gemischen zur Alkoxilierung eingesetzt werden. Bevorzugt werden die Alkylenoxid-Einheiten blockartig angeordnet, so dass sich wenigstens zwei unterschiedliche Alkylenoxid-Blöcke ergeben, die jeweils aus mehreren Einheiten gleicher Alkylenoxide gebildet werden. Sofern derartige Blockalkoxilate verwendet werden, ist es bevorzugt, dass sich der Alkylenoxidteil aus 2 bis 5, bevorzugt 2 oder 3 und insbesondere aus 2 Blöcken zusammensetzt. Die Blockcopolyether können durch Umsetzung eines der zuvor beschriebenen Polyisobutenylalkohole oder Polyisobutenylamine mit einem ersten Alkylenoxid, nachfolgende Reaktion mit einem davon verschiedenen zweiten Alkylenoxid und gegebenenfalls weitere sequentielle Addition jeweils eines von dem gerade vorher addierten Alkylenoxid verschiedenen Alkylenoxids erhalten werden, bis die gewünschte Blockstruktur erzielt ist.

Bevorzugt sind EO-PO-Coalkoxilate, bei denen das molare Verhältnis von EO zu PO bevorzugt in einem Bereich von 10 : 1 bis 1 : 10 liegt. Bevorzugt wird das Ethylenoxid in der gleichen molaren Menge wie das Propylenoxid oder wird Ethylenoxid in einem molaren Überschuss über Propylenoxid eingesetzt. Bevorzugt sind dann EO-PO-Coalkoxilate, bei denen das molare Verhältnis von EO zu PO bevorzugt in einem Bereich von 1 : 1 bis 10 : 1 und insbesondere 1,5 : 1 bis 5 : 1 liegt.

Die Umsetzung der Alkohole bzw. Amine mit dem/den Alkylenoxid(en) erfolgt nach üblichen, dem Fachmann bekannten Verfahren und in dafür üblichen Apparaturen, die für das Arbeiten unter Druck ausgestattet sind.

Die Alkoxylierung kann durch starke Basen, wie Alkalihydroxide und Erdalkalihydroxide, Brönstedsäuren oder Lewissäuren, wie AlCl₃, BF₃ etc. katalysiert werden. Für eng verteilte Alkoholoxylate können Katalysatoren wie Hydrotalcit oder Doppelmetallcyanide (DMC) verwendet werden.

Die Alkoxilierung wird vorzugsweise bei Temperaturen von etwa 50 bis 250 °C, besonders bevorzugt von 90 bis 200 °C durchgeführt. Das Alkylenoxid oder die Mischung verschiedener Alkylenoxide wird der Mischung aus erfindungsgemäß eingesetztem Alkohol oder Amin und Katalysator unter dem bei der gewählten Reaktionstemperatur herrschenden Dampfdruck des Alkylenoxidgemischs oder einem höheren Druck zugeführt.

Gewünschtenfalls kann das Alkylenoxid mit einem Inertgas (beispielsweise Edelgase, Stickstoff, CO₂) bis zu 99,9 % verdünnt werden. Dadurch wird insbesondere im Fall des Ethylenoxids eine zusätzliche Sicherheit gegen den Gasphasenzerfall dieses Alkylenoxids gegeben, wobei bei dieser Ausführungsform auch ein weiteres Alkylenoxid, beispielsweise Propylenoxid, als Inertgas im Sinne der Erfindung verwendet werden kann.

Geeignete Alkoxilierungsbedingungen sind auch in Nikolaus Schönfeldt, Grenzflächenaktive Äthylenoxid-Addukte, Wissenschaftliche Verlagsgesellschaft mbH, Stuttgart 1984, beschrieben. In der Regel wird die Alkoxylierung in Gegenwart des Katalysators ohne Zusatz eines Lösungsmittels durchgeführt. Die Alkoxylierung kann jedoch auch unter Mitverwendung eines unter den Alkoxilierungsbedingungen inerten Lösungsmittels durchgeführt werden.

In einer geeigneten Ausführung wird die Alkoxylierung durch wenigstens eine starke Base katalysiert. Geeignete starke Basen sind z. B. Alkalialkoholate, Alkalihydroxide, Erdalkalioxide oder Erdalkalihydroxide. Die Basen werden in der Regel in einer Menge von 0,01 bis 1 Gew.-% bezogen auf die Menge des zu alkoxilierenden Alkohols eingesetzt (vgl. G. Gee et al., J. Chem. Soc. (1961), S. 1345; B. Wojtech, Makromol. Chem. 66, (1966), S. 180).

Auch eine saure Katalyse der Alkoxilierungsreaktion ist möglich. Neben Brönstedsäuren eignen sich auch Lewissäuren wie zum Beispiel AlCl₃, BF₃, BF₃-Dietherate, BF₃ x H₃PO₄, SbCl₄ x 2 H₂O, Hydrotalcit (vgl. P. H. Plesch, The Chemistry of Cationic Polymerization, Pergamon Press, New York(1963)).

In einer weiteren Ausführungsform wird die Alkoxilierung in Gegenwart einer Doppelmetallcyanid-Verbindung als Katalysator durchgeführt. Als DMC-Verbindung können prinzipiell alle dem Fachmann bekannten geeigneten Verbindungen verwendet werden. Als Katalysator geeignete DMC-Verbindungen sind beispielsweise in der WO 99/16775 und der DE-A-101 17 273 beschrieben.

### Umsetzungsprodukte H1) von PIB mit Carbonsäureendgruppe mit Polyalkylenoxidalkoholen oder Polyalkylenoxidaminen

Eine spezielle Ausführung von Verbindungen H1) sind die Umsetzungsprodukte aus wenigstens einem Polyisobutenen mit mindestens einer Carbonsäureendgruppe oder einem Derivat davon und wenigstens einem Polyalkylenoxid mit einer terminalen reaktiven Gruppe, die gegenüber Carbonsäuregruppen, Carbonsäureanhydridgruppen oder anders derivatisierten Carbonsäuregruppen reaktiv ist.

Bezüglich geeigneter Polyisobutene zur Herstellung dieser Umsetzungsprodukte H1) mit mindestens einer Carbonsäureendgruppe oder einer durch Veresterung, Amidierung oder Imidierung erhaltenen Gruppe wird auf die vorherige Offenbarung zu diesen Polyisobutenen in vollem Umfang Bezug genommen. Weiterhin wird bezüglich geeigneter Polyalkylenoxide mit wenigstens einer terminalen OH-Gruppe oder wenigstens einer terminalen primären oder sekundären Aminogruppe auf die vorherige Offenbarung zu diesen Polyalkylenoxiden in vollem Umfang Bezug genommen.

Verfahren zur Veresterung, Amidierung oder Imidierung von Polyisobutenen mit mindestens einer Carbonsäureendgruppe oder einem Derivat davon sind prinzipiell bekannt und z. B. in der EP 0 744 413 A2 und der darin in Bezug genommenen US 5,137,980 beschrieben.

Bevorzugt wird zur Umsetzung ein Polyisobutenylanhydrid, speziell PIBSA eingesetzt. Des Weiteren bevorzugt wird zur Umsetzung eine Verbindung mit einer hydrophilen Gruppe eingesetzt, die ausgewählt ist unter Polyethylenoxiddiolen, Polyethylenoxidmonoalkoholen, Polypropylenoxiddiolen, Polypropylenoxidmonoalkoholen, Poly-(ethylenoxid/propylenoxid)diolen, Poly(ethylenoxid/propylenoxid)monoalkoholen, Polyethylenoxiddiaminen, Polyethylenoxidmonoaminen, Polypropylenoxiddiaminen, Polypropylenoxidmonoaminen, Poly(ethylenoxid/propylenoxid)diaminen, Poly(ethylenoxid/-propylenoxid)monoaminen und Mischungen davon.

Bevorzugt wird zur Umsetzung PIBSA und ein Polyethylenoxidmonoalkylether eingesetzt. Besonders bevorzugt wird zur Umsetzung PIBSA und ein Polyethylenoxidmonomethylether eingesetzt. Auf die vorherigen Ausführungen zu geeigneten Pluriol®-Marken wird Bezug genommen.

In einer speziellen Ausführungsform umfasst der Hilfsstoff H1) ein Umsetzungsprodukt von PIBSA und einem Polyethylenoxidmonomethylether. In einer ganz speziellen Ausführungsform besteht der Hilfsstoff H1) aus einem Umsetzungsprodukt von PIBSA und einem Polyethylenoxidmonomethylether. Bevorzugt wird zur Herstellung dieses Hilfsstoffs H1) ein PIBSA und ein Polyethylenoxidmonomethylether in einem Molverhältnis von 0,9 : 1 bis 1 : 2,5, besonders bevorzugt von etwa 1 : 1, eingesetzt. Das PIBSA hat vorzugsweise ein Molekulargewicht in einem Bereich von 350 bis 2500, vorzugsweise 500 bis 1500. Der Polyethylenoxidmonomethylether hat vorzugsweise ein Molekulargewicht in einem Bereich von 350 bis 2500, vorzugsweise 500 bis 2000.

### Siliconverbindungen (H1), die wenigstens eine Polyethergruppe aufweisen

Wie zuvor bereits ausgeführt, eignen sich die zuvor beschriebenen Siliconverbindungen (E), (G), (J) und (K), die auch bereits wenigstens eine hydrophile Gruppe (speziell wenigstens eine Gruppe B)) aufweisen, als solche für den Einsatz als Komponente H1). Das gilt speziell für die Verbindungen (E), (G), (J) und (K), die wenigstens eine Gruppe (F) mit Alkylenoxid-Wiederholungseinheiten aufweisen. Auf die vorherige Offenbarung zu Siliconverbindungen (E), (G), (J) und (K) wird hier in vollem Umfang Bezug genommen. Speziell geeignet ist auch die Verbindung mit der INCI-Bezeichnung Methoxy PEG/PPG-7/3 Aminopropyl Dimethicone (z. B. ABIL® Soft AF 100, Evonik Industries).

Vorzugsweise wird die Komponente H1) in einer Menge von 0,1 bis 15 Gew.-Teilen, besonders bevorzugt 0,3 bis 10 Gew.-Teilen, bezogen auf 100 Gew.-Teile der zur Polymerisation eingesetzten Monomere, verwendet.

### Basische Verbindung H2)

Die Verbindung H2) ist ausgewählt unter NH₃, (NH₄)₂CO₃, NH₄HCO₃, Monoalkylaminen, Dialkylaminen, Trialkylaminen, Aminoalkoholen, stickstoffhaltigen Heterocyclen und Mischungen davon.

Geeignete Verbindungen H2) sind NH₃ und Verbindungen, die unter den Reaktionsbedingungen befähigt sind, NH₃ freizusetzen. Dazu zählen bevorzugt (NH₄)₂CO₃ und NH₄HCO₃. Besonders bevorzugt umfasst die Komponente H2) Ammoniumhydrogencarbonat oder besteht aus Ammoniumhydrogencarbonat.

Die in dem erfindungsgemäßen Verfahren eingesetzte Aminogruppen-haltige Verbindung ist vorzugsweise wasserfrei. Unter "wasserfrei" wird im Rahmen der Erfindung verstanden, dass die mit der Aminogruppen-haltigen Verbindung dem Reaktionsgemisch zugefügte Wassermenge und die gegebenenfalls beim Einsatz von Verbindungen mit quartären Ammoniumgruppen freigesetzte Wassermenge so gering ist, dass das zur Herstellung der Copolymerzusammensetzung CP) eingesetzte Reaktionsgemisch im gesamten Verlauf der Copolymerisation einen Wassergehalt von höchstens 2 Gew.-% aufweist.

Bevorzugte Aminogruppen-haltige Verbindungen H2) sind C₁-C₆-Alkylamine, besonders bevorzugt n-Propylamin und n-Butylamin.

Bevorzugte Aminogruppen-haltige Verbindungen H2) sind weiterhin Di(C₁-C₆-alkyl)amine, besonders bevorzugt Diethylpropylamin und Dipropylmethylamin.

Bevorzugte Aminogruppen-haltige Verbindungen H2) sind weiterhin Tri(C₁-C₆-alkyl)amine, besonders bevorzugt Trimethylamin, Triethylamin, Triisopropylamin, etc.

Bevorzugte Aminogruppen-haltige Verbindungen H2) sind weiterhin Aminoalkohole, z. B. Monoalkanolamine, Dialkanolamine, Trialkanolamine, Alkyldialkanolamine, Dialkylalkanolamine und Mischungen davon.

Besonders bevorzugt ist die Verbindung H2) ausgewählt unter Ethanolamin, Diethanolamin, Triethanolamin, Methyldiethanolamin, Ethyldiethanolamin, Dimethylethanolamin, 2-Amino-2-methyl-1-propanol und Mischungen davon.

Des Weiteren bevorzugt ist die Base ausgewählt unter stickstoffhaltigen Heterocyclen. Vorzugsweise sind die stickstoffhaltigen Heterocyclen ausgewählt aus der Gruppe der Pyrrole, Pyrrolidine, Pyridine, Chinoline, Isochinoline, Purine, Pyrazole, Imidazole, Triazole, Tetrazole, Indolizine, Pyridazine, Pyrimidine, Pyrazine, Triazine, Indole, Isoindole, Oxazole, Oxazolidone, Oxazolidine, Morpholine, Piperazine, Piperidine und deren Derivaten. Geeignete Derivate der zuvor genannten stickstoffhaltigen Heterocyclen können wenigstens einen weiteren Substituenten aufweisen, der vorzugsweise ausgewählt ist unter Alkyl, Cycloalkyl, Heterocycloalkyl, Aryl, Hetaryl, Alkoxy, Cycloalkoxy, Aryloxy, COOH, Carboxylat, SO₃H, Sulfonat, Alkoxycarbonyl, Acyl und Nitro. Die stickstoffhaltigen Heterocyclen können speziell einen oder mehrere C₁-C₆-Alkylsubstituenten, wie Methyl, Ethyl, n-Propyl, Isopropyl, n-Butyl, sec.-Butyl, tert.-Butyl etc., aufweisen.

Vorzugsweise umfasst die Komponente H2) Imidazol oder ein Derivat davon. Besonders bevorzugt ist N-Vinylimidazol. Bevorzugt sind weiterhin Imidazol und ImidazolDerivate der allgemeinen Formel (L), 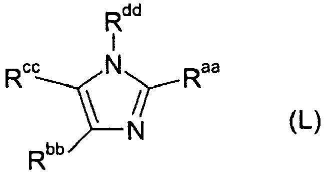 worin R^{aa}, R^{bb} und R^{cc} unabhängig voneinander ausgewählt sind unter Wasserstoff, C₁-C₄-Alkyl, speziell Methyl, Ethyl, n-Propyl, Isopropyl, n-Butyl und tert.-Butyl, C₅-C₆-Cycloalkyl, speziell Cyclohexyl, und C₆-C₁₀-Aryl, speziell Phenyl.

Beispiele für Verbindungen der allgemeinen Formel (L) sind folgender Tabelle 1 zu entnehmen:

**Tabelle 1**

| **R^{aa}** | **R^{bb}** | **R^{cc}** | **R^{dd}** |
|---|---|---|---|
| H | H | H | H |
| Me | H | H | H |
| H | Me | H | H |
| H | H | Me | H |
| Me | Me | H | H |
| H | Me | Me | H |
| Me | H | Me | H |
| Ph | H | H | H |
| H | Ph | H | H |
| H | H | Ph | H |
| Ph | Me | H | H |
| Ph | H | Me | H |
| Me | Ph | H | H |
| H | Ph | Me | H |
| H | Me | Ph | H |
| Me | H | Ph | H |
| H | H | H | Me |
| H | H | H | C₂H₅ |

| | | | |
|---|---|---|---|
| Me = Methyl Ph = Phenyl | | | |

Ein weiteres bevorzugtes Imidazolderivat ist Histidin.

Bevorzugt umfasst die Komponente H2) wenigstens eine Imidazolverbindung oder besteht aus wenigstens einer Imidazolverbindung. Besonders bevorzugt ist die Imidazolverbindung H2) ausgewählt unter Imidazol und N-Alkylimidazolen. Bevorzugte N-Alkylimidazole sind N-Methylimidazol und N-Ethylimidazol. Besonders bevorzugt umfasst die Komponente H2) Imidazol oder besteht aus Imidazol.

Die zuvor genannten Aminogruppen-haltigen Verbindungen können einzeln oder in Form von beliebigen Mischungen eingesetzt werden.

Vorzugsweise wird die Komponente H2) in einer Menge von 0,1 bis 15 Gew.-Teile, besonders bevorzugt 0,3 bis 10 Gew.-Teile, bezogen auf 100 Gew.-Teile der zur Polymerisation eingesetzten Monomere, verwendet.

### Copolymerzusammensetzung CP)

Die Herstellung der erfindungsgemäßen Copolymerzusammensetzungen CP) erfolgt durch Fällungspolymerisation. Bei der Fällungspolymerisation sind die eingesetzten Monomere im Reaktionsmedium (Monomer, Lösungsmittel) löslich, das entsprechende Polymer aber nicht. Das entstehende Polymer wird unter den gewählten Polymerisationsbedingungen unlöslich und fällt aus dem Reaktionsgemisch aus. Das erfindungsgemäße Verfahren selbst zeichnet sich durch vorteilhafte Eigenschaften aus und führt zudem auch zu Copolymerzusammensetzungen mit besonders vorteilhaften Eigenschaften. Die in den erfindungsgemäßen Polymerzusammensetzungen enthaltenen Fällungspolymerisate zeichnen sich durch ihre Befähigung als Rheologiemodifizierungsmittel (speziell als Verdicker) aus. Die getrockneten Formulierungen sind sehr gut redispergierbar und zeichnen sich durch eine hohe Auflösungsgeschwindigkeit aus. Sie eignen sich zur Formulierung von Gelen mit verbesserter Klarheit und/oder verbesserten Struktureigenschaften und/oder verbesserter Auswaschbarkeit gegenüber Gelen auf Basis konventioneller Polymerzusammensetzungen. Zudem kommt es bei der Polymerisation nicht zu einer unerwünscht starken Erhöhung der Viskosität des Reaktionsmediums. Eine Belagbildung kann in der Regel erfolgreich vermieden werden.

Bevorzugt weist das zur Herstellung der Copolymerzusammensetzung CP) eingesetzte Reaktionsgemisch im gesamten Verlauf der Copolymerisation einen Wassergehalt von höchstens 5 Gew.-%, besonders bevorzugt höchstens 3 Gew.-%, insbesondere höchstens 2 Gew.-%, auf.

### Monomer a)

Zur Herstellung der erfindungsgemäßen Copolymerzusammensetzungen CP) wird als Komponente a) Acrylsäure eingesetzt. Die Komponente a) wird erfindungsgemäß in einer Menge von 70 bis 100 Gew.-%, bezogen auf das Gesamtgewicht der zur Polymerisation eingesetzten Verbindungen, eingesetzt. Die Komponente a) wird besonders bevorzugt in einer Menge von 70 bis 99,99 Gew.-%, insbesondere 75 bis 99,9 Gew.-%, bezogen auf das Gesamtgewicht der zur Polymerisation eingesetzten Verbindungen, eingesetzt (d. h. die Monomere a) und, soweit vorhanden, b) bis f) addieren sich zu 100 Gew.-%).

In einer ersten bevorzugten Ausführungsform besteht die zur Herstellung der Copolymerzusammensetzung CP) durch radikalische Copolymerisation eingesetzte Monomerzusammensetzung nur aus den Komponenten a) und c). Dann wird die Komponente a) bevorzugt in einer Menge von 95 bis 99,99 Gew.-%, besonders bevorzugt von 98 bis 99,9 Gew.-%, bezogen auf das Gesamtgewicht der zur Polymerisation eingesetzten Verbindungen a) und c), eingesetzt. Das erfindungsgemäße Verfahren dient dann speziell zur Herstellung vernetzter Polyacrylsäure.

In einer zweiten bevorzugten Ausführungsform wird die Komponente a) in einer Menge von 70 bis 99,99 Gew.-%, vorzugsweise 75 bis 99,9 Gew.-%, bezogen auf das Gesamtgewicht der zur Polymerisation eingesetzten Verbindungen (d. h. Komponenten a) bis g)), eingesetzt. Bei dieser Ausführungsform umfasst die zur Herstellung der Copolymerzusammensetzung CP) eingesetzte Monomerzusammensetzung neben den Komponenten a) und c) noch wenigstens ein weiteres Monomer.

### Hydrophiles, nichtionisches Monomer b)

Das Monomer b) ist vorzugsweise ausgewählt unter den im Folgenden aufgeführten Monomeren b1) bis b4), Mischungen von Monomeren aus einer der Monomerklassen b1) bis b4) sowie Mischungen von Monomeren aus mehreren Monomerklassen b1) bis b4).

Die Komponente b) wird erfindungsgemäß in einer Menge von 0 bis 30 Gew.-%, bezogen auf das Gesamtgewicht der zur Polymerisation eingesetzten Verbindungen, eingesetzt. Vorzugsweise wird die Komponente b) in einer Menge von 0,1 bis 29,99 Gew.-%, besonders bevorzugt 0,5 bis 25 Gew.-%, bezogen auf das Gesamtgewicht der zur Polymerisation eingesetzten Monomere, eingesetzt.

### Amidgruppenhaltiges Monomer b1)

Die zur Herstellung der Copolymerzusammensetzung CP) eingesetzte Monomerzusammensetzung kann zusätzlich wenigstens ein amidgruppenhaltiges Monomer b1) der allgemeinen Formel (IV) einpolymerisiert enthalten 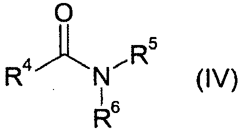 wobei
einer der Reste R⁴ bis R⁶ für eine Gruppe der Formel CH₂=CR⁷- mit R⁷ = H oder C₁-C₄-Alkyl steht und die übrigen Reste R⁴ bis R⁶ unabhängig voneinander für H, Alkyl, Cycloalkyl, Heterocycloalkyl, Aryl oder Hetaryl stehen,
wobei R⁴ und R⁵ gemeinsam mit der Amidgruppe, an die sie gebunden sind, auch für ein Lactam mit 5 bis 8 Ringatomen stehen können,
wobei R⁵ und R⁶ gemeinsam mit dem Stickstoffatom, an das sie gebunden sind, auch für einen fünf- bis siebengliedrigen Heterocyclus stehen können,
mit der Maßgabe, dass die Summe der Kohlenstoffatome der Reste R⁴, R⁵ und R⁶ höchstens 8 beträgt.

Vorzugsweise weisen die Verbindungen der Komponente b1) zusätzlich zu dem Carbonyl-Kohlenstoffatom der Amidgruppe höchstens 7 weitere Kohlenstoffatome auf. Bevorzugt sind die Verbindungen der Komponente b1) ausgewählt unter primären Amiden α,β-ethylenisch ungesättigter Monocarbonsäuren, N-Vinylamiden gesättigter Monocarbonsäuren, N-Vinyllactamen, N-Alkyl- und N,N-Dialkylamiden α,β-ethylenisch ungesättigter Monocarbonsäuren und Mischungen davon.

Bevorzugte Monomere b1) sind N-Vinyllactame und deren Derivate, die z. B. einen oder mehrere C₁-C₆-Alkylsubstituenten, wie Methyl, Ethyl, n-Propyl, Isopropyl, n-Butyl, sec.-Butyl, tert.-Butyl, etc. aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc.

Besonders bevorzugt werden N-Vinylpyrrolidon und/oder N-Vinylcaprolactam eingesetzt.

Geeignete Monomere b1) sind weiterhin Acrylsäureamid und Methacrylsäureamid.

Geeignete N-Alkyl- und N,N-Dialkylamide α,β-ethylenisch ungesättigter Monocarbonsäuren, die zusätzlich zu dem Carbonyl-Kohlenstoffatom der Amidgruppe höchstens 7 weitere Kohlenstoffatome aufweisen, sind beispielsweise N-Methyl(meth)acrylamid, N-Ethyl(meth)acrylamid, N-Propyl(meth)acrylamid, N-(n-Butyl)(meth)acrylamid, N-tert.-Butyl(meth)acrylamid, n-Pentyl(meth)acrylamid, n-Hexyl(meth)acrylamid, n-Heptyl(meth)acrylamid, N,N-Dimethyl(meth)acrylamid, N,N-Diethyl(meth)acrylamid, Piperidinyl(meth)acrylamid, Morpholinyl(meth)acrylamid und Mischungen davon.

Als Monomere b1) geeignete offenkettige N-Vinylamidverbindungen sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid, N-Vinyl-butyramid und Mischungen davon. Bevorzugt wird N-Vinylformamid eingesetzt.

Vorzugsweise wird die Komponente b1) in einer Menge von 0,1 bis 29,99 Gew.-%, besonders bevorzugt 0,5 bis 25 Gew.-%, bezogen auf das Gesamtgewicht der zur Polymerisation eingesetzten Monomere, eingesetzt.

### Monomer b2)

In einer weiteren Ausführungsform kann die zur Herstellung der Copolymerzusammensetzung eingesetzte Monomerzusammensetzung zusätzlich wenigstens ein weiteres Monomer b2) einpolymerisiert enthalten, das eine Gruppe der Formeln (Va) oder (Vb) 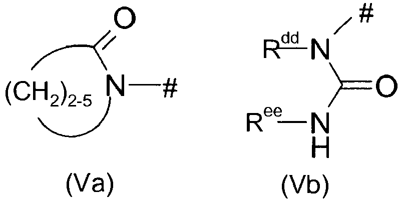 aufweist, worin
# für die Bindungsstelle zu einer Gruppe mit einer radikalisch polymerisierbaren, α,β-ethylenisch ungesättigten Doppelbindung steht, wobei in den Verbindungen (Va) # nicht für die Bindungsstelle zu einer Gruppe der Formel CH₂=CR⁷- mit R⁷ = H oder C₁-C₄-Alkyl steht (= Monomere b1),
R^{dd} für H oder C₁-C₄-Alkyl steht,
R^{ee} für H oder C₁-C₄-Alkyl steht, oder
R^{dd} und R^{ee} gemeinsam für (CH₂)₁₋₄ stehen.

Bevorzugt ist das Monomer b2) ausgewählt unter Monomeren mit einer Gruppe der Formeln (Va.1) oder (Vb.1)

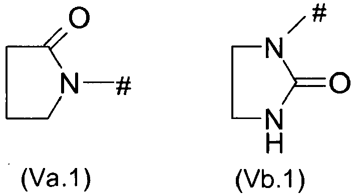

Bevorzugt als Monomere b2) sind die Verbindungen der Formel: 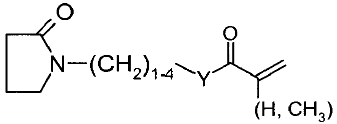 worin Y für O oder NR^{y} steht, worin R^{y} für H, Alkyl, Cycloalkyl oder Aryl steht.

Spezielle Monomere b2) sind die Verbindungen der Formel: 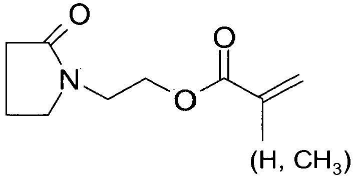 Geeignete Harnstoffgruppen aufweisende Monomere b2) sind z. B. N-Vinyl- oder N-Allylharnstoff oder Derivate des Imidazolidin-2-ons. Dazu zählen N-Vinyl- und N-Allylimidazolidin-2-on, N-Vinyloxyethylimidazolidin-2-on, N-(2-(Meth)acrylamidoethyl)imidazolidin-2-on, N-(2-(Meth)acryloxyethyl)imidazolidin-2-on (= 2-Ureido(meth)acrylat), N-[2-((Meth)acryloxyacetamido)ethyl]imidazolidin-2-on etc.

Bevorzugte Harnstoffgruppen aufweisende Monomere b2) sind N-(2-Acryloxyethyl)imidazolidin-2-on und N-(2-Methacryloxyethyl)imidazolidin-2-on. Besonders bevorzugt ist N-(2-Methacryloxyethyl)imidazolidin-2-on (2-Ureidomethacrylat, UMA).

Vorzugsweise wird die Komponente b2) in einer Menge von 0,1 bis 20 Gew.-%, besonders bevorzugt 0,5 bis 10 Gew.-%, bezogen auf das Gesamtgewicht der zur Polymerisation eingesetzten Monomere, eingesetzt.

### Monomer b3)

In einer weiteren Ausführungsform kann die zur Herstellung der Copolymerzusammensetzung eingesetzte Monomerzusammensetzung zusätzlich wenigstens ein weiteres Monomer b3) einpolymerisiert enthalten, das ausgewählt ist unter Estern α,β-ethylenisch ungesättigter Mono- und Dicarbonsäuren mit C₂-C₃₀-Diolen, Amiden α,β-ethylenisch ungesättigter Mono- und Dicarbonsäuren mit C₂-C₃₀-Aminoalkoholen, die eine primäre oder sekundäre Aminogruppe aufweisen, und Mischungen davon.

Geeignete zusätzliche Monomere b3) sind weiterhin 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat und 3-Hydroxy-2-ethylhexylmethacrylat.

Geeignete zusätzliche Monomere b3) sind weiterhin 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.

Vorzugsweise wird die Komponente b3) in einer Menge von 0,1 bis 25 Gew.-%, besonders bevorzugt 0,5 bis 20 Gew.-%, bezogen auf das Gesamtgewicht der zur Polymerisation eingesetzten Monomere, eingesetzt.

### Monomer b4)

Zur Herstellung der erfindungsgemäßen Copolymerzusammensetzung CP) kann zusätzlich wenigstens ein von den Komponenten a) bis f) verschiedenes, damit copolymerisierbares Monomer b4) eingesetzt werden.

Geeignete Verbindungen b4) sind ausgewählt ist unter Verbindungen der allgemeinen Formeln VI a) und VI b) 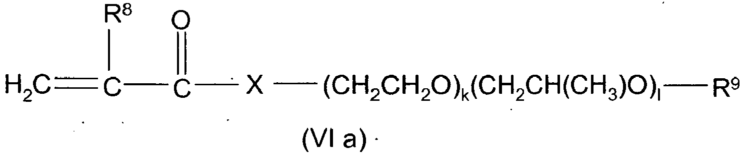 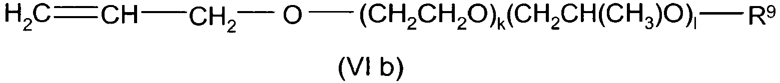 worin
die Reihenfolge der Alkylenoxideinheiten beliebig ist,
k und I unabhängig voneinander für eine ganze Zahl von 0 bis 1000 stehen, wobei die Summe aus k und l mindestens 5 beträgt,
R⁸ für Wasserstoff oder C₁-C₈-Alkyl steht
R⁹ für Wasserstoff, C₁-C₃₀-Alkyl, C₂-C₃₀-Alkenyl oder C₅-C₈-Cycloalkyl steht,
X für O oder eine Gruppe der Formel NR¹⁰ steht, worin R¹⁰ für H, Alkyl, Alkenyl, Cycloalkyl, Heterocycloalkyl, Aryl oder Hetaryl steht.

In den Formeln VI a) und VI b) steht k vorzugsweise für eine ganze Zahl von 1 bis 500, insbesondere 3 bis 250. Bevorzugt steht I für eine ganze Zahl von 0 bis 100.

Bevorzugt steht R⁸ in der Formel VI a) für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht R⁹ in den Formeln VI a) und VI b) für Wasserstoff, Methyl, Ethyl, n-Propyl, Isopropyl, n-Butyl, sec.-Butyl, n-Pentyl, n-Hexyl, n-Heptyl, n-Octyl, 1,1,3,3-Tetramethylbutyl, Ethylhexyl, n-Nonyl, n-Decyl, n-Undecyl, Tridecyl, Myristyl, Pentadecyl, Palmityl, Heptadecyl, Octadecyl, Nonadecyl, Arrachinyl, Behenyl, Lignocerenyl, Cerotinyl, Melissinyl, Palmitoleinyl, Oleyl, Linolyl, Linolenyl, Stearyl, Lauryl.

Vorzugsweise steht X in der Formel VI a) für O oder NH.

Geeignete Polyetheracrylate VI a) sind z. B. die Polykondensationsprodukte der zuvor genannten α,β-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R⁹-OH hergestellt werden. Die Alkylenoxide können einzeln, alternierend nacheinander oder als Mischung eingesetzt werden. Die Polyetheracrylate VI a) können allein oder in Mischungen zur Herstellung der erfindungsgemäß eingesetzten Polymere verwendet werden.

Geeignete Allylalkoholalkoxilate VI b) sind z. B. die Veretherungsprodukte von Allylchlorid mit entsprechenden Polyetherolen. Geeignete Polyetherole können leicht durch Umsetzung von Ethylenoxid, 1,2-Propylenoxid und/oder Epichlorhydrin mit einem Starteralkohol R⁹-OH hergestellt werden. Die Alkylenoxide können einzeln, alternierend nacheinander oder als Mischung eingesetzt werden. Die Allylalkoholalkoxilate VI b) können allein oder in Mischungen zur Herstellung der erfindungsgemäß eingesetzten Polymere verwendet werden.

### Vernetzer c)

Zur Herstellung der Copolymerzusammensetzungen CP) wird erfindungsgemäß wenigstens ein Vernetzer, d. h. eine Verbindung mit zwei oder mehr als zwei ethylenisch ungesättigten, nichtkonjugierten Doppelbindungen, eingesetzt.

Vorzugsweise werden Vernetzer in einer Menge von 0,01 bis 5 Gew.-%, besonders bevorzugt 0,1 bis 4 Gew.-%, bezogen auf das Gesamtgewicht der zur Polymerisation eingesetzten Verbindungen, eingesetzt.

Geeignete Vernetzer c) sind zum Beispiel Acrylester, Methacrylester, Allylether oder Vinylether von mindestens zweiwertigen Alkoholen. Die OH-Gruppen der zugrunde liegenden Alkohole können dabei ganz oder teilweise verethert oder verestert sein; die Vernetzer enthalten aber mindestens zwei ethylenisch ungesättigte Gruppen.

Beispiele für die zugrunde liegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thiapentan-1,5-diol, sowie Polyethylenglykole, Polypropylenglykole und Polytetrahydrofurane mit Molekulargewichten von jeweils 200 bis 10 000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrunde liegende Alkohole mit mehr als zwei OH-Gruppen sind Trimethylolpropan, Glycerin, Pentaerythrit, 1,2,5-Pentantriol, 1,2,6-Hexantriol, Cyanursäure, Sorbitane, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden. Bevorzugt sind Ethylenglykoldi(meth)acrylat und Polyethylenglykoldi(meth)acrylate.

Weitere geeignete Vernetzer c) sind die Vinylester oder die Ester einwertiger, ungesättigter Alkohole mit ethylenisch ungesättigten C₃-C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer c) sind von (Meth)acrylestern verschiedene Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Vernetzer c) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20 000.

Als Vernetzer c) sind ferner geeignet die Acrylsäureamide, Methacrylsäureamide und N-Allylamine von mindestens zweiwertigen Aminen. Solche Amine sind zum Beispiel 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylinonoalkylammoniumsalze, z. B. Triallylmethylammoniumchlorid oder -methylsulfat, als Vernetzer c) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff.

Weitere geeignete Vernetzer c) sind Divinyldioxan, Tetraallylsilan oder Tetravinylsilan.

Selbstverständlich können auch Mischungen der vorgenannten Verbindungen c) eingesetzt werden.

Ganz besonders bevorzugt als Vernetzer c) sind Ethylenglykoldi(meth)acrylat, Polyethylenglykoldi(meth)acrylate, Pentaerythrittriallylether, Methylenbisacrylamid, N,N'-Divinylethylenharnstoff, Triallylamin und Triallylmonoalkylammoniumsalze.

### Kationogenes/kationisches Monomer d)

Die zur Herstellung der Copolymerzusammensetzung CP) eingesetzte Monomerzusammensetzung kann zusätzlich wenigstens eine Verbindung d) mit einer radikalisch polymerisierbaren, α,β-ethylenisch ungesättigten Doppelbindung und wenigstens einer kationogenen und/oder kationischen Gruppen pro Molekül enthalten.

Vorzugsweise wird die Komponente d) in einer Menge von 0,1 bis 29,99 Gew.-%, besonders bevorzugt 0,2 bis 25 Gew.-%, bezogen auf das Gesamtgewicht der zur Polymerisation eingesetzten Verbindungen, eingesetzt.

Bevorzugt weisen die in der Copolymerzusammensetzung CP) enthaltenen Copolymere einen Überschuss an anionogenen und/oder anionischen Gruppen auf. Werden daher Monomere d) eingesetzt, dann vorzugsweise in solchen Mengen, dass das Copolymer in CP) einen molaren Überschuss an anionogenen/anionischen Gruppen gegenüber kationogenen/kationischen Gruppen oder von wenigstens 5 : 1, vorzugsweise wenigstens 10 : 1, aufweist.

Bevorzugt handelt es sich bei den kationogenen und/oder kationischen Gruppen der Komponente d) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen oder quaternäre Ammoniumgruppen. Geladene kationische Gruppen lassen sich aus den Aminstickstoffen entweder durch Protonierung oder durch Quaternisierung mit Säuren oder Alkylierungsmitteln erzeugen. Dazu zählen z. B. Carbonsäuren, wie Milchsäure, oder Mineralsäuren, wie Phosphorsäure, Schwefelsäure und Salzsäure, oder als Alkylierungsmittel C₁-C₄-Alkylhalogenide oder -sulfate, wie Ethylchlorid, Ethylbromid, Methylchlorid, Methylbromid, Dimethylsulfat und Diethylsulfat. Eine Protonierung oder Quaternisierung kann im Allgemeinen sowohl vor als auch nach der Polymerisation erfolgen.

Vorzugsweise ist die Komponente d) ausgewählt unter Estern α,β-ethylenisch ungesättigter Mono- und Dicarbonsäuren mit Aminoalkoholen, welche am Aminstickstoff mono- oder dialkyliert sein können, Amiden α,β-ethylenisch ungesättigter Mono- und Dicarbonsäuren mit Diaminen, welche mindestens eine primäre oder sekundäre Aminogruppe aufweisen, N,N-Diallylamin, N,N-Diallyl-N-alkylaminen und deren Derivaten, vinyl- und allylsubstituierten Stickstoffheterocyclen, vinyl- und allylsubstituierten heteroaromatischen Verbindungen, und Mischungen davon.

Bevorzugte Verbindungen d) sind die Ester von α,β-ethylenisch ungesättigten Mono- und Dicarbonsäuren mit Aminoalkoholen. Bevorzugte Aminoalkohole sind C₂-C₁₂-Aminoalkohole, welche am Aminstickstoff C₁-C₈-mono- oder -dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden als Säurekomponente Acrylsäure, Methacrylsäure und deren Gemische eingesetzt.

Bevorzugte Monomere d) sind N-Methylaminoethyl(meth)acrylat, N-Ethylaminoethyl(meth)acrylat, N-(n-Propyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminomethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat.

Besonders bevorzugt sind N,N-Dimethylaminoethylacrylat, N,N-Dimethylaminoethylmethacrylat und Mischungen davon. Bevorzugte Monomere d) sind insbesondere auch die Quaternisierungsprodukte der zuvor genannten Verbindungen.

In einer ganz speziellen Ausführung besteht die Komponente d) nur aus N,N-Dimethylaminoethyl(meth)acrylat.

Geeignete Monomere d) sind weiterhin die Amide der zuvor genannten α,β-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen.

Bevorzugt als Monomere d) sind z. B. N-[tert.-Butylaminoethyl](meth)acrylamid, N-[2-(Dimethylamino)ethyl]acrylamid, N-[2-(Dimethylamino)ethyl]methacrylamid, N-[3-(Dimethylamino)propyl]acrylamid, N-[3-(Dimethylamino)propyl]methacrylamid, N-[4-(Dimethylamino)butyl]acrylamid, N-[4-(Dimethylamino)butyl]methacrylamid, N-[2-(Diethylamino)ethyl]acrylamid, N-[4-(Dimethylamino)cyclohexyl]acrylamid und N-[4-(Dimethylamino)cyclohexyl]methacrylamid.

In einer geeigneten Ausführungsform umfasst die Komponente d) als vinylsubstituierte heteroaromatische Verbindung wenigstens eine N-Vinylimidazol-Verbindung. In einer speziellen Ausführungsform ist die Komponente d) ausgewählt unter N-Vinylimidazol-Verbindungen und Mischungen, die wenigstens eine N-Vinylimidazol-Verbindung enthalten.

Geeignete N-Vinylimidazol-Verbindungen sind Verbindungen der Formel 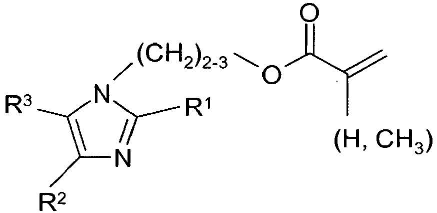 worin R¹ bis R³ unabhängig voneinander für Wasserstoff, C₁-C₄-Alkyl oder Phenyl stehen. Bevorzugt stehen R¹ bis R³ für Wasserstoff.

Geeignet sind weiterhin N-Vinylimidazol-Verbindungen der allgemeinen Formel (VII) 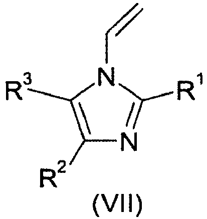 worin R¹ bis R³ unabhängig voneinander für Wasserstoff, C₁-C₄-Alkyl oder Phenyl stehen.

Beispiele für Verbindungen der allgemeinen Formel (VII) sind folgender Tabelle 1a zu entnehmen:

**Tabelle 1a**

| **R¹** | **R²** | **R³** |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Bevorzugt als Monomer d) ist 1-Vinylimidazol (N-Vinylimidazol) und sind Mischungen, die N-Vinylimidazol enthalten.

Geeignete Monomere d) sind auch die durch Protonierung oder Quaternisierung der zuvor genannten N-Vinylimidazolverbindungen erhältlichen Verbindungen. Beispiele für solche geladenen Monomere d) sind quaternisierte Vinylimidazole, insbesondere 3-Methyl-1-vinylimidazoliumchlorid, -methosulfat und -ethosulfat. Geeignete Säuren und Alkylierungsmittel sind die zuvor aufgeführten. Bevorzugt erfolgt eine Protonierung oder Quaternisierung nach der Polymerisation.

Geeignete Monomere d) sind weiterhin von Vinylimidazolen verschiedene vinyl- und allylsubstituierte Stickstoffheterocyclen, wie 2- und 4-Vinylpyridin, 2- und 4-Allylpyridin, und die Salze davon.

### Anionogenes/anionisches Monomer e)

In dem erfindungsgemäßen Verfahren zur Herstellung der Copolymerzusammensetzung CP) kann gegebenenfalls als Komponente e) eine von Acrylsäure verschiedene Verbindung mit einer radikalisch polymerisierbaren, α,β-ethylenisch ungesättigten Doppelbindung und mindestens einer anionogenen und/oder anionischen Gruppe pro Molekül eingesetzt werden. Die Komponente e) wird vorzugsweise in einer Menge von 0 bis 29,99 Gew.-%, besonders bevorzugt von 0 bis 25 Gew.-%, bezogen auf das Gesamtgewicht der zur Polymerisation eingesetzten Verbindungen, eingesetzt. Soweit vorhanden wird die Komponente e) vorzugsweise in einer Menge von 0,1 bis 29,99 Gew.-%, besonders bevorzugt von 0,5 bis 25 Gew.-%, bezogen auf das Gesamtgewicht der zur Polymerisation eingesetzten Verbindungen, eingesetzt.

Vorzugsweise sind die Verbindungen e) ausgewählt unter monoethylenisch ungesättigten Carbonsäuren, Sulfonsäuren, Phosphonsäuren und Mischungen davon.

Zu den Monomeren e) zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren e) zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu den Monomeren e) zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Zu den Monomeren e) zählen auch die Salze der zuvor genannten Säuren, insbesondere die Natrium-, Kalium- und Ammoniumsalze sowie die Salze mit Aminen. Die Monomere e) können als solche oder als Mischungen untereinander eingesetzt werden. Die angegebenen Gewichtsanteile beziehen sich sämtlich auf die Säureform.

Vorzugsweise ist die Komponente e) ausgewählt unter Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure und Mischungen davon.

Besonders bevorzugt wird als Komponente e) Methacrylsäure eingesetzt.

### Weitere Monomere f)

Zur Herstellung der Copolymerzusammensetzung CP) kann zusätzlich wenigstens eine Verbindung f) eingesetzt werden, die ausgewählt ist unter Estern α,β-ethylenisch ungesättigter Mono- und Dicarbonsäuren mit C₁-C₃₀-Alkanolen, Amiden α,β-ethylenisch ungesättigter Mono- und Dicarbonsäuren mit Mono- und Di-(C₁-C₃₀-Alkyl)aminen, N,N-Diallylaminen, deren Säureadditionssalzen und Quaternisierungsprodukten, N,N-Diallyl-N-alkylaminen, deren Säureadditionssalzen und Quaternisierungsprodukten, Urethan(meth)acrylaten mit Alkylenoxidgruppen, Estern von Vinylalkohol und Allylalkohol mit C₁-C₃₀-Monocarbonsäuren, C₁-C₃₀-Alkylvinylethern, Vinylaromaten, Vinylhalogeniden, Vinylidenhalogeniden, C₂-C₈-Monoolefinen, nicht aromatischen Kohlenwasserstoffen mit mindestens zwei konjugierten Doppelbindungen und Mischungen davon.

Geeignete Ester α,β-ethylenisch ungesättigter Mono- und Dicarbonsäuren mit C₁-C₃₀-Alkanolen sind z. B. Methyl(meth)acrylat, Methylethacrylat, Ethyl(meth)acrylat, Ethylethacrylat, n-Propyl(meth)acrylat, Isopropyl(meth)acrylat, n-Butyl(meth)acrylat, tert.-Butyl(meth)acrylat, tert.-Butylethacrylat, n-Pentyl(meth)acrylat, n-Hexyl(meth)acrylat, n-Heptyl(meth)acrylat, n-Octyl(meth)acrylat,1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon.

Geeignete Amide α,β-ethylenisch ungesättigter Mono- und Dicarbonsäuren mit Mono- und Di-(C₁-C₃₀-Alkyl)aminen sind z. B. Methyl(meth)acrylamid, Methylethacrylamid, Ethyl(meth)acrylamid, Ethylethacrylamid, n-Propyl(meth)acrylamid, Isopropyl(meth)-acrylamid, n-Butyl(meth)acrylamid, tert.-Butyl(meth)acrylamid, tert.-Butylethacrylamid, n-Pentyl(meth)acrylamid, n-Hexyl(meth)acrylamid, n-Heptyl(meth)acrylamid, n-Octyl(meth)acrylamid,1,1,3,3-Tetramethylbutyl(meth)acrylamid, Ethylhexyl(meth)acrylamid, n-Nonyl(meth)acrylamid, n-Decyl(meth)acrylamid, n-Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arrachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid, N-Methyl-N-(n-octyl)(meth)acrylamid, N,N'-Di-(n-octyl)(meth)acrylamid und Mischungen davon.

Geeignete C₁-C₃₀-Alkylvinylether sind z. B. Methylvinylether, Ethylvinylether, n-Propylvinylether, Isopropylvinylether, n-Butylvinylether, tert.-Butylvinylether, n-Pentylvinylether, n-Hexylvinylether, n-Heptylvinylether, n-Octylvinylether, 1,1,3,3-Tetramethylbutylvinylether, Ethylhexylvinylether, n-Nonylvinylether, n-Decylvinylether, n-Undecylvinylether, Tridecylvinylether, Myristylvinylether, Pentadecylvinylether, Palmitylvinylether, Heptadecylvinylether, Octadecylvinylether, Nonadecylvinylether, Arrachinylvinylether, Behenylvinylether, Lignocerenylvinylether, Cerotinylvinylether, Melissinylvinylether, Palmitoleinylvinylether, Oleylvinylether, Linolylvinylether, Linolenylvinylether, Stearylvinylether, Laurylvinylether und Mischungen davon.

Geeignete Ester von Vinylalkohol mit C₁-C₃₀-Monocarbonsäuren sind z. B. Methylvinylester, Ethylvinylester, n-Propylvinylester, Isopropylvinylester, n-Butylvinylester, tert.-Butylvinylester, n-Pentylvinylester, n-Hexylvinylester, n-Heptylvinylester, n-Octylvinylester, 1,1,3,3-Tetramethylbutylvinylester, Ethylhexylvinylester, n-Nonylvinylester, n-Decylvinylester, n-Undecylvinylester, Tridecylvinylester, Myristyl-vinylester, Pentadecylvinylester, Palmitylvinylester, Heptadecylvinylester, Octadecyl-vinylester, Nonadecylvinylester, Arrachinylvinylester, Behenylvinylester, Lignocerenylvinylester, Cerotinylvinylester, Melissinylvinylester, Palmitoleinylvinylester, Oleylvinylester, Linolylvinylester, Linolenylvinylester, Stearylvinylester, Laurylvinylester und Mischungen davon.

Geeignete Monomere f) sind weiterhin N,N-Diallylamine und N,N-Diallyl-N-alkylamine und deren Säureadditionssalze und Quaternisierungsprodukte. Alkyl steht dabei vorzugsweise für C₁-C₂₄-Alkyl. Bevorzugt sind N,N-Diallyl-N-methylamin und N,N-Diallyl-N,N-dimethylammonium-Verbindungen, wie z. B. die Chloride und Bromide. Besonders bevorzugt ist N,N-Diallyl-N-methylammmoniumchlorid (DADMAC). Geeignete Urethan(meth)acrylate mit Alkylenoxidgruppen f) sind in der DE 198 38 851 (Komponente e2)) beschrieben, worauf hier in vollem Umfang Bezug genommen wird.

Geeignete zusätzliche Monomere f) sind weiterhin Vinylacetat, Vinylpropionat, Vinylbutyrat und Mischungen davon.

Geeignete zusätzliche Monomere f) sind weiterhin Ethylen, Propylen, Isobutylen, Butadien, Styrol, α-Methylstyrol, Acrylnitril, Methacrylnitril, Vinylchlorid, Vinylidenchlorid, Vinylfluorid, Vinylidenfluorid und Mischungen davon.

Die zuvor genannten Monomere f) können jeweils einzeln oder in Form von beliebigen Mischungen eingesetzt werden.

Vorzugsweise wird die Komponente f) in einer Menge von 0 bis 20 Gew.-%, bezogen auf das Gesamtgewicht der zur Polymerisation eingesetzten Monomere eingesetzt. Eine geeignete Einsatzmenge für zusätzliche Monomere f) liegt in einem Bereich von 0,1 bis 10 Gew.-%, insbesondere 0,2 bis 5 Gew.-%, bezogen auf das Gesamtgewicht der zur Polymerisation eingesetzten Verbindungen.

In einer ersten bevorzugten Ausführungsform des erfindungsgemäßen Verfahrens werden zur Herstellung der Copolymerzusammensetzung CP), bezogen auf das Gesamtgewicht der zur Polymerisation eingesetzten Monomere,
- 98 bis 99,9 Gew.-% Acrylsäure a), und
- 0,1 bis 2 Gew.-% wenigstens einer vernetzenden Verbindung c),
eingesetzt.

In einer weiteren bevorzugten Ausführungsform des erfindungsgemäßen Verfahrens werden zur Herstellung der Copolymerzusammensetzung CP), bezogen auf das Gesamtgewicht der zur Polymerisation eingesetzten Monomere,
- 93 bis 99,7 Gew.-%, Acrylsäure a),
- 0,2 bis 5 Gew.-%, wenigstens einer Verbindung d) mit einer radikalisch polymerisierbaren, α,β-ethylenisch ungesättigten Doppelbindung und wenigstens einer kationogenen und/oder kationischen Gruppen pro Molekül, vorzugsweise Vinylimidazol, und
- 0,1 bis 2 Gew.-% wenigstens einer vernetzenden Verbindung c), eingesetzt.

In einer weiteren bevorzugten Ausführungsform des erfindungsgemäßen Verfahrens werden zur Herstellung der Copolymerzusammensetzung CP), bezogen auf das Gesamtgewicht der zur Polymerisation eingesetzten Monomere,
- 70 bis 99,4 Gew.-% Acrylsäure a),
- 0 bis 29,4 Gew.-% wenigstens einer von Acrylsäure verschiedenen Verbindung e) mit einer radikalisch polymerisierbaren α,β-ethylenisch ungesättigten Doppelbindung und mindestens einer anionogenen und/oder anionischen Gruppe pro Molekül,
- 0,5 bis 20 Gew.-% wenigstens einer Verbindung b3) und/oder b4), und
- 0,1 bis 2 Gew.-% wenigstens einer vernetzenden Verbindung c),
eingesetzt, mit der Maßgabe, das die Gesamtmenge der Monomere a) und b) 78 bis 99,4 Gew.-% beträgt.

In einer speziellen Ausführung zu der zuvor genannten Ausführungsform werden zur Herstellung der Copolymerzusammensetzung CP), bezogen auf das Gesamtgewicht der zur Polymerisation eingesetzten Monomere,
- 70 bis 99,4 Gew.-% Acrylsäure a),
- 0 bis 29,9 Gew.-% wenigstens einer von Acrylsäure verschiedenen Verbindung e) mit einer radikalisch polymerisierbaren α,β-ethylenisch ungesättigten Doppelbindung und mindestens einer anionogenen und/oder anionischen Gruppe pro Molekül,
- 0 bis 20 Gew.-% wenigstens eines Monomers b3), das ausgewählt ist unter C₁-C₇-Alkyl(meth)acrylaten, insbesondere Methyl(meth)acrylat, Ethyl(meth)acrylat, n-Butyl(meth)acrylat und Mischungen davon,
- 0 bis 20 Gew.-% wenigstens einer Verbindung b4), die vorzugsweise ausgewählt ist unter C₈-C₂₂-Alkyl(meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon, und
- 0,1 bis 2 Gew.-% wenigstens einer vernetzenden Verbindung c) eingesetzt, mit der Maßgabe, das die Gesamtmenge der Monomere a) und e) 78 bis 99,4 Gew.-% beträgt und die Summe aus Monomeren b3) und b4) 0,5 bis 20 Gew.-% beträgt.

In den zwei zuvor genannten Ausführungsformen wird als Komponente e) vorzugsweise Methacrylsäure eingesetzt. Ein bevorzugter Ester einer α,β-ethylenisch ungesättigten Monocarbonsäure mit einem C₁-C₇-Alkanol ist Methylmethacrylat. Eine Mischung eines C₁₈₋₂₂-Alkyl-polyethylenglykolmethacrylats mit Methylmethacrylat ist kommerziell unter der Bezeichnung Plex-6877-O erhältlich. Eine Mischung eines C₁₆₋₁₈-Alkylpolyethylenglykolmethacrylats mit Methacrylsäure ist kommerziell unter der Bezeichnung Lutencryl 250 erhältlich.

In einer weiteren bevorzugten Ausführungsform des erfindungsgemäßen Verfahrens werden zur Herstellung der Copolymerzusammensetzung CP), bezogen auf das Gesamtgewicht der zur Polymerisation eingesetzten Monomere,
- 70 bis 99,4 Gew.-% Acrylsäure a),
- 0 bis 29,4 Gew.-% wenigstens einer von Acrylsäure verschiedenen Verbindung e) mit einer radikalisch polymerisierbaren α,β-ethylenisch ungesättigten Doppelbindung und mindestens einer anionogenen und/oder anionischen Gruppe pro Molekül,
- 0,5 bis 10 Gew.-%, bezogen auf das Gesamtgewicht von a) und e), wenigstens einer Verbindung b4), die vorzugsweise ausgewählt ist unter Estern α,β-ethylenisch ungesättigter Mono- und Dicarbonsäuren mit C₈-C₃₀-Alkanolen, und
- 0,1 bis 2 Gew.-% wenigstens einer vernetzenden Verbindung c),
eingesetzt, mit der Maßgabe, das die Gesamtmenge der Monomere a) und e) 88 bis 99,4 Gew.-% beträgt.

In der zuvor genannten Ausführungsform wird als Komponente e) vorzugsweise Methacrylsäure eingesetzt.

In einer weiteren bevorzugten Ausführungsform des erfindungsgemäßen Verfahrens werden zur Herstellung der Copolymerzusammensetzung CP), bezogen auf das Gesamtgewicht der zur Polymerisation eingesetzten Monomere,
- 70 bis 99,4 Gew.-% Acrylsäure a),
- 0 bis 29,8 Gew.-% wenigstens einer von Acrylsäure verschiedenen Verbindung b) mit einer radikalisch polymerisierbaren α,β-ethylenisch ungesättigten Doppelbindung und mindestens einer anionogenen und/oder anionischen Gruppe pro Molekül,
- 0 bis 5 Gew.-%, wenigstens einer Verbindung d) mit einer radikalisch polymerisierbaren, α,β-ethylenisch ungesättigten Doppelbindung und wenigstens einer kationogenen und/oder kationischen Gruppen pro Molekül, vorzugsweise Vinylimidazol,
- 0,1 bis 30 Gew.-%, wenigstens einer amidgruppenhaltigen Verbindung e), vorzugsweise Vinylpyrrolidon und/oder Vinylcaprolactam,
- 0,1 bis 2 Gew.-% wenigstens einer vernetzenden Verbindung c),
eingesetzt, mit der Maßgabe, das die Gesamtmenge der Monomere a) und e) 65 bis 99,8 Gew.-% beträgt.
In einer speziellen Ausführung zu der zuvor genannten Ausführungsform werden zur Herstellung der Copolymerzusammensetzung CP), bezogen auf das Gesamtgewicht der zur Polymerisation eingesetzten Monomere,
- 65 bis 98,7 Gew.-% Acrylsäure a),
- 0,2 bis 5 Gew.-% Vinylimidazol,
- 1 bis 30 Gew.-% Vinylpyrrolidon und/oder Vinylcaprolactam,
- 0,1 bis 2 Gew.-% wenigstens einer vernetzenden Verbindung c),
eingesetzt.

### Fällungspolymerisation

Erfindungsgemäß erfolgt die Herstellung der Copolymerzusammensetzung CP) nach der Methode der Fällungspolymerisation. Für diese Polymerisation werden Lösungsmittel verwendet, in denen die Ausgangsstoffe für die Polymerisation löslich und das entstehende Polymer unlöslich sind. Bevorzugt wird ein wasserfreies aprotisches Lösungsmittel oder Lösungsmittelgemisch eingesetzt. Geeignete Lösungsmittel sind beispielsweise aromatische Kohlenwasserstoffe wie Toluol, Xylole, Benzol; aliphatische und cycloaliphatische Kohlenwasserstoffe wie n-Alkane oder Cyclohexan; Ester der Essigsäure wie Ethylacetat oder Butylacetat; Ether wie z. B. Diethylether, Dipropylether, Dibutylether, Methyl-tert.-butylether oder Diethylenglykoldimethylether; Ketone wie Aceton oder Methylethylketon, und Mischungen dieser Lösungsmittel.

Bevorzugt erfolgt die Polymerisation in einem Gemisch aus Cyclohexan und Ethylacetat. Das Verhältnis von Cyclohexan zu Ethylacetat liegt vorzugsweise in einem Bereich von 60 : 40 bis 30 : 70. Ein bevorzugtes Gemisch ist das azeotrope Gemisch aus 53 % Cyclohexan und 47 % Ethylacetat.

Die Fällungspolymerisation wird üblicherweise bei Temperaturen von 20 bis 150 °C, bevorzugt 40 bis 120 °C, insbesondere 60 bis100 °C durchgeführt.

Die Fällungspolymerisation wird üblicherweise bei Drücken von 1 bis 15 bar, insbesondere 1 bis 6 bar durchgeführt. Sofern die Polymerisation nicht unter erhöhtem Druck durchgeführt wird, bestimmt das Lösungsmittel bzw. Lösungsmittelgemisch durch die entsprechenden Siedetemperaturen die maximale Reaktionstemperatur.

Zur Herstellung der Polymerisate können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, tert.-Butylperoctoat, Dilauroylperoxid, tert.-Butylperisobutyrat, tert.-Butylperacetat, Di-tert.-amylperoxid, tert.-Butylhydroperoxid, 2,2'-Azo-bis-isobutyronitril, Azo-bis-(2-amidinopropan)dihydrochlorid, Azobis(2,4-dimethylvaleronitril) oder 2,2'-Azo-bis-(2-methyl-butyronitril).

In einer speziellen Ausführung werden zur Herstellung der erfindungsgemäßen Copolymere wenigstens zwei Radikalinitiatoren eingesetzt, die eine im Wesentlichen unabhängige Initiierung in wenigstens zwei Phasen ermöglichen. Dabei können Copolymere mit besonders geringen Restmonomergehalten erzielt werden. Bevorzugt werden zur Copolymerisation dann wenigstens zwei Initiatoren eingesetzt, deren Zerfallstemperaturen um wenigstens 10 °C voneinander verschieden sind. Im Rahmen der Erfindung ist die Zerfallstemperatur definiert als die Temperatur, bei der 50 % der Moleküle innerhalb von 2,5 Stunden in freie Radikale zerfallen. Vorzugsweise erfolgt die Copolymerisation bei dieser Vorgehensweise bis zum Abschluss der Fällung des Copolymers bei einer Temperatur größer oder gleich der niedrigeren Zerfallstemperatur und kleiner der höheren Zerfallstemperatur, und nach der Fällung erfolgt eine weitere Umsetzung bei einer Temperatur größer oder gleich der höheren Zerfallstemperatur.

Bevorzugt umfasst das erfindungsgemäße Verfahren eine erste Polymerisationsphase bei einer ersten Polymerisationstemperatur und eine zweite Polymerisationsphase bei einer zweiten Polymerisationstemperatur oberhalb der ersten Polymerisationstemperatur, wobei zur Polymerisation wenigstens zwei Initiatoren eingesetzt werden, deren Halbwertszeiten bei der ersten Polymerisationstemperatur sich so unterscheiden, dass wenigstens einer dieser Initiatoren während der ersten Polymerisationsphase in Radikale zerfällt und wenigstens einer dieser Initiatoren während der ersten Polymerisationsphase im Wesentlichen nicht in Radikale zerfällt und während der zweiten Polymerisationsphase in Radikale zerfällt. Vorzugsweise beginnt bei dieser Vorgehensweise die zweite Polymerisationsphäse im Wesentlichen nach Fällung des Copolymers. Unter "im Wesentlichen" nach Fällung des Copolymers wird verstanden, dass das Copolymer vorzugsweise zu mindestens 80 Gew.-%, vorzugsweise mindestens 90 Gew.-%, insbesondere wenigstens 95 Gew.-%, bezogen auf das Gesamtgewicht des Copolymers, in gefällter Form vorliegt.

Die Halbwertszeit eines Initiators kann nach üblichen, dem Fachmann bekannten Verfahren bestimmt werden, wie z. B. in der Druckschrift "Initiators for high polymers", Akzo Nobel, Nr. 10737, beschrieben. Vorzugsweise liegt die Halbwertszeit des ersten Polymerisationsinitiators bei der ersten Polymerisationstemperatur und des zweiten Polymerisationsinitiators bei der zweiten Polymerisationstemperatur in einem Bereich von etwa 1 Minute bis 3 Stunden, besonders bevorzugt 5 Minuten bis 2,5 Stunden. Gewünschtenfalls können auch kürzere Halbwertszeiten z. B. von 1 Sekunde bis 1 Minute oder längere Halbwertszeiten als 3 Stunden zum Einsatz kommen, solange sichergestellt ist, dass der/die bei der höheren Temperatur zerfallende(n) Initiator(en) im Wesentlichen während der zweiten Polymerisationsphase in Radikale zerfällt.

Vorzugsweise enthält das eingesetzte Initiatorsystem wenigstens zwei Initiatoren, deren Zerfallstemperaturen sich um wenigstens 15 °C voneinander unterscheiden. Der bei der niedrigeren Temperatur zerfallende Initiator weist vorzugsweise eine Zerfallstemperatur von 50 bis 100 °C auf. Der bei der höheren Temperatur zerfallende Initiator weist vorzugsweise eine Zerfallstemperatur von 80 bis 150 °C auf.

Im Allgemeinen kann die Fällungspolymerisation bei Feststoffgehalten bis ca. 30 % durchgeführt werden. Bevorzugt wird ein Bereich von 15 bis 26 %. Durch den Einsatz der Hilfsstoffe H1) und H2) kann in der Regel auf den Einsatz weiterer Schutzkolloide verzichtet werden. Gewünschtenfalls kann in dem erfindungsgemäßen Verfahren jedoch zusätzlich ein von H1) und H2) verschiedenes Schutzkolloid eingesetzt werden. Geeignet sind die bekannten Schutzkolloid-Polymere, die sich gut in den verwendeten Lösungsmitteln lösen und nicht mit den Monomeren reagieren. Geeignete Polymere sind z. B. Copolymere der Maleinsäure mit Vinylalkylethern und/oder Olefinen mit 8 bis 20 C-Atomen oder entsprechende Copolymere der Maleinsäurehalbester mit C₁₀-C₂₀-Alkoholen oder auch Mono- und Diamide der Maleinsäure mit C₁₀-C₂₀-Alkylaminen sowie Polyvinylalkoholether mit Alkylgruppen, die 1 bis 20 C-Atome tragen oder auch Polyvinylmethyl-, -ethyl-,-isobutyl- oder -octadecylether. Die Menge an verwendetem Schutzkolloid-Polymer beträgt in der Regel 0,05 bis 4 Gew.-%, vorzugsweise 0,1 bis 2 Gew.-% (bezogen auf das Gesamtgewicht der eingesetzten Monomere).

Die Polymerisation kann so durchgeführt werden, dass man einen Teil des Lösungsmittels, die Hilfsstoffe H1) und/oder H2) und/oder gegebenenfalls Schutzkolloidpolymer vorlegt, aufheizt und die Polymerisation durch Zugabe von Initiator, Monomer(en) und Vernetzer (jeweils eventuell gelöst im gleichen Lösungsmittel oder Lösungsmittelgemisch) durchführt.

In einer alternativen Ausführung kann man den Vernetzer c) teilweise oder vollständig vorlegen. Ebenso ist es möglich, einen Teil der Monomere und des Initiators vorzulegen (z. B. bis zu 50 %, bevorzugt bis zu 35 %). Die Vorlage kann dann auf Polymerisationstemperatur erhitzt und nach Anspringen der Reaktion den Rest der zu polymerisierenden Mischung nach Fortschritt der Polymerisation zugegeben werden.

Ebenso ist es möglich, den verwendeten Vernetzer c) nicht vorzulegen, sondern vollständig im Verlauf der Polymerisation zuzugeben.

Der Hilfsstoff H1) wird vorzugsweise zumindest teilweise vor Beginn der Polymerisation vorgelegt. Alternativ dazu kann der Hilfsstoff H1) zumindest teilweise mit einem der Monomerzuläufe oder als separater Zulauf zugegeben werden. Der Hilfsstoff H2) wird vorzugsweise zusammen mit der Acrylsäure a) zugegeben.

Das ausgefallene Polymer wird anschließend aus der Reaktionsmischung isoliert, wofür jede allgemeine Methode zur Isolierung der Polymere in der konventionellen Fällungspolymerisation verwendet werden kann. Solche Methoden sind Filtration, Zentrifugation, Eindampfen des Lösungsmittels oder Kombinationen dieser Methoden.

Die Copolymerzusammensetzung kann gewünschtenfalls einer Reinigung unterzogen werden. Diese dient z. B. zur Abtrennung von nichtpolymerisierten Bestandteilen und/oder wenigstens einem Teil der Hilfsstoffe. In einer bevorzugten Ausführung wird die Copolymerzusammensetzung CP) nach der Fällungspolymerisation isoliert und einer Wäsche mit einem flüssigen Waschmedium unterzogen. Geeignete Waschmedien sind prinzipiell die gleichen Lösungsmittel wie sie für die Polymerisation geeignet sind. Zur leichteren Trocknung der Polymere empfiehlt es sich aber, niedrig siedende Lösungsmittel, wie z. B Aceton, zu verwenden.

Zur Entfernung von Verunreinigungen kann die Copolymerzusammensetzung CP) einmal oder mehrmals nacheinander einer Behandlung mit einem Waschmedium unterzogen werden. Dazu wird die Copolymerzusammensetzung in einer geeigneten Vorrichtung mit dem Waschmedium in innigen Kontakt gebracht und das Waschmedium anschließend von der Copolymerzusammensetzung abgetrennt. Geeignete Vorrichtungen sind z. B. Rührkessel. Dabei kann die Behandlung mit dem Waschmedium in dem auch zur Polymerisation eingesetzten Behälter erfolgen. Die Trennung von Copolymer und Waschmedium erfolgt z. B. durch Filtration oder Zentrifugieren. Zur Beschleunigung kann die Filtration unter polymerseitig erhöhtem oder auslaufseitig vermindertem Druck erfolgen.

Ein weiterer Gegenstand der Erfindung ist die nach dem zuvor beschriebenen Verfahren erhältliche Copolymerzusammensetzung CP).

Die erfindungsgemäße Copolymerzusammensetzung CP) enthält zusätzlich zu den bei der Fällungspolymerisation erhaltenen Polymerteilchen in der Regel wenigstens eine der Hilfsstoffkomponenten H1) und/oder H2). In einer speziellen Ausführung werden die zur Polymerisation eingesetzten Hilfsstoffe H1) und H2) nicht aus der erfindungsgemäßen Copolymerzusammensetzung CP) entfernt. Solche Copolymerzusammensetzungen CP) weisen in der Regel besonders vorteilhafte Eigenschaften auf. Die Copolymerzusammensetzung CP) auf Basis dieses Hilfsstoffsystems lässt sich gut trocknen, die erhaltenen trockenen Zusammensetzungen sind sehr gut redispergierbar und zeichnen sich durch eine hohe Auflösungsgeschwindigkeit aus. Gewünschtenfalls lassen sich die Hilfsstoffe H1) und/oder H2) teilweise oder vollständig aus der Copolymerzusammensetzung CP) entfernen, z. B. durch wenigstens einen Waschschritt, wie zuvor beschrieben.

Die Hilfsstoffe H1) und/oder H2) können sich vorteilhaft auf weitere anwendungstechnischen Eigenschaften der Copolymerzusammensetzung CP) auswirken, z. B. durch eine Verringerung der Staubbildung, die Förderung rieselfähiger Produkte oder eine Steuerung von Teilchengröße, Molekulargewicht, Morphologie, etc.

Die Hilfsstoffe H1) und/oder H2) können sich auch vorteilhaft auf eine oder mehrere andere anwendungstechnische Eigenschaften von Formulierungen der Copolymerzusammensetzung CP) auswirken. So kann sich z. B. die Anwesenheit wenigstens eines dieser Hilfsstoffe vorteilhaft auf die Klarheit der mit CP) formulierten Gele auswirken.

Die erfindungsgemäßen Copolymerzusammensetzungen CP) und die darin enthaltenen Copolymere zeichnen sich durch ihre pH-abhängige Löslichkeit aus. Dabei sind sie vorteilhafterweise in der Regel auch in einem physiologisch verträglichen pH-Bereich von 5 bis 9 gut löslich. Des Weiteren wird in der Regel auch in diesem pH-Bereich eine gute Verdickungswirkung erzielt.

Sollen die in der Copolymerzusammensetzung CP) enthaltenen Copolymere sowohl quaternisiert als auch neutralisiert werden, so erfolgt vorzugsweise zuerst die Quaternisierung und anschließend die Neutralisierung.

Die in der Copolymerzusammensetzung CP) enthaltenen Copolymere eignen sich in vorteilhafter Weise zur Modifizierung der rheologischen Eigenschaften wässriger Zusammensetzungen. Dabei kann es sich z. B. um eine wässriger Wirk- oder Effektstoffzusammensetzung handeln. Es kann sich ganz allgemein beispielsweise um kosmetische Zusammensetzungen, pharmazeutische Zusammensetzungen, Hygieneprodukte, Anstrichmittel, Zusammensetzungen für die Papierindustrie sowie die Textilindustrie handeln.

In einer bevorzugten Ausführungsform enthalten die Zusammensetzungen wenigstens einen wasserlöslichen oder zumindest wasserdispergierbaren Wirk- oder Effektstoff. Selbstverständlich eignen sich die in der Copolymerzusammensetzung CP) enthaltenen Copolymere auch zur Modifizierung der rheologischen Eigenschaften von Zusammensetzungen, die wenigstens einen wasserunlöslichen (hydrophoben) Wirk- oder Effektstoff enthalten.

"Modifizierung rheologischer Eigenschaften" wird im Rahmen der vorliegenden Erfindung weit verstanden. Die in der Copolymerzusammensetzung CP) enthaltenen Copolymere eignen sich im Allgemeinen zur Verdickung der Konsistenz von wässrigen Zusammensetzungen in einem weiten Bereich. Je nach Grundkonsistenz der flüssigen Zusammensetzungen können in Abhängigkeit von der Einsatzmenge des Copolymers in der Regel Fließeigenschaften von dünnflüssig bis hin zu fest (im Sinne von "nicht mehr fließend") erzielt werden. Unter "Modifizierung rheologischer Eigenschaften" wird daher u. a. die Erhöhung der Viskosität von Flüssigkeiten, die Verbesserung der Tixotropie-Eigenschaften von Gelen, die Verfestigung von Gelen und Wachsen etc. verstanden. Die erfindungsgemäßen Mittel eignen sich vorzugsweise zur Formulierung von wässrigen kosmetischen und pharmazeutischen Produkten. Vorzugsweise sind dabei die Zusammensetzungen der Copolymere CP) im Allgemeinen klar. Vorteilhafterweise können somit Formulierungen, insbesondere kosmetische Formulierungen, ohne Beeinträchtigung durch die Eigenfarbe der Zusammensetzungen angefärbt werden. Des Weiteren können die Zusammensetzungen in Form von klaren Gelen formuliert werden.

Die in Gegenwart des erfindungsgemäßen Hilfsstoffsystems hergestellten Copolymerzusammensetzungen CP) zeichnen sich insgesamt durch vorteilhafte rheologische Eigenschaften aus. Eine weitere Steuerung der rheologiemodifizierenden Eigenschaften kann über die Art und Einsatzmenge der zur Herstellung der Copolymerzusammensetzungen CP) eingesetzten Monomere erfolgen. Dies gilt speziell für die Art und Menge des eingesetzten Vernetzers c). Dies gilt weiterhin speziell für den Einsatz oberflächenaktiver Monomere bei der Herstellung von CP), wie z. B. die Polyetheracrylate IV a) oder Allylalkoholalkoxilate IV b).

Eine 0,2 gew.-%ige wässrige Lösung einer Copolymerzusammensetzungen CP) weist im Allgemeinen eine Viskosität im Bereich von 7000 bis 15 000 mPas (Werte bestimmt mittels Brookfield-Viskosimeter bei 23 °C und 100 s⁻¹) auf.

Eine 0,5 gew.-%ige wässrige Lösung einer Copolymerzusammensetzungen CP) weist im Allgemeinen eine Viskosität im Bereich von 15 000 bis 60 000 mPas (Werte bestimmt mittels Brookfield-Viskosimeter bei 23 °C und 100 s⁻¹) auf.

Die Copolymerzusammensetzungen CP) eignen sich sowohl zur Herstellung homogenphasiger wässriger Zusammensetzungen als auch zur Formulierung von heterogenphasigen Zusammensetzungen, die zusätzlich wenigstens eine wasserunlösliche (hydrophobe) flüssige oder feste Verbindung umfassen. "Homogenphasige Zusammensetzungen" weisen unabhängig von der Anzahl ihrer Bestandteile nur eine einzige Phase auf. "Heterogenphasige Zusammensetzungen" sind disperse Systeme von zwei oder mehreren miteinander nicht mischbaren Komponenten. Dazu zählen fest/flüssig-, flüssig/flüssig- und fest/flüssig/flüssig-Zusammensetzungen, wie Dispersionen und Emulsionen, z. B. O/W- und W/O-Formulierungen, die wenigstens eine der im Folgenden näher beschriebenen Öl- bzw. Fettkomponenten und Wasser als nicht mischbare Phasen aufweisen. Prinzipiell können die Copolymere CP) sowohl in der Wasserphase als auch in der Ölphase eingesetzt werden. Im Allgemeinen enthalten heterogenphasige flüssig/flüssig-Zusammensetzungen die Copolymere CP) im Wesentlichen in der Wasserphase.

Die erfindungsgemäßen Copolymerzusammensetzungen CP) eignen sich ganz allgemein zur Herstellung von Wirk- oder Effektstoffzusammensetzungen, enthaltend
A) wenigstens eine Copolymerzusammensetzung CP), wie zuvor definiert,
B) wenigstens einen Wirk- oder Effektstoff und
C) gegebenenfalls wenigstens einen weiteren, von A) und B) verschiedenen Hilfsstoff.

Wirkstoffe für Kosmetika (z. B. Haar- oder Hautkosmetika), Arzneimittel, Hygienemittel, Textilbehandlungsmittel etc., d. h. Substanzen, die im Allgemeinen bereits in geringer Konzentration eine Wirkung entfalten, z. B. eine kosmetische Wirkung auf Haut und/oder Haaren, eine pharmakologische Wirkung in einem Organismus, eine reinigende und/oder desinfizierende Wirkung, eine Modifizierung eines textilen Stoffes, z. B. eine knitterfreie Ausrüstung, sowie Effektstoffe, die Lebewesen oder unbelebten Substraten eine bestimmte Eigenschaft verleihen, beispielsweise Farbpigmente für Schminken oder Dispersionsfarben, werden häufig in Form wässriger Wirk- oder Effektstoffzusammensetzungen formuliert und angewendet.

Die erfindungsgemäßen Wirk- und Effektstoffzusammensetzungen enthalten die Polymerkomponente A) vorzugsweise in einer Menge von 0,01 bis 50 Gew.-%, besonders bevorzugt 0,05 bis 30 Gew.-%, insbesondere 0,1 bis 20 Gew.-%, bezogen auf das Gesamtgewicht des Mittels. Vorteilhafterweise zeigen die erfindungsgemäßen Copolymerzusammensetzungen bereits in geringen Einsatzmengen gute anwendungstechnische Eigenschaften, z. B. eine gute verdickende Wirkung. In einer speziellen Ausführung enthalten die erfindungsgemäßen Wirk- und Effektstoffzusammensetzungen Polymerkomponente A) in einer Menge von 0,1 bis 5 Gew.-%, bezogen auf das Gesamtgewicht des Mittels.

Die Komponenten B) und C) werden nach dem gewünschten Einsatzbereich der Zusammensetzung ausgewählt. Neben einsatzbereichtypischen Komponenten (z. B. bestimmten pharmazeutischen Wirkstoffen) sind sie z. B. ausgewählt unter Trägern, Exzipienten, Emulgatoren, Tensiden, Konservierungsmitteln, Duftstoffen, von Komponente A) verschiedenen Verdickern, Polymeren, Gelbildnern, Farbstoffen, Pigmenten, Lichtschutzmitteln, Konsistenzgebern, Antioxidantien, Entschäumern, Antistatika, Harzen, Lösemitteln, Lösungsvermittlern, Neutralisierungsmitteln, Stabilisatoren, Sterilantien, Treibmitteln, Trocknungsmitteln, Trübungsmitteln, etc.

Vorzugsweise weisen die Zusammensetzungen eine Trägerkomponente C) auf, die ausgewählt ist unter Wasser, von Wasser verschiedenen hydrophilen Trägern und Mischungen davon.

Geeignete hydrophile Träger C) sind z. B. ein-, zwei- oder mehrwertige Alkohole mit vorzugsweise 1 bis 8 Kohlenstoffatomen, wie Ethanol, n-Propanol, Isopropanol, Propylenglycol, Glycerin, Sorbit, etc.

Die erfindungsgemäßen Zusammensetzungen können als Wirkstoff, z. B. als kosmetischen und/oder pharmazeutischen Wirkstoff B) (wie auch gegebenenfalls als Hilfsstoff C)), wenigstens ein Polymer enthalten, das sich von den erfindungsgemäßen Copolymerzusammensetzungen CP) unterscheidet. Dazu zählen ganz allgemein anionische, kationische, amphotere und neutrale Polymere.

Beispiele für anionische Polymere sind Copolymere von Acrylsäure und Acrylamid und deren Salze; Natriumsalze von Polyhydroxycarbonsäuren, wasserlösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z. B. Luviset®-Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. C₄-C₃₀-Alkylester der (Meth)acrylsäure, C₄-C₃₀-Alkylvinylester, C₄-C₃₀-Alkylvinylether und Hyaluronsäure. Ein Beispiel für ein anionisches Polymer ist weiterhin das unter der Bezeichnung Luviset® Shape (INCI Name: Polyacrylate-22) erhältliche Methylmethacrylat/Methacrylsäure/Acrylsäure/Urethanacrylat-Copolymer. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester. Weiterhin geeignet sind Vinylpyrrolidon/Ethylmethacrylat/Methacrylsäure-Copolymere wie sie von der Fa. Stepan unter den Bezeichnungen Stepanhold-Extra und - R1 vertrieben werden und die Carboset®-Marken der Fa. BF Goodrich.

Geeignete kationische Polymere sind z. B. kationische Polymere mit der Bezeichnung Polyquaternium nach INCI, z. B. Copolymere aus Vinylpyrrolidon/N-Vinylimidazoliumsalzen (Luviquat® FC, Luviquat® HM, Luviquat® MS, Luviset Clear ®, Luviquat Supreme ®, Luviquat® Care), Copolymere aus N-Vinylpyrrolidon/Dimethylaminoethyl-methacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Ganz besonders geeignete Polymere sind neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF SE), oder Polyvinylpyrrolidon und deren Copolymere, insbesondere mit Vinylestern, wie Vinylacetat, z. B. Luviskol® VA 37, VA 55, VA 64, VA 73 (BASF SE); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/- Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

In einer speziellen Ausführung enthalten die erfindungsgemäßen Zusammensetzungen wenigstens ein Polymer, das sich von den in den Copolymerzusammensetzungen CP) enthaltenen Polymeren unterscheidet und das als Verdicker wirkt.

Geeignete polymere Verdicker sind beispielsweise gegebenenfalls modifizierte polymere Naturstoffe (Carboxymethylcellulose und andere Celluloseether, Hydroxyethyl- und -propylcellulose und dergleichen) sowie synthetische polymere Verdicker (Polyacryl- und Polymethacryl-Verbindungen, Vinylpolymere, Polycarbonsäuren, Polyether, Polyimine, Polyamide). Dazu zählen die zum Teil bereits zuvor genannten Polyacryl- und Polymethacryl-Verbindungen, beispielsweise die hochmolekularen mit einem Polyalkenylpolyether, insbesondere einem Allylether von Saccharose, Pentaerythrit oder Propylen, vernetzten Homopolymere der Acrylsäure (INCI-Bezeichnung: Carbomer). Solche Polyacrylsäuren sind u. a. von der Fa. BF Goodrich unter dem Handelsnamen Carbopol ® erhältlich, z. B. Carbopol 940 (Molekulargewicht ca. 4 000 000), Carbopol 941 (Molekulargewicht ca. 1 250 000) oder Carbopol 934 (Molekulargewicht ca. 3 000 000). Weiterhin fallen darunter Acrylsäure-Copolymere, die beispielsweise von der Fa. Rohm & Haas unter den Handelsnamen Aculyn ® und Acusol ® erhältlich sind, z. B. die anionischen, nicht-assoziativen Polymere Aculyn 22, Aculyn 28, Aculyn 33 (vernetzt), Acusol 810, Acusol 823 und Acusol 830 (CAS 25852-37-3). Speziell geeignet sind auch assoziative Verdicker, z. B. auf Basis modifizierter Polyurethane (HEUR) oder hydrophob modifizierter Acryl- oder Methacrylsäure-Copolymere (HASE-Verdicker, High Alkali Swellable Emulsion).

Die Einsatzmenge der zusätzlichen Verdicker liegt vorzugsweise in einem Bereich von 0,001 bis 5 Gew.-%, bevorzugt 0,1 bis 3 %, bezogen auf das Gesamtgewicht der Zusammensetzung.

Beispiele für Effektstoffe, die als erfindungsgemäße wässrige Wirkstoffzusammensetzung formuliert werden können, sind Farbstoffe: z. B. die in DE-A 102 45 209 beschriebenen Farbstoffe sowie die gemäß Colour-Index als Disperse-Farbstoffe und als Solvent-Farbstoffe bezeichneten Verbindungen, die auch als Dispersionsfarbstoffe bezeichnet werden. Eine Zusammenstellung geeigneter Dispersionsfarbstoffe findet sich beispielsweise in Ullmanns Enzyklopädie der technischen Chemie, 4. Auflage, Bd. 10, S. 155-165 (siehe auch Bd. 7, S. 585ff - Anthrachinonfarbstoffe; Bd. 8, S. 244ff - Azofarbstoffe; Bd. 9, S. 313ff - Chinophthalonfarbstoffe). Auf diese Literaturstelle und die darin genannten Verbindungen wird hiermit ausdrücklich Bezug genommen. Erfindungsgemäß geeignete Dispersionsfarbstoffe und Solvent-Farbstoffe umfassen verschiedenste Farbstoffklassen mit unterschiedlichen Chromophoren, beispielsweise Anthrachinonfarbstoffe, Monoazo- und Disazofarbstoffe, Chinophthalone, Methin- und Azamethinfarbstoffe, Naphthalimidfarbstoffe, Naphthochinonfarbstoffe und Nitrofarbstoffe. Beispiele für erfindungsgemäß geeignete Dispersionsfarbstoffe sind die Dispersionsfarbstoffe der folgenden Colour-Index Liste: C. I. Disperse Yellow 1 - 228, C. I. Disperse Orange 1 - 148, C. I. Disperse Red 1 - 349, C. I. Disperse Violet 1 - 97, C. I. Disperse Blue 1 - 349, C. I. Disperse Green 1 - 9, C. I. Disperse Brown 1 - 21, C. I. Disperse Black 1 - 36. Beispiele für erfindungsgemäß geeignete Solvent-Farbstoffe sind die Verbindungen der folgenden Colour-Index Liste: C. I. Solvent Yellow 2 - 191, C. I. Solvent Orange 1 - 113, C. I. Solvent Red 1 - 248, C. I. Solvent Violet 2 - 61, C. I. Solvent Blue 2 - 143, C. I. Solvent Green 1 - 35, C. I. Solvent Brown 1 - 63, C. I. Solvent Black 3 - 50. Erfindungsgemäß geeignete Farbstoffe sind weiterhin Derivate des Naphthalins, des Anthracens, des Perylens, des Terylens, des Quarterylens, sowie Diketopyrrolopyrrolfarbstoffe, Perinonfarbstoffe, Cumarinfarbstoffe, Isoindolin- und Isoindolinonfarbstoffe, Porphyrinfarbstoffe, Phthalocyanin- und Naphthalocyaninfarbstoffe.

Neben den vorgenannten Bestandteilen können die erfindungsgemäßen Wirk- und Effektstoffzusammensetzungen auch konventionelle oberflächenaktive Substanzen und sonstige Additive enthalten. Zu den oberflächenaktiven Substanzen zählen Tenside, Dispergierhilfsmittel und Netzmittel. Zu den sonstigen Additiven zählen insbesondere Verdickungsmittel, Entschäumer, Konservierungsmittel, Frostschutzmittel, Stabilisierungsmittel, etc.

Prinzipiell brauchbar sind anionische, kationische, nichtionische und amphotere Tenside, wobei Polymertenside sowie Tenside mit Heteroatomen in der hydrophoben Gruppe eingeschlossen sind.

Zu den anionischen Tensiden gehören beispielsweise Carboxylate, insbesondere Alkali-, Erdalkali- und Ammoniumsalze von Fettsäuren, z. B. Kaliumstearat, die üblicherweise auch als Seifen bezeichnet werden; Acylglutamate; Sarkosinate, z. B. Natriumlauroylsarkosinat; Taurate; Methylcellulosen; Alkylphosphate, insbesondere Mono- und Diphosphorsäurealkylester; Sulfate, insbesondere Alkylsulfate und Alkylethersulfate; Sulfonate, weitere Alkyl- und Alkylarylsulfonate, insbesondere Alkali-, Erdalkali- und Ammoniumsalze von Arylsulfonsäuren sowie alkylsubstituierten Arylsulfonsäuren, Alkylbenzolsulfonsäuren, wie beispielsweise Lignin- und Phenolsulfonsäure, Naphthalinund Dibutylnaphthalinsulfonsäuren, oder Dodecylbenzolsulfonate, Alkylnaphthalinsulfonate, Alkylmethylestersulfonate, Kondensationsprodukte von sulfoniertem Naphthalin und Derivaten davon mit Formaldehyd, Kondensationsprodukte von Naphthalinsulfonsäuren, Phenol- und/oder Phenolsulfonsäuren mit Formaldehyd oder mit Formaldehyd und Harnstoff, Mono- oder Dialkylbernsteinsäureestersulfonate; sowie Eiweißhydrolysate und Lignin-Sulfitablaugen. Die zuvor genannten Sulfonsäuren werden vorteilhafterweise in Form ihrer neutralen oder gegebenenfalls basischen Salze verwendet.

Zu den kationischen Tensiden gehören beispielsweise quaternierte Ammoniumverbindungen, insbesondere Alkyltrimethylammonium- und Dialkyldimethylammonium-Halogenide und -alkylsulfate sowie Pyridin- und Imidazolin-Derivate, insbesondere Alkylpyridinium-Halogenide.

Zu den nichtionischen Tensiden gehören beispielsweise:
- Fettalkoholpolyoxyethylenester, beispielsweise Laurylalkoholpolyoxyethylenetheracetat,
- Alkyl-Polyoxyethylen- und -polyoxypropylenether, z. B. von iso-Tridecylalkohol und Fettalkohol-Polyoxyethylenether,
- Alkylarylalkohol-Polyoxyethylenether, z. B. Octylphenol-Polyoxyethylenether,
- alkoxylierte tierische und/oder pflanzliche Fette und/oder Öle, beispielsweise Maisölethoxylate, Rizinusölethoxylate, Talgfettethoxylate,
- Glycerinester, wie beispielsweise Glycerinmonostearat,
- Fettalkoholalkoxylate und Oxoalkoholalkoxylate, insbesondere vom Typ RO-(R₁₈O)ᵣ(R₁₉O)ₛR₂₀ mit R₁₈ und R₁₉ unabhängig voneinander = C₂H₄, C₃H₆, C₄H₈ und R₂₀ = H, oder C₁-C₁₂-Alkyl, R = C₃-C₃₀-Alkyl oder C₆-C₃₀-Alkenyl, r und s unabhängig voneinander 0 bis 50, wobei nicht beide für 0 stehen können, wie iso-Tridecylalkohol und Oleylalkoholpolyoxyethylenether,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Zuckertenside, Sorbitester, wie beispielsweise Sorbitanfettsäureester (Sorbitanmonooleat, Sorbitantristearat), Polyoxyethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Zu den amphoteren Tensiden gehören beispielsweise Sulfobetaine, Carboxybetaine und Alkyldimethylaminoxide, z. B. Tetradecyldimethylaminoxid.

Weitere Tenside, die hier beispielhaft genannt werden sollen, sind Perfluortenside, Silicontenside, Phospholipide, wie beispielsweise Lecithin oder chemisch modifizierte Lecithine, Aminosäuretenside, z. B. N-Lauroylglutamat.

Sofern nicht spezifiziert, handelt es sich bei den Alkylketten der oben aufgeführten Tenside um lineare oder verzweigte Reste mit üblicherweise 8 bis 20 Kohlenstoffatomen.

Die erfindungsgemäßen Wirk- oder Effektstoffzusammensetzungen können wasserlösliche Salze als Komponente B) und/oder C) enthalten, z. B. NaCl.

Die erfindungsgemäßen Wirk- oder Effektstoffzusammensetzungen können für einige Anwendungen organische Lösungsmittel, Öle und/oder Fette enthalten. Bevorzugt sind solche Lösungsmittel, Öle und/oder Fette, die umweltverträglich oder biokompatibel sind. Dazu zählen z. B.:
- Paraffinöle, aromatische Kohlenwasserstoffe und aromatische Kohlenwasserstoffgemische, z. B. Xylole, Solvesso 100, 150 oder 200, und dergleichen,
- Phenole und Alkylphenole, z. B. Phenol, Hydrochinon, Nonylphenol, etc.
- Ketone mit mehr als 4 C-Atomen wie Cyclohexanon, Isophoron, Isopheron, Acetophenon, Acetonaphthon,
- Alkohole mit mehr als 4 C-Atomen wie acetylierter Lanolinalkohol, Cetylalkohol, 1-Decanol, 1-Heptanol, 1-Hexanol, Isooctadecanol, Isopropylalkohol, Oleylalkohol, Benzylalkohol,
- Carbonsäureester, z. B. Adipinsäuredialkylester wie Adipinsäurebis(2-ethylhexyl)ester, Phthalsäuredialkylester wie Phthalsäurebis(2-ethylhexyl)ester, Essigsäurealkylester (auch verzweigte Alkylgruppen) wie Ethylacetat und Acetoessigsäureethylester, Stearate wie Butylstearat, Glycerinmonostearat, Citrate wie Acetyltributylcitrat, weiterhin Cetyloctanoat, Methyloleat, Methyl-p-hydroxybenzoat, Methyltetradecanoat, Propyl-p-hydroxybenzoat, Methylbenzoat, Milchsäureester wie Isopropyllactat, Butyllactat und 2-Ethylhexyllactat,
- Pflanzenöle wie Palmöl, Rapsöl, Rizinusöl und Derivate davon wie z. B. oxydiert, Kokosnussöl, Lebertran, Maiskeimöl, Sojabohnenöl, Leinsamenöl, Olivenöl, Erdnussöl, Färberdistelöl, Sesamsamenöl, Grapefruitöl, Basilikumöl, Aprikosenöl, Ingweröl, Geranienöl, Orangenöl, Rosmarinöl, Macadamiaöl, Zwiebelöl, Mandarinenöl, Kiefernöl, Sonnenblumenöl,
- hydrogenierte Pflanzenöle wie hydrogeniertes Palmöl, hydrogeniertes Rapsöl, hydrogeniertes Sojabohnenöl,
- tierische Öle wie Schweinefettöl, Fischöle,
- Dialkylamide mittel- bis langkettiger Fettsäuren, z. B. Hallcomide, sowie
- Pflanzenölester wie Rapsölmethylester.

Die Copolymerzusammensetzungen CP) können gemeinsam mit konventionellen Verdickern eingesetzt werden. Dazu zählen die zuvor genannten als Verdicker wirksamen Polymere. Dazu zählen weiterhin Polysaccharide und organische Schichtmineralien wie Xanthan Gum^{®} (Kelzan^{®} der Fa. Kelco), Rhodopol^{®} 23 (Rhone Poulenc) oder Veegum^{®} (Firma R. T. Vanderbilt) oder Attaclay^{®} (Firma Engelhardt). Geeignete Verdickungsmittel sind auch organische natürliche Verdickungsmittel (Agar-Agar, Carrageen, Tragant, Gummi arabicum, Alginate, Pektine, Polyosen, Guar-Mehl, Johannisbrotbaumkernmehl, Stärke, Dextrine, Gelatine, Casein) und anorganische Verdickungsmittel (Polykieselsäuren, Tonmineralien wie Montmorillonite, Zeolithe, Kieselsäuren). Weitere Verdickungsmittel sind die Polysaccharide und Heteropolysaccharide, insbesondere die Polysaccharidgummen, beispielsweise Gummi arabicum, Agar, Alginate, Carrageene und ihre Salze, Guar, Guaran, Traganth, Gellan, Ramsan, Dextran oder Xanthan und ihre Derivate, z. B. propoxyliertes Guar, sowie ihre Mischungen. Andere Polysaccharidverdicker sind beispielsweise Stärken verschiedensten Ursprungs und Stärkederivate, z. B. Hydroxyethylstärke, Stärkephosphatester oder Stärkeacetate, oder Carboxymethylcellulose bzw. ihr Natriumsalz, Methyl-, Ethyl-, Hydroxyethyl-, Hydroxypropyl-, Hydroxypropyl-methyl- oder Hydroxyethyl-methyl-cellulose oder Celluloseacetat. Als Verdickungsmittel können weiterhin Schichtsilikate eingesetzt werden. Hierzu zählen beispielsweise die unter dem Handelsnamen Laponite® erhältlichen Magnesium- oder Natrium-Magnesium-Schichtsilikate der Firma Solvay Alkali sowie die Magnesiumsilikate der Firma Süd-Chemie.

Die Einsatzmenge der zusätzlichen Verdicker liegt vorzugsweise in einem Bereich von 0,001 bis 10 Gew.-%, bevorzugt 0,1 bis 5 %, bezogen auf das Gesamtgewicht der Zusammensetzung.

Als für erfindungsgemäße Dispersionen geeignete Antischaummittel kommen beispielsweise Siliconemulsionen (wie z. B. Silikon® SRE, Firma Wacker oder Rhodorsil® der Firma Rhodia), langkettige Alkohole, Fettsäuren, fluororganische Verbindungen und deren Gemische in Betracht.

Bakterizide können zur Stabilisierung den erfindungsgemäßen Zusammensetzungen gegen Befall mit Mikroorganismen zugesetzt werden. Geeignete Bakterizide sind beispielsweise Proxel® der Fa. ICI oder Acticide® RS der Fa. Thor Chemie und Kathon® MK der Firma Rohm & Haas.

Geeignete Frostschutzmittel sind organische Polyole, z. B. Ethylenglycol, Propylenglycol oder Glycerin. Diese werden üblicherweise in Mengen von nicht mehr als 10 Gew.-%, bezogen auf das Gesamtgewicht der Wirkstoffzusammensetzung eingesetzt, um den gewünschten Gehalt an flüchtigen Verbindungen nicht zu überschreiten. In einer Ausführungsform der Erfindung beträgt der Anteil hiervon verschiedener flüchtiger organischer Verbindungen vorzugsweise nicht mehr als 1 Gew.-%, insbesondere nicht mehr als 1000 ppm.

Gegebenenfalls können die erfindungsgemäßen Wirkstoffzusammensetzungen 1 bis 5 Gew.-% Puffer, bezogen auf die Gesamtmenge der hergestellten Formulierung, zur pH-Wert Regulation enthalten, wobei sich die Menge und Art des eingesetzten Puffers nach den chemischen Eigenschaften des Wirkstoffes bzw. der Wirkstoffe richtet. Beispiele für Puffer sind Alkalisalze schwacher anorganischer oder organischer Säuren wie z. B. Phosphorsäure, Borsäure, Essigsäure, Propionsäure, Citronensäure, Fumarsäure, Weinsäure, Oxalsäure und Bernsteinsäure.

In einer besonders bevorzugten Ausführungsform werden die erfindungsgemäßen Copolymere als Komponente in einem kosmetischen Mittel eingesetzt. Sie können dabei, wie zuvor beschrieben, zur Modifizierung der rheologischen Eigenschaften eines kosmetischen Mittels auf Basis eines wässrigen Mediums dienen.

Ein weiterer Gegenstand der Erfindung ist ein kosmetisches Mittel, enthaltend
A) wenigstens eine Copolymerzusammensetzung CP), erhältlich durch ein Verfahren, wie zuvor definiert,
B) wenigstens einen kosmetisch akzeptablen Wirkstoff und
C) gegebenenfalls wenigstens einen weiteren, von A) und B) verschiedenen kosmetisch akzeptablen Hilfsstoff.

Bevorzugt umfasst die Komponente C) wenigstens einen kosmetisch oder pharmazeutisch akzeptablen Träger.

Vorzugsweise ist die Träger-Komponente C) ausgewählt unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

Geeignete hydrophile Komponenten C) sind die zuvor genannten organischen Lösungsmittel, Öle und Fette.

Speziell geeignete kosmetisch verträgliche Öl- bzw. Fettkomponenten C) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Bei den erfindungsgemäßen kosmetischen Mitteln kann es sich um hautkosmetische, haarkosmetische, dermatologische, hygienische oder pharmazeutische Mittel handeln. Aufgrund ihrer verdickenden Eigenschaften eignen sich die zuvor beschriebenen Copolymerzusammensetzungen CP) insbesondere als Zusatzstoffe für Haar- und Hautkosmetika. Sie eignen sich speziell zur Formulierung von Gelen.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch aktiven Mittel können zusätzlich kosmetisch und/oder dermatologisch aktive Wirk- und Effektstoffe sowie Hilfsstoffe enthalten. Geeignet sind prinzipiell die zuvor genannten Wirk- und Effektstoffe B) sowie Hilfsstoffe C).

Vorzugsweise enthalten die erfindungsgemäßen kosmetischen Mittel wenigstens eine wie vorstehend definierte Copolymerzusammensetzung CP), wenigstens einen wie vorstehend definierten Träger C) und wenigstens einen davon verschiedenen Bestandteil, der vorzugsweise ausgewählt ist unter kosmetisch aktiven Wirkstoffen, Emulgatoren, Tensiden, Konservierungsmitteln, Parfümölen, zusätzlichen Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien und Weichmachern.

Zusätzlich zu den Copolymerzusammensetzungen CP) für den Einsatz in kosmetischen Mitteln geeignete konventionelle Verdickungsmittel sind die zuvor genannten.

Geeignete kosmetisch und/oder dermatologisch aktive Wirkstoffe sind z. B. Haut- und Haarpigmentierungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, desodorierende Wirkstoffe, sebostatische Wirkstoffe, Pflanzenextrakte, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B-und/oder UV-A-Bereich absorbieren. Geeignete UV-Filter sind die zuvor genannten. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen Wirkstoff (wie auch gegebenenfalls als Hilfsstoff) wenigstens ein kosmetisch oder pharmazeutisch akzeptables Polymer enthalten, das sich von den erfindungsgemäßen Copolymeren CP) unterscheidet. Dazu zählen ganz allgemein anionische, kationische, amphotere und neutrale Polymere. Auf die zuvor genannten Polymere wird hier voll Bezug genommen.

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -Iotionen und -cremes.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyeliner, Rouges, Puder und Augenbrauenstifte.

Außerdem können die Copolymerzusammensetzungen CP) verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Bei den erfindungsgemäßen Hautpflegemitteln handelt es sich insbesondere um W/O-oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Copolymere CP) zeigen vorteilhafte Wirkungen. Die Polymere können unter anderem zur Feuchthaltung und Konditionierung der Haut und zur Verbesserung des Hautgefühls beitragen. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens eine Copolymerzusammensetzungen CP) in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder.treibmittelhaltiger Spray) oder Lotion, appliziert werden.

Die hautkosmetischen Zubereitungen können neben den Copolymerzusammensetzungen CP) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäßen Polymere auch mit herkömmlichen Polymeren, wie zuvor beschrieben, abmischen, falls spezielle Eigenschaften eingestellt werden sollen.

Zur Einstellung bestimmter Eigenschaften, wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/O)- oder Öl-in-Wasser-(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einer Copolymerzusammensetzung CP) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstypspezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann eine Copolymerzusammensetzung CP) eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Neben den Copolymerzusammensetzungen CP) können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens eine Copolymerzusammensetzung CP) sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionischen, neutralen, amphoteren oder kationischen Tenside verwendet werden.

Geeignete Tenside sind die zuvor Genannten.

Weiterhin können die Duschgel-/Shampoo-Formulierungen zusätzliche Verdicker, wie z. B. Kochsalz, PEG-55, Propylenglykol-Oleat, PEG-120-Methylglucosedioleat und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens eine Copolymerzusammensetzung CP) in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen oder "Hot-Oil-Treatments" vor. Je nach Anwendungsgebiet können die haarkosmetischen Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform
a) 0,05 bis 5 Gew.-%, vorzugsweise 0,1 bis 3 Gew.-%, wenigstens einer Copolymerzusammensetzung CP),
b) 20 bis 99,95 Gew.-% Wasser und/oder Alkohol,
c) 0 bis 50 Gew.-% wenigstens eines Treibgases,
d) 0 bis 5 Gew.-% wenigstens eines Emulgators,
e) 0 bis 3 Gew.-% wenigstens eines von a) verschiedenen Verdickers, sowie
f) 0 bis 20 Gew.-%, vorzugsweise 0,1 bis 10 Gew.-%, wenigstens eines von a) bis e) und g) verschiedenen wasserlöslichen oder wasserdispergierbaren Polymers,
g) 0 bis 45 Gew.-%, vorzugsweise 0,05 bis 25 Gew.-%, weitere Bestandteile,
wobei sich die Komponenten a) bis g) zu 100 Gew.-% addieren.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Copolymerzusammensetzungen eignen sich insbesondere als Verdicker in Haarstyling-Zubereitungen, insbesondere Haarschäumen und Haargelen.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Eine erfindungsgemäß für Styling-Gele geeignete Zubereitung kann beispielsweise wie folgt zusammengesetzt sein:
a) 0,1 bis 5 Gew.-% wenigstens einer Copolymerzusammensetzung CP),
b) 0 bis 5 Gew.-% wenigstens eines von CP) verschiedenen kosmetisch akzeptablen wasserlöslichen oder wasserdispergierbaren Haarfestigerpolymers,
c) 80 bis 99,85 Gew.-% Wasser und/oder Alkohol,
d) 0 bis 1 Gew.-% eines von CP) verschiedenen Gelbildners,
e) 0 bis 20 Gew.-% weitere Bestandteile.

Als zusätzliche Gelbildner können alle in der Kosmetik üblichen Gelbildner eingesetzt werden. Hierzu wird auf die zuvor genannten konventionellen Verdicker Bezug genommen.

Die erfindungsgemäßen Copolymerzusammensetzungen CP) eignen sich auch für Shampooformulierungen, die zusätzlich übliche Tenside enthalten.

In den Shampooformulierungen können zur Erzielung bestimmter Effekte übliche Konditioniermittel in Kombination mit den Copolymerzusammensetzungen CP) eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Die erfindungsgemäß zu verwendenden Copolymerzusammensetzungen CP) eignen sich ebenso für die Verwendung zur Modifizierung der rheologischen Eigenschaften in pharmazeutischen Zubereitungen jeder Art.

Ein weiterer Gegenstand der Erfindung ist daher ein pharmazeutisches Mittel, enthaltend
A) wenigstens eine Copolymerzusammensetzung CP), wie zuvor definiert,
B) wenigstens einen pharmazeutisch akzeptablen Wirkstoff und
C) gegebenenfalls wenigstens einen weiteren, von A) und B) verschiedenen pharmazeutisch akzeptablen Hilfsstoff.

Die Formulierungsgrundlage der erfindungsgemäßen pharmazeutischen Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB, Ph. Eur., BP, NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, zusätzliche Verdicker, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung von erfindungsgemäßen pharmazeutischen Mitteln können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Insbesondere handelt es sich dabei um wässrige Lösungen bzw. Solubilisate zur oralen oder zur parenteralen Applikation. Des Weiteren eignen sich die erfindungsgemäß zu verwendenden Copolymere auch zum Einsatz in oralen Darreichungsformen wie Tabletten, Kapseln, Pulvern, Lösungen. Hier können sie den schwerlöslichen Arzneistoff mit einer erhöhten Bioverfügbarkeit zur Verfügung stellen. Bei der parenteralen Applikation können neben Solubilisaten auch Emulsionen, beispielsweise Fettemulsionen eingesetzt werden.

Pharmazeutische Formulierungen der oben genannten Art können durch Verarbeiten der erfindungsgemäß zu verwendenden Copolymerzusammensetzungen CP) mit pharmazeutischen Wirkstoffen nach herkömmlichen Methoden und unter Einsatz bekannter und neuer Wirkstoffe erhalten werden.

Die erfindungsgemäße Anwendung kann zusätzlich pharmazeutische Hilfsstoffe und/oder Verdünnungsmittel enthalten. Als Hilfsstoffe werden Cosolventien, Stabilisatoren, Konservierungsmittel besonders aufgeführt.

Die verwendeten pharmazeutischen Wirkstoffe sind in Wasser lösliche oder auch unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10 000 Teilen Lösungsmittel). Die Wirkstoffe können dabei aus jedem Indikationsbereich kommen.

Der Gehalt an Copolymer CP) in den pharmazeutischen Mitteln liegt, abhängig vom Wirkstoff, im Bereich von 0,01 bis 50 Gew.-%, bevorzugt 0,1 bis 40 Gew.-%, besonders bevorzugt 1 bis 30 Gew.-%, bezogen auf das Gesamtgewicht des Mittels.

Zur Herstellung der erfindungsgemäßen pharmazeutischen Mittel eignen sich prinzipiell alle pharmazeutischen Wirkstoffe und Pro-Drugs. Hierzu zählen Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel. Beispiele für geeignete pharmazeutische Wirkstoffe sind die insbesondere die in den Absätzen 0105 bis 0131 der US 2003/0157170 genannten Wirkstoffe.

Neben der Anwendung in der Kosmetik und in der Pharmazie eignen sich die erfindungsgemäß zu verwendenden Copolymerzusammensetzungen CP) auch im Lebensmittelbereich zur Modifizierung der rheologischen Eigenschaften. Gegenstand der Erfindung sind daher auch lebensmitteltechnische Zubereitungen, die mindestens eine der erfindungsgemäß zu verwendenden Copolymerzusammensetzungen CP) enthalten. Zu den Lebensmittelzubereitungen sind im Rahmen der vorliegenden Erfindung auch Nahrungsergänzungsmittel wie z. B. Lebensmittelfarbstoffe enthaltende Zubereitungen und diätetische Lebensmittel zu verstehen. Darüber hinaus eigenen sich die genannten Copolymerzusammensetzungen CP) auch zur Modifizierung der rheologischen Eigenschaften für Futtermittelzusätze für die Tierernährung.

Außerdem eignen sich die Copolymerzusammensetzungen CP) zur Herstellung wässriger Zubereitungen von Nahrungsergänzungsmitteln wie wasserunlöslichen Vitaminen und Provitaminen wie Vitamin A, Vitamin A-Acetat, Vitamin D, Vitamin E, Tocopherol-Derivate wie Tocopherolacetat und Vitamin K.

Ein weiterer Gegenstand der Erfindung ist die Verwendung einer Copolymerzusammensetzung CP), wie zuvor definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

Die Erfindung wird anhand der folgenden, nicht einschränkenden Beispiele näher erläutert.

### Beispiele

### I. Herstellung der Hilfsstoffzusammensetzung H1)

### I.1 Allgemeine Vorschrift zur Herstellung der Urethanverbindung H1

In einem 4-Hals-Kolben, der mit Rührer, Tropftrichter, Thermometer, Rückflusskühler und einer Vorrichtung für das Arbeiten unter Stickstoff ausgestattet war, wurden langkettiges Diol, Neopentylglykol, N-Methyldiethanolamin und Polyethylenglykolmonomethylether, d. h. die Komponenten a), b), c) und d) in den in Tabelle 2 angegebenen Mengen und 0,2 Gew.-% (bezogen auf alle Komponenten einschließlich des Lösungsmittels Essigsäureethylester) 1,4-Diazabicyclo[2.2.2]octan (DABCO) in Essigsäureethylester (Feststoffgehalt des Reaktionsgemischs etwa 70 %; für ca. 400 g Produkt etwa 100 g Essigsäureethylester) vorgelegt und unter Erwärmen auf eine Temperatur von 60 bis 65 °C und unter Rühren gelöst. Anschließend dosierte man innerhalb 30 Minuten Hexamethylendiisocyanat (Komponente e1) in der in Tabelle 2 angegebenen Mengen so zu, dass die Reaktionstemperatur unterhalb 80 °C blieb. Danach wurde das Reaktionsgemisch 30 Minuten bei 75 bis 80 °C nachgerührt, bis der NCO-Gehalt des Gemischs praktisch konstant blieb. Anschließend gab man 0,2 Gew.- %, bezogen auf alle Komponenten einschließlich des Lösungsmittels Essigsäureethylester, DABCO zu und dosierte Isophorondiisocyanat (Komponente e2) in der in Tabelle 2 angegebenen Menge bei einer Innentemperatur unterhalb 85 °C innerhalb von 30 Minauten zu. Man rührte das Reaktionsgemisch 4 bis 8 Stunden bei 82 ± 3 °C nach, bis der NCO-Gehalt konstant blieb. Danach ließ man das Reaktionsgemisch abkühlen und verdünnte mit Essigsäureethylester auf einen Feststoffgehalt von 50 %. Anschließend gab man die Komponente f) so zu, dass die Reaktionstemperatur unterhalb 40 °C blieb. Man rührte 1 Stunde bei 40 bis 50 °C nach. Man verdünnte das Reaktionsprodukt erneut mit Essigsäureethylester.

**Tabelle 2:**

| Kompo nente | a) langkettiges Diol | b) kurzkettiges Diol | c) NME-DA | d) MPEG-OH | e1): HDI | e2): IPDI | f) Kettenverlängerer bzw. Abstopper |
|---|---|---|---|---|---|---|---|
| H1-1 | PTHF 2 mol | NPG 1,5 mol | 0,5 mol | Pluriol A500E 1,5 mol | 2 mol | 3 mol | A Si 2322 0,054 mol |
| H1-2 | PTHF 2 mol | NPG 1,5 mol | 0,5 mol | Pluriol A500E 1,5 mol | 2 mol | 3 mol | Isophorondiamin 0,054 mol |
| H1-3 | VP 9186 1 mol | NPG 1 mol | -- | Pluriol A500E 1,5 mol | 1,5 mol | 1,5 mol | 1,5-Pentandiamin 0,054 mol |
| H1-4 | VP 9186 1 mol | NPG 1 mol | -- | Pluriol A500E 1,5 mol | 1,5 mol | 1,5 mol | Isophorondiamin 0,054 mol |
| H1-5 | PTHF 2 mol | NPG 2 mol | -- | Pluriol A500E 1,5 mol | 1 mol | 4 mol | Hexamethylendiamin 0,25 mol |
| H1-6 | PTHF (0,8 mol) + VP 9186 (0,8 mol) | NPG 0,4 mol | 0,25 mol | Pluriol A500E 1,5 mol | 1,5 mol | 1,5 mol | 1,5 mol -- |
| H1-7 | T 22/98/99 1,6 mol | NPG 0,4 mol | 0,25 mol | Pluriol A1000E 1,5 mol | 1,5 mol | 1,5 mol | 1,5 mol -- |
| H1-8 | T 22/98/99 1 mol | -- | -- | Pluriol A1000E 1,5 mol | -- | 2,0 mol | Isophoron-diamin 0,25 mol |
| H1-9 | -- | -- | -- | Pluriol A1000E (0,5 mol) + Pluriol A2000E (0,5 mol) | -- | 1,05 mol | Kerocom PIBA 1 mol |
| H1-10 | PTHF 2 mol | NPG 1,7 mol | 0,3 mol | Pluriol A1000E 1,5 mol | 2,5 mol | 2,5 mol | Isophoron-diamin 0,25 mol |
| H1-11 | PTHF 2 mol | NPG 1,7 mol | 0,3 mol | Pluriol A1000E 1,5 mol | 2,5 mol | 2,5 mol | Kerocom PIBA 1 mol |
| H1-12 | PTHF 2 mol | NPG 2,0 mol | -- | Pluriol A1000E 1,5 mol | 2,5 mol | 2,5 mol | Isophoron-diamin 0,25 mol |
| H1-13 | PTHF 2 mol | NPG 2,0 mol | -- | Pluriol A1000E 1,5 mol | 2,5 mol | 2,5 mol | 3-Amino-propylimidazol |

- PTHF: Polytetrahydrofuran, Mₙ etwa 1000 g/mol
- VP 9186: Lupraphen® VP 9186 der BASF SE (Polyesterdiol aus Adipinsäure und 1,4-Butandiol), Mₙ etwa 2000 g/mol
- T 22/98/99: Polyesterdiol aus Isophthalsäure, 1,6-Hexandiol und Neopentylglykol, Mₙ etwa 2000 g/mol
- NPG: Neopentylglykol
- NMEDA: N-Methyldiethanolamin
- MPEG-OH: Polyethylenglykolmonomethylether
- Pluriol® A500E: Polyethylenglykolmonomethylether, Mₙ 500 g/mol, BASF SE
- Pluriol® A1000E: Polyethylenglykolmonomethylether, Mₙ etwa 1000 g/mol, BASF SE
- Pluriol® A2000E: Polyethylenglykolmonomethylether, Mₙ etwa 2000 g/mol, BASF SE
- HDI: Hexamethylendiisocyanat
- IPDI: Isophorondiisocyanat
- A Si 2322: Tegomer® A Si 2322, Goldschmidt, α,ω-amino-funktionelles Polysiloxan
- Kerocom® PIBA: Polyisobutenamin, Mₙ etwa 1000 g/mol, BASF SE

### 1.2 Polyisobutenylalkoholalkoxilate und Polyisobutenylaminalkoxilate H1

### Allgemeine Methode A:

Umsetzung von Polyisobutenylbemsteinsäureanhydrid mit Methylpolyethylenglykol bzw. Polyethylenglykol zum entsprechenden Halbester

In einem 4 I-Dreihalskolben mit Stickstoffzuleitung wurden 1 mol PIBSA₁₀₀₀ und Polyether in der in Tabelle 3 angegebenen Menge sowie Essigsäureethylester (etwa 20 Gew.-%, bezogen auf das Gesamtgemisch) eingewogen und 1 Stunde auf 50 °C erwärmt. Anschließend erwärmte man auf 70 bis 80 °C. Während der Reaktion destillierten teilweise flüchtige Bestandteile über. Zur Vollständigkeit wurde gegen Ende der Reaktion bei einer Temperatur von 80 bis 100 °C der Druck auf 20 hPa gesenkt. Danach kühlt man auf Raumtemperatur ab.

### Allgemeine Methode B:

Umsetzung von Polyisobutenylbemsteinsäureanhydrid mit Alkanolamin

In einem Vierhalskolben wurde 1 mol PIBSA₁₀₀₀ in Essigsäureethylester (30 Gew.-%, bezogen auf alle Komponenten) eingewogen und danach wurde bei 30 °C das Alkanolamin gemäß Tabelle 3 zugetropft. Man erwärmte auf 40 bis 50 °C und hielt das Reaktionsgemisch 1 h bei dieser Temperatur. Anschließend erwärmte man auf 70 bis 80 °C. Während der Reaktion destillierten teilweise flüchtige Bestandteile über. Zur Vollständigkeit wurde gegen Ende der Reaktion bei einer Temperatur von 80 bis 100 °C der Druck auf 20 hPa gesenkt. Die gesamte Destillationszeit betrug ca. 1 h. Danach kühlte man auf Raumtemperatur ab und gab Essigsäureethylester zu, so dass der Feststoffgehalt des Reaktionsgemischs 40 % betrug.

### Allgemeine Methode C:

Umsetzung von Urethanprepolymeren mit Polyisobutylenamin

In einem 4-Hals-Kolben, der mit Rührer, Tropftrichter, Thermometer, Rückflusskühler und einer Vorrichtung für das Arbeiten unter Stickstoff ausgestattet war, wurden der Polyether und gegebenenfalls Neopentylglykol in den in Tabelle 3 angegebenen Mengen und 0,2 Gew.-% (bezogen auf alle Komponenten einschließlich des Lösungsmittels Essigsäureethylester) DABCO in Essigsäureethylester (Feststoffgehalt des Reaktionsgemischs etwa 70 %) eingewogen. Anschließend dosierte man innerhalb 30 Minuten 1 mol Isophorondiisocyant so zu, dass die Reaktionstemperatur unterhalb 80 °C blieb. Danach wurde das Reaktionsgemisch 2 bis 4 Stunden bei 75 bis 80 °C nachgerührt, bis der NCO-Gehalt des Gemischs praktisch konstant blieb. Man verdünnte das Reaktionsgemisch mit Essigsäureethylester auf einen Feststoffgehalt von 30 bis 50 %. Anschließend gab man 1 mol Polyisobutylenamin so zu, dass die Reaktionstemperatur im Bereich von 40 bis 80 °C lag. Zur Vervollständigung der Umsetzung rührte man 1 Stunde bei 40 bis 80 °C nach.

**Tabelle 3**

| | PIBSA₁₀₀₀ | PIB₁₀₀₀-NH₂ | Polyether | Alkanolamin | NPG | IPDI | Metho de |
|---|---|---|---|---|---|---|---|
| H1-14 | 1 mol | -- | MPEG₁₀₀₀ 1 mol | | -- | -- | A |
| H1-15 | 1 mol | -- | MPEG₅₀₀ 1 mol | | -- | -- | A |
| H1-16 | 1 mol | -- | MPEG₃₅₀ 1 mol | | -- | -- | A |
| H1-17 | 1 mol | -- | PEG₂₀₀ 2 mol | | -- | -- | A |
| H1-18 | 1 mol | -- | -- | AE-Gly 1 mol | -- | -- | B |
| H1-19 | 1 mol | -- | -- | DEA 1 mol | -- | -- | B |
| H1-20 | -- | 1 mol | MPEG₁₀₀₀ 1 mol | | -- | 1 mol | C |
| H1-21 | -- | 1 mol | MPEG₅₀₀ 1 mol | | -- | 1 mol | C |
| H1-22 | -- | 1mol | MPEG 350 1 mol | | -- | 1 mol | C |
| H1-23 | -- | 1 mol | PEG₂₀₀ 2 mol | | -- | 1 mol | C* |
| H1-24 | -- | 1 mol | MPEG₅₀₀ 1 mol | | 1 mol | 2 mol | C |

| | | | | | | | |
|---|---|---|---|---|---|---|---|
| * Umsetzung wird variiert: Zuerst Umsetzung aus PIB-Amin mit IPDI, anschließend mit PEG₂₀₀ | | | | | | | |

- PIBSA₁₀₀₀: Polyisobutenylbemsteinsäureanhydrid, Mₙ etwa 1000 g/mol
- PIB₁₀₀₀-NH₂: Polyisobutylenamin, Mₙ etwa 1000 g/mol
- MPEG_{300, 500, 1000}: Methylpolyethylenglykol, Mₙ 300, 500 bzw. 1000 g/mol
- PEG₂₀₀: Polyethylenglykol, Mₙ 200 g/mol
- AE-GLy: 2-(2-Aminoethoxy)ethanol
- DEA: Diethanolamin
- NPG: Neopentylglykol
- IPDI: Isophorondiisocyanat

### II Synthese von acrylsäurehaltigen Polymeren

### Allgemeines Verfahren

### (Beispiel 6 in Tabelle 4)

| | | |
|---|---|---|
| Vorlage | Essigsäureethylester | 204,55 g |
| | Cyclohexan | 180,85 g |
| | H1-26 | 11,92 g |
| | | |
| Zulauf 1 | Essigsäureethylester | 109,80 g |
| | Cyclohexan | 97,38 g |
| | Acrylsäure | 592,00 g |
| | Pentaerythrittriallylether, 70% | 4,24 g |
| | NH₄CO₃ | 17,76 g |
| | | |
| Zulauf 2 | Essigsäureethylester | 170,91 g |
| | Cyclohexan | 151,55 g |
| | Wako® V65 | 0,12 g |
| | tert.-Butylperoctoat, 98%ig | 1,08 g |
| | | |
| Zulauf 3 | Essigsäureethylester | 446,66 g |
| | Cyclohexan | 396,10 g |

| | | |
|---|---|---|
| Wako® V65 Azobis(2,4-dimethylvaleronitril), erhältlich von Wako Chemicals | | |

### Durchführung der Reaktion:

In einer Druckapparatur mit Rückflusskühler, Innenthermometer und drei separaten Zulaufvorrichtungen wurde die Vorlage eingewogen, dreimal mit 2 bar Stickstoff abgepresst und unter Rühren auf ca. 70 °C erwärmt. Man dosierte Zulauf 1 und Zulauf 2 bei 70 °C zu, wobei Zulauf 1 innerhalb 3 Stunden und Zulauf 2 innerhalb 5 Stunden zudosiert wurden. 1 Stunde nach dem Start von Zulauf 1 dosierte man Zulauf 3 innerhalb 3 Stunden zu. Nach beendeter Zugabe des Zulaufs 2 erwärmte man das Reaktionsgemisch auf 75 °C und ließ 9 Stunden nachpolymerisieren. Man filtrierte das erhaltene Produkt und trocknete es in einem Trockenschrank.

In analoger Weise wurden die Copolymerzusammensetzungen der Beispiele 1 bis 5, 10, 11 und 12 in Tabelle 4 hergestellt. In analoger Weise wurde die Copolymerzusammensetzungen der Beispiele 7, 8 und 9 hergestellt, wobei in Beispiel 7 Zulauf 1 zusätzlich die in Tabelle 4 angegebene Menge an Vinylpyrrolidon, in Beispiel 8 zusätzlich die in Tabelle 4 angegebene Menge an Methacrylamid und in Beispiel 9 zusätzlich die in Tabelle 4 angegebenen Mengen an Vinylpyrrolidon und Vinylimidazol enthielt.

Die nach dem obigen Verfahren erhaltenen Copolymerzusammensetzungen sind in Tabelle 4 aufgelistet. Die Mengenangaben in Tabelle 4 sind (soweit nicht abweichend angegeben) in Gew.-%, bezogen auf die zur Polymerisation eingesetzten ungesättigten Monomeren. Aufgelistet sind ebenfalls Vergleichszusammensetzungen VB1, VB2 und VB3, die in analoger Weise hergestellt wurden.

**Tabelle 4**

| Beispiel | AS | VP | MAM | PETAE | VI | H1)** | H2)*** NH₄HCO₃ [%] |
|---|---|---|---|---|---|---|---|
| VB1 | 99,5 | -- | -- | 0,5 | -- | -- | -- |
| VB2 | 99,5 | -- | -- | 0,5 | -- | -- | 3 % |
| VB3 | 99,5 | -- | -- | 0,5 | -- | H1-15 2 % | -- |
| 1 | 99,5 | -- | -- | 0,5 | -- | H1-15 2% | 1 % |
| 2 | 99,5 | -- | -- | 0,5 | -- | H1-15 2 % | 2 % |
| 3 | 99,5 | -- | -- | 0,5 | -- | H1-15 2% | 3 % |
| 4 | 99,5 | -- | -- | 0,5 | -- | H1-15 2 % | 6 % |
| 5 | 99,5 | -- | -- | 0,5 | -- | H1-25 2 % | 3 % |
| 6 | 99,5 | -- | -- | 0,5 | -- | H1-26 2% | 3 % |
| 7 | 84,5 | 15 | -- | 0,5 | -- | H1-26 2 % | 3 % |
| 8 | 84,5 | -- | 15 | 0,5 | -- | H1-26 2 % | 3 % |
| 9 | 84,5 | 13 | -- | 0,5 | 2 | H1-26 2% | 3 % |
| 10 | 99,5 | -- | -- | 0,5 | -- | H1-11 2 % | 2 % |
| 11 | 99,5 | -- | -- | 0,5 | -- | H1-18 2 % | 2 % |
| 12 | 99,5 | -- | -- | 0,5 | -- | H1-21 2% | 2 % |

- AS:: Acrylsäure
- VP:: Vinylpyrrolidon
- VI:: Vinylimidazol
- MAM:: Methacrylamid
- PETAE: Pentaerythrittriallylether
- H1-15: PIBSA₁₀₀₀/ Pluriol A500E
- H1-25 :: Belsil DMC 6031 (Wacker)
- H1-26:: ABIL Soft AF 100 (Evonik)
- **): %-Angabe: bezogen auf 100 % Monomere (inklusiv. Vernetzer)
- ***): %-Angabe: Einsatz von NH₄HCO₃ bezogen auf Acrylsäure

In Tabelle 5 sind Angaben zu Produkteigenschaften der erfindungsgemäßen Copolymerzusammensetzungen sowie der Vergleichscopolymerzusammensetzungen VB1, VB2 und VB3 aufgelistet. Zugrunde gelegt wurde eine Skala von 1 bis 5.

**Tabelle 5:**

| Beispiel | redispergierte Geschwindigkeit*) Note | Stabilität / Bemerkung *) |
|---|---|---|
| VB1 | 5 | 0 / unlösliche Masse |
| VB2 | 4 | +++ / hohe Viskosität. |
| VB3 | 2 | 0 / sofort abgesetzt |
| 1 | 2+ | +/-; nach ca. 20 min abgesetzt |
| 2 | 2 | ++; gerade stabil, leicht abgesetzt nach 24 h |
| 3 | 2- | +++; stabil nach 24 h, Viskosität niedrig |
| 4 | 3 | +++; hohe Viskosität, stabil nach 24 h |
| 5 | 3-4 | +++; stabil nach 24 h, Viskosität niedrig |
| 6 | 3 | +++; stabil nach 24 h, Viskosität niedrig |
| 7 | 3 | +++; stabil nach 24 h, Viskosität niedrig |
| 8 | 3 | +++; stabil nach 24 h, Viskosität niedrig |
| 9 | 3 | +++; Stabil nach 24 h, Viskosität niedrig |

| | | |
|---|---|---|
| *) Herstellung einer 1%igen wässrigen Polymerstammdispersion | | |

| Bewertung redispergierter Geschwindigkeit | Bewertung der Stabilität / Bemerkung: |
|---|---|
| Note 1: 0 - 5 min | 0 = nicht stabil, viel unlöslicher Anteil |
| Note 2: 5 - 20 min | +/- stabil, allerdings unmittelbar nach dem Ansetzen |
| Note 3: 20 - 60 min | + stabil ca. 1 h |
| Note 4: > 60 min | ++ stabil ca. 24 h |
| Note 5: unlöslich | +++ stabil länger als 24 h |

### II. Anwendungstechnische Beispiele

### Anwendungsbeispiel 1: Haargel mit PVP K90

### Phase 1:

| | |
|---|---|
| Polymer aus Beispiel Nr. 1 (1 %ige wässrige Dispersion) Additive (Parfüm, UV-Absorber, Silikon). | 50,00 g |

### Phase 2:

| | |
|---|---|
| Luviskol® K90-F (BASF SE) | 3 g |
| Wasser | 47 g |

### Herstellung:

Die Phasen 1 und 2 wurden getrennt eingewogen und homogenisiert. Dann wurde Phase 2 langsam in Phase 1 eingerührt. Man stellte die Mischung mit Triethanolamin auf pH 7. Es bildete sich ein klares, stabiles Gel.

Das Beispiel wurde mit den Copolymerisaten der Beispiele 2, 3, 4, 5, 6, 7, 8, 9, 10, 11 bzw. 12 wiederholt. Es bildete sich ein klares, stabiles Gel.

## Patentansprüche

1. Verfahren zur Herstellung einer Copolymerzusammensetzung CP) durch radikalische Copolymerisation einer Monomerzusammensetzung, enthaltend
a) 70 bis 100 Gew.-%, bezogen auf das Gesamtgewicht der zur Polymerisation eingesetzten Monomere, Acrylsäure,
b) 0 bis 30 Gew.-%, bezogen auf das Gesamtgewicht der zur Polymerisation eingesetzten Monomere, wenigstens einer von a) verschiedenen hydrophilen nichtionischen Verbindung mit einer radikalisch polymerisierbaren, α,β-ethylenisch ungesättigten Doppelbindung,
c) 0 bis 1 Gew.-%, bezogen auf das Gesamtgewicht der zur Polymerisation eingesetzten Monomere, wenigstens einer radikalisch polymerisierbaren vernetzenden Verbindung, die wenigstens zwei α,β-ethylenisch ungesättigte Doppelbindungen pro Molekül enthält,
nach der Methode der Fällungspolymerisation in Gegenwart einer Hilfsstoffzusammensetzung H), enthaltend
H1) wenigstens eine Verbindung mit Blockstruktur, die wenigstens eine hydrophobe Gruppe und wenigstens eine hydrophile Gruppe umfasst, wobei die Verbindung mit Blockstruktur H1) ausgewählt ist unter
- Polyisobutenylalkoholalkoxilaten,
- Polyisobutenylaminalkoxilaten,
- Umsetzungsprodukten aus wenigstens einem Polyisobuten mit mindestens einer Carbonsäureendgruppe oder einem Derivat davon und wenigstens einem Polyalkylenoxid mit einer terminalen, gegenüber Anhydridgruppen reaktiven Gruppe,
- Siliconverbindungen, die wenigstens eine Polyethergruppe aufweisen,
- Umsetzungsprodukten aus wenigstens einer Verbindung, die mindestens eine hydrophobe Gruppe und mindestens eine gegenüber Isocyanatgruppen reaktive Gruppe umfasst, wenigstens einer Verbindung, die mindestens eine hydrophile Gruppe und mindestens eine gegenüber Isocyanatgruppen reaktive Gruppe umfasst, und wenigstens einem Polyisocyanat,
und
H2) wenigstens eine von H1) verschiedene basische Verbindung, wobei die Komponente H2) ausgewählt ist unter NH₃, (NH₄)₂CO₃, NH₄HCO₃, Monoalkylaminen, Dialkylaminen, Trialkylaminen, Aminoalkoholen, stickstoffhaltigen Heterocyclen und Mischungen davon.

2. Verfahren nach Anspruch 1, wobei die zur Herstellung der Copolymerzusammensetzung CP) eingesetzte Monomerzusammensetzung
a) 70 bis 99,99 Gew.-%, bezogen auf das Gesamtgewicht der zur Polymerisation eingesetzten Monomere, Acrylsäure,
b) 0 bis 29,99 Gew.-%, bezogen auf das Gesamtgewicht der zur Polymerisation eingesetzten Monomere, wenigstens einer von a) verschiedenen hydrophilen nichtionischen Verbindung mit einer radikalisch polymerisierbaren, α,β-ethylenisch ungesättigten Doppelbindung,
c) 0,01 bis 1 Gew.-%, bezogen auf das Gesamtgewicht der zur Polymerisation eingesetzten Monomere, wenigstens einer radikalisch polymerisierbaren vernetzenden Verbindung, die wenigstens zwei α,β-ethylenisch ungesättigte Doppelbindungen pro Molekül enthält,
enthält.

3. Verfahren nach Anspruch 1, wobei die Herstellung der Verbindungen H1) mit Polyisobutenylgruppen ausgeht von Polyisobutenen, die ausgewählt sind unter:
- hochreaktiven Polyisobutenen,
- Polyisobutenen mit mindestens einer stickstoffhaltigen Endgruppe,
- Polyisobutenylalkoholen,
- Polyisobutenylaldehyden,
- Polyisobutenen mit mindestens einer Carbonsäureendgruppe oder einem Derivat davon,
- Mischungen aus zwei oder mehr als zwei der zuvor genannten Verbindungen.

4. Verfahren nach einem der vorhergehenden Ansprüche, wobei der Hilfsstoff H1) ein Umsetzungsprodukt von PIBSA und einem Polyethylenoxidmonomethylether umfasst oder aus einem Umsetzungsprodukt von PIBSA und einem Polyethylenoxidmonomethylether besteht.

5. Verfahren nach einem der vorhergehenden Ansprüche, wobei die Verbindung mit Blockstruktur H1) ausgewählt ist unter Urethanverbindungen, die
p1) wenigstens eine Verbindung, die mindestens eine hydrophobe Gruppe und mindestens eine gegenüber Isocyanatgruppen reaktive Gruppe umfasst,
p2) wenigstens eine Verbindung, die mindestens eine hydrophile Gruppe und mindestens eine gegenüber Isocyanatgruppen reaktive Gruppe umfasst, und
p3) wenigstens ein Polyisocyanat,
eingebaut enthalten.

6. Verfahren nach Anspruch 5, wobei die Urethanverbindungen H1) zusätzlich wenigstens eine Verbindung p4) eingebaut enthalten, die ausgewählt ist unter Verbindungen mit einem Molekulargewicht im Bereich von 56 bis 280 g/mol, die zwei gegenüber Isocyanatgruppen reaktive Gruppen pro Molekül enthalten, vorzugsweise ausgewählt unter Diolen, Diaminen, Aminoalkoholen und Mischungen davon.

7. Verfahren nach einem der Ansprüche 5 oder 6, wobei die Urethanverbindungen H1) zusätzlich wenigstens eine Verbindung p5) eingebaut enthalten, die ausgewählt ist unter Verbindungen mit einem Molekulargewicht im Bereich von 56 bis 280 g/mol, die nur eine gegenüber Isocyanatgruppen reaktive Gruppe pro Molekül enthalten.

8. Verfahren nach einem der vorhergehenden Ansprüche, wobei die Komponente H2) Ammoniumhydrogencarbonat umfasst oder aus Ammoniumhydrogencarbonat besteht.

9. Verfahren nach einem der vorhergehenden Ansprüche, wobei das zur Herstellung der Copolymerzusammensetzung CP) eingesetzte Reaktionsgemisch im gesamten Verlauf der Copolymerisation einen Wassergehalt von höchstens 5 Gew.-%. bevorzugt höchstens 3 Gew.-%, insbesondere höchstens 2 Gew.-%, aufweist.

10. Verfahren nach einem der vorhergehenden Ansprüche, wobei die zur Herstellung der Copolymerzusammensetzung CP) eingesetzte Monomerzusammensetzung zusätzlich wenigstens eine Verbindung d) mit einer radikalisch polymerisierbaren, α,β-ethylenisch ungesättigten Doppelbindung und wenigstens einer kationogenen und/oder kationischen Gruppe pro Molekül enthält.

11. Verfahren nach einem der vorhergehenden Ansprüche, wobei die zur Herstellung der Copolymerzusammensetzung CP) eingesetzte Monomerzusammensetzung zusätzlich wenigstens eine von Acrylsäure verschiedene Verbindung e) mit einer radikalisch polymerisierbaren, α,β-ethylenisch ungesättigten Doppelbindung und wenigstens einer anionogenen und/oder anionischen Gruppe pro Molekül enthält.

12. Verfahren nach einem der vorhergehenden Ansprüche, wobei die zur Herstellung der Copolymerzusammensetzung CP) eingesetzte Monomerzusammensetzung zusätzlich wenigstens eine Verbindung f) enthält, die ausgewählt ist unter Estern α,β-ethylenisch ungesättigter Mono- und Dicarbonsäuren mit C₁-C₃₀-Alkanolen, Amiden α,β-ethylenisch ungesättigter Mono- und Dicarbonsäuren mit Mono- und Di-(C₁-C₃₀-Alkyl)aminen, N,N-Diallylaminen, deren Säureadditionssalzen und Quaternisierungsprodukten, N,N-Diallyl-N-alkylaminen, deren Säureadditionssalzen und Quaternisierungsprodukten, Urethan(meth)acrylaten mit Alkylenoxidgruppen, Estern von Vinylalkohol und Allylalkohol mit C₁-C₃₀-Monocarbonsäuren, C₁-C₃₀-Alkylvinylethern, Vinylaromaten, Vinylhalogeniden, Vinylidenhalogeniden, C₂-C₈-Monoolefinen, nicht aromatischen Kohlenwasserstoffen mit mindestens zwei konjugierten Doppelbindungen und Mischungen davon.

13. Copolymerzusammensetzung CP), erhältlich durch ein Verfahren, wie in einem der Ansprüche 1 bis 12 definiert.

14. Kosmetisches Mittel, enthaltend
A) wenigstens eine Copolymerzusammensetzung CP), erhältlich durch ein Verfahren, wie in einem der Ansprüche 1 bis 12 definiert,
B) wenigstens einen kosmetisch akzeptablen Wirkstoff und
C) gegebenenfalls wenigstens einen weiteren, von CP) und B) verschiedenen kosmetisch akzeptablen Hilfsstoff.

15. Pharmazeutisches Mittel, enthaltend
A) wenigstens eine Copolymerzusammensetzung, erhältlich durch ein Verfahren, wie in einem der Ansprüche 1 bis 12 definiert,
B) wenigstens einen pharmazeutisch akzeptablen Wirkstoff und
C) gegebenenfalls wenigstens einen weiteren, von A) und B) verschiedenen pharmazeutisch akzeptablen Hilfsstoff.

16. Verwendung einer Copolymerzusammensetzungen CP), erhältlich durch ein Verfahren, wie in einem der Ansprüche 1 bis 12 definiert, im Lebensmittelbereich zur Modifizierung der rheologischen Eigenschaften, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

## Claims

1. A process for the preparation of a copolymer composition CP) by free-radical copolymerization of a monomer composition comprising
a) 70 to 100% by weight, based on the total weight of the monomers used for the polymerization, of acrylic acid,
b) 0 to 30% by weight, based on the total weight of the monomers used for the polymerization, of at least one hydrophilic nonionic compound, different from a), having a free-radically polymerizable, α,β-ethylenically unsaturated double bond,
c) 0 to 1% by weight, based on the total weight of the monomers used for the polymerization, of at least one free-radically polymerizable crosslinking compound which comprises at least two α,β-ethylenically unsaturated double bonds per molecule,
by the method of precipitation polymerization in the presence of an auxiliary composition H) comprising
H1) at least one compound with a block structure which comprises at least one hydrophobic group and at least one hydrophilic group, where the compound with a block structure H1) is selected from
- polyisobutenyl alcohol alkoxylates,
- polyisobutenyl amine alkoxylates,
- reaction products of at least one polyisobutene with at least one carboxylic acid end group or a derivative thereof and at least one polyalkylene oxide with a terminal group reactive towards anhydride groups,
- silicone compounds which have at least one polyether group,
- reaction products of at least one compound which comprises at least one hydrophobic group and at least one group reactive towards isocyanate groups, at least one compound which comprises at least one hydrophilic group and at least one group reactive towards isocyanate groups, and at least one polyisocyanate,
and
H2) at least one basic compound different from H1), where the component H2) is selected from NH₃. (NH4)₂CO₃. NH₄HCO₃. monoalkylamines, dialkylamines, trialkylamines, amino alcohols, nitrogen-containing heterocycles and mixtures thereof.

2. The process according to claim 1, where the monomer composition used for the preparation of the copolymer composition CP) comprises
a) 70 to 99.99% by weight, based on the total weight of the monomers used for the polymerization, of acrylic acid,
b) 0 to 29.99% by weight, based on the total weight of the monomers used for the polymerization, of at least one hydrophilic nonionic compound, different from a), having a free-radically polymerizable, α,β-ethylenically unsaturated double bond,
c) 0.01 to 1% by weight, based on the total weight of the monomers used for the polymerization, of at least one free-radically polymerizable crosslinking compound which comprises at least two α,β-ethylenically unsaturated double bonds per molecule.

3. The process according to claim 1, where the preparation of the compounds H1) having polyisobutenyl groups starts from polyisobutenes which are selected from:
- high-reactivity polyisobutenes,
- polyisobutenes with at least one nitrogen-containing end group,
- polyisobutenyl alcohols,
- polyisobutenyl aldehydes,
- polyisobutenes having at least one carboxylic acid end group or a derivative thereof,
- mixtures of two or more than two of the aforementioned compounds.

4. The process according to any one of the preceding claims, where the auxiliary H1) comprises a reaction product of PIBSA and a polyethylene oxide monomethyl ether or consists of a reaction product of PIBSA and a polyethylene oxide monomethyl ether.

5. The process according to any one of the preceding claims, where the compound with a block structure H1) is selected from urethane compounds which comprise, in incorporated form,
p1) at least one compound which comprises at least one hydrophobic group and at least one group reactive towards isocyanate groups,
p2) at least one compound which comprises at least one hydrophilic group and at least one group reactive towards isocyanate groups, and
p3) at least one polyisocyanate.

6. The process according to claim 5, where the urethane compounds H1) additionally comprise at least one compound p4) in incorporated form which is selected from compounds with a molecular weight in the range from 56 to 280 g/mol which comprise two groups reactive towards isocyanate groups per molecule, preferably selected from diols, diamines, amino alcohols and mixtures thereof.

7. The process according to any one of claims 5 or 6, where the urethane compounds H1) additionally comprise at least one compound p5) in incorporated form which is selected from compounds with a molecular weight in the range from 56 to 280 g/mol which comprise only one group reactive towards isocyanate groups per molecule.

8. The process according to any one of the preceding claims, where the component H2) comprises ammonium hydrogencarbonate or consists of ammonium hydrogencarbonate.

9. The process according to any one of the preceding claims, where the reaction mixture used for the preparation of the copolymer composition CP) throughout the entire course of the copolymerization has a water content of at most 5% by weight, preferably at most 3% by weight, in particular at most 2% by weight.

10. The process according to any one of the preceding claims, where the monomer composition used for the preparation of the copolymer composition CP) additionally comprises at least one compound d) having a free-radically polymerizable, α,β-ethylenically unsaturated double bond and at least one cationogenic and/or cationic group per molecule.

11. The process according to any one of the preceding claims, where the monomer composition used for the preparation of the copolymer composition CP) additionally comprises at least one compound e), different from acrylic acid, having a free-radically polymerizable, α,β-ethylenically unsaturated double bond and at least one anionogenic and/or anionic group per molecule.

12. The process according to any one of the preceding claims, where the monomer composition used for the preparation of the copolymer composition CP) additionally comprises at least one compound f) which is selected from esters of α,β-ethylenically unsaturated mono- and dicarboxylic acids with C1-C₃₀-alkanols, amides of α,β-ethylenically unsaturated mono- and dicarboxylic acids with mono- and di- (C₁-C₃₀-alkyl) amines, N,N-diallylamines, their acid addition salts and quaternization products, N,N-diallyl-N-alkylamines, their acid addition salts and quaternization products, urethane (meth)acrylates with alkylene oxide groups, esters of vinyl alcohol and allyl alcohol with C₁-C₃₀-monocarboxylic acids, C₁-C₃₀-alkyl vinyl ethers, vinyl aromatics, vinyl halides, vinylidene halides, C₂-C₈-monoolefins, nonaromatic hydrocarbons with at least two conjugated double bonds and mixtures thereof.

13. A copolymer composition CP) obtainable by a process as defined in any one of claims 1 to 12.

14. A cosmetic composition comprising
A) at least one copolymer composition CP) obtainable by a process as defined in any one of claims 1 to 12,
B) at least one cosmetically acceptable active ingredient and
C) optionally at least one further cosmetically acceptable auxiliary different from CP) and B).

15. A pharmaceutical composition comprising
A) at least one copolymer composition obtainable by a process as defined in any one of claims 1 to 12,
B) at least one pharmaceutically acceptable active ingredient and
C) optionally at least one further pharmaceutically acceptable auxiliary different from A) and B).

16. The use of a copolymer composition CP) obtainable by a process as defined in any one of claims 1 to 12 in the food sector for modifying rheological properties, as auxiliary in pharmacy, preferably as or in (a) coating composition(s) for solid medicament forms, for modifying rheological properties, as surface-active compound, as or in (an) adhesive(s) and as or in (a) coating composition(s) for the textile, paper, printing and leather industry.

## Revendications

1. Procédé pour la préparation d'une composition de copolymères CP) par copolymérisation radicalaire d'une composition de monomères, contenant
a) 70 à 100% en poids, par rapport au poids total des monomères utilisés pour la polymérisation, d'acide acrylique,
b) 0 à 30% en poids, par rapport au poids total des monomères utilisés pour la polymérisation, d'au moins un composé non ionique hydrophile différent de a) présentant une double liaison polymérisable par voie radicalaire α,β-éthyléniquement insaturée,
c) 0 à 1% en poids, par rapport au poids total des monomères utilisés pour la polymérisation, d'au moins un composé polymérisable par voie radicalaire, réticulant, qui contient au moins deux doubles liaisons α,β-éthyléniquement insaturées par molécule,
selon le procédé de la polymérisation par précipitation en présence d'une composition adjuvante H), contenant
H1) au moins un composé présentant une structure à blocs qui comprend au moins un groupe hydrophobe et au moins un groupe hydrophile, le composé présentant une structure à blocs H1) étant choisi parmi
- les alcoxylates de poly(alcool isobuténylique),
- les alcoxylates de poly(isobuténylamine),
- les produits de transformation d'au moins un polyisobutène présentant au moins un groupe terminal acide carboxylique ou un dérivé de celui-ci et d'au moins un poly(oxyde d'alkylène) présentant un groupe terminal réactif par rapport aux groupes anhydride,
- les composés siliconés qui présentent au moins un groupe polyéther,
- les produits de transformation d'au moins un composé qui comprend au moins un groupe hydrophobe et au moins un groupe réactif par rapport aux groupes isocyanate, d'au moins un composé qui comprend au moins un groupe hydrophile et au moins un groupe réactif par rapport aux groupes isocyanate et d'au moins un polyisocyanate,
et
H2) au moins un composé basique différent de H1), le composant H2) étant choisi parmi NH₃, (NH₄)₂CO₃, NH₄HCO₃, les monoalkylamines, les dialkylamines, les trialkylamines, les aminoalcools, les hétérocycles contenant de l'azote et les mélanges de ceux-ci.

2. Procédé selon la revendication 1, la composition de monomères utilisée pour la préparation de la composition de copolymères CP) contenant
a) 70 à 99,99% en poids, par rapport au poids total des monomères utilisés pour la polymérisation, d'acide acrylique,
b) 0 à 29,99% en poids, par rapport au poids total des monomères utilisés pour la polymérisation, d'au moins un composé non ionique hydrophile différent de a) présentant une double liaison polymérisable par voie radicalaire α,β-éthyléniquement insaturée,
c) 0,01 à 1% en poids, par rapport au poids total des monomères utilisés pour la polymérisation, d'au moins un composé polymérisable par voie radicalaire, réticulant, qui contient au moins deux doubles liaisons α,β-éthyléniquement insaturées par molécule.

3. Procédé selon la revendication 1, la préparation des composés H1) présentant des groupes polyisobutényle partant de polyisobutènes, qui sont choisis parmi :
- les polyisobutènes hautement réactifs,
- les polyisobutènes présentant au moins un groupe terminal contenant de l'azote,
- les poly(alcools isobutényliques),
- les polyisobuténylaldéhydes,
- les polyisobutènes présentant au moins un groupe terminal acide carboxylique ou un dérivé de ceux-ci,
- les mélanges de deux ou de plus de deux des composés susmentionnés.

4. Procédé selon l'une quelconque des revendications précédentes, l'adjuvant H1) comprenant un produit de transformation de PIBSA et un poly(oxyde d'éthylène)monométhyléther ou étant constitué d'un produit de transformation de PIBSA et d'un poly(oxyde d'éthylène)monométhyléther.

5. Procédé selon l'une quelconque des revendications précédentes, le composé présentant une structure à blocs H1) étant choisi parmi les composés d'uréthane qui contiennent, sous forme intégrée,
p1) au moins un composé qui comprend au moins un groupe hydrophobe et au moins un groupe réactif par rapport aux groupes isocyanate,
p2) au moins un composé qui comprend au moins un groupe hydrophile et au moins un groupe réactif par rapport aux groupes isocyanate, et
p3) au moins un polyisocyanate.

6. Procédé selon la revendication 5, les composés d'uréthane H1) contenant en outre sous forme intégrée au moins un composé p4) qui est choisi parmi les composés présentant un poids moléculaire dans la plage de 56 à 280 g/mole, qui contiennent deux groupes réactifs par rapport aux groupes isocyanate par molécule, de préférence choisi parmi les diols, les diamines, les aminoalcools et les mélanges de ceux-ci.

7. Procédé selon l'une quelconque des revendications 5 ou 6, les composés d'uréthane H1) contenant en outre sous forme intégrée au moins un composé p5) qui est choisi parmi les composés présentant un poids moléculaire dans la plage de 56 à 280 g/mole, qui ne contiennent qu'un groupe réactif par rapport aux groupes isocyanate par molécule.

8. Procédé selon l'une quelconque des revendications précédentes, le composant H2) comprenant de l'hydrogénocarbonate d'ammonium ou étant constitué d'hydrogénocarbonate d'ammonium.

9. Procédé selon l'une quelconque des revendications précédentes, le mélange réactionnel utilisé pour la préparation de la composition de copolymères CP) présentant, tout au long de la copolymérisation, une teneur en eau d'au plus 5% en poids, de préférence d'au plus 3% en poids, en particulier d'au plus 2% en poids.

10. Procédé selon l'une quelconque des revendications précédentes, la composition de monomères utilisée pour la préparation de la composition de copolymères CP) contenant en outre au moins un composé d) présentant une double liaison α,β-éthyléniquement insaturée, polymérisable par voie radicalaire et au moins un groupe cationogène et/ou cationique par molécule.

11. Procédé selon l'une quelconque des revendications précédentes, la composition de monomères utilisée pour la préparation de la composition de copolymères CP) contenant en outre au moins un composé e) différent de l'acide acrylique présentant une double liaison α,β-éthyléniquement insaturée, polymérisable par voie radicalaire et au moins un groupe anionogène et/ou anionique par molécule.

12. Procédé selon l'une quelconque des revendications précédentes, la composition de monomères utilisée pour la préparation de la composition de copolymères CP) contenant en outre au moins un composé f) qui est choisi parmi les esters d'acides monocarboxyliques et dicarboxyliques α,β-éthyléniquement insaturés avec des C₁-C₃₀-alcanols, les amides d'acides monocarboxyliques et dicarboxyliques α,β-éthyléniquement insaturés avec des mono-(C₁-C₃₀-alkyl) amines et di-(C₁-C₃₀-alkyl) amines, les N,N-diallylamines, leurs produits d'addition d'acide et leurs produits de quaternisation, les N,N-diallyl-N-alkylamines, leurs produits d'addition d'acide et leurs produits de quaternisation, les uréthane(méth)acrylates avec des groupes oxyde d'alkylène, les esters d'alcool vinylique et d'alcool allylique avec des acides C₁-C₃₀-monocarboxyliques, les C₁-C₃₀-alkylvinyléthers, les aromatiques de vinyle, les halogénures de vinyle, les halogénures de vinylidène, les C₂-C₈-monooléfines, les hydrocarbures non aromatiques présentant au moins deux doubles liaisons conjuguées et les mélanges de ceux-ci.

13. Composition de copolymères CP), pouvant être obtenue par un procédé tel que défini dans l'une quelconque des revendications 1 à 12.

14. Agent cosmétique contenant
A) au moins une composition de copolymères CP), pouvant être obtenue par un procédé tel que défini dans l'une quelconque des revendications 1 à 12,
B) au moins une substance active cosmétiquement acceptable et
C) le cas échéant au moins un autre adjuvant cosmétiquement acceptable différent de CP) et de B).

15. Agent pharmaceutique contenant
A) au moins une composition de copolymères, pouvant être obtenue par un procédé tel que défini dans l'une quelconque des revendications 1 à 12,
B) au moins une substance active pharmaceutiquement acceptable et
C) le cas échéant au moins un autre adjuvant pharmaceutiquement acceptable différent de A) et de B).

16. Utilisation d'une composition de copolymères CP), pouvant être obtenue par un procédé tel que défini dans l'une quelconque des revendications 1 à 12, dans le domaine alimentaire pour la modification des propriétés rhéologiques, comme adjuvant dans la pharmacie, de préférence comme ou dans un/des agents de revêtement pour des formes médicamenteuses solides, pour la modification de propriétés rhéologiques, comme composé tensioactif, comme ou dans un/des adhésifs ainsi que comme ou dans un/des agents de revêtement pour l'industrie du textile, du papier, l'imprimerie et l'industrie du cuir.
